# EUROPEAN PATENT SPECIFICATION

(11) **EP 2 763 666 B1**
(45) Date of publication and mention of the grant of the patent: **16.12.2015**
(21) Application number: 12788282.7
(22) Date of filing: 03.10.2012
(51) Int. Cl.: A61K 31/00, A61K 31/197, A61K 31/341, A61K 31/415, A61K 39/04

(54) **COMPOSITION COMPRISING BACILLUS CALMETTE GUERIN WITH COX2 SELECTIVE/PREFERENTIAL INHIBITOR AND USES THEREOF.**
ZUSAMMENSETZUNG ENTHALTEND BACILLUS CALMETTE GUERIN MIT COX2 SELEKTIVEM/BEVORZUGTEM INHIBITOR UND DEREN VERWENDUNGEN
COMPOSITION CONTENANT BACILLUS CALMETTE GUERIN AVEC INHIBITEUR SELECTIVF/PRÉFÉRENTIEL DE COX2 ET UTILISATIONS.

(30) Priority: 05.10.2011 IT RM20110529
(43) Date of publication of application: 13.08.2014
(73) Proprietor: Universita' Degli Studi Di Camerino, 62032 Camerino (MC) (IT)
(72) Inventor: ROSSI, Giacomo, I-54100 Pisa (PI) (IT); SCARPONA, Silvia, I-60128 Ancona (AN) (IT)
(74) Representative: Currado, Luisa
(86) International application number: PCT/IB2012/055303
(87) International publication number: WO 2013/050947

(56) References cited:
- DOVEDI S J ET AL: "Celecoxib has Potent Antitumour Effects as a Single Agent and in Combination with BCG Immunotherapy in a Model of Urothelial Cell Carcinoma", EUROPEAN UROLOGY, ELSEVIER BV, NL, vol. 54, no. 3, 1 September 2008 (2008-09-01), pages 621-630, XP023316477, ISSN: 0302-2838, DOI: 10.1016/J.EURURO.2008.01.013 [retrieved on 2008-01-15]
- DOVEDI ET AL: "CYCLOOXYGENASE-2 INHIBITION: A POTENTIAL MECHANISM FOR INCREASING THE EFFICACY OF BACILLUS CALMETTE-GUERIN IMMUNOTHERAPY FOR BLADDER CANCER", JOURNAL OF UROLOGY, LIPPINCOTT WILLIAMS & WILKINS, BALTIMORE, MD, US, vol. 174, no. 1, 1 July 2005 (2005-07-01), pages 332-337, XP005529098, ISSN: 0022-5347, DOI: 10.1097/01.JU.0000161589.85869.AE
- Xing-Qing Pan: "The mechanism of the anticancer function of M1 macrophages and their use in the clinic", Chinese Journal of Cancer, 13 November 2012 (2012-11-13), XP55196872, ISSN: 1000-467X, DOI: 10.5732/cjc.012.10046
- G. Solinas ET AL: "Tumor-associated macrophages (TAM) as major players of the cancer-related inflammation", Journal of Leukocyte Biology, vol. 86, no. 5, 9 September 2009 (2009-09-09), pages 1065-1073, XP055141844, ISSN: 0741-5400, DOI: 10.1189/jlb.0609385
- BOURDETTE ET EL.: "BCG vaccine for clinically isolated syndrome and MS", AMERICAN ACADEMY OF NEUROLOGY, vol. 82, 2013, pages 15-16,
- LANCET, 2002, 359: 1221-31,
- IMMUNOPHARMACOLOGY AND IMMUNOTOXICOLOGY, 2011; 33(3): 545-563,
- TRENDS IN IMMUNOLOGY 23,9,2002,
- RISTORI ET AL.: "effects of BCG after...", AMERICAN ACADEMY OF NEUROLOGY, 2013, page 15,

## Description

The present invention refers to the field of human medicine and veterinary science and more in particular to the combination of Bacillus Calmette Guerin (BCG) with anti-COX-2 selective molecules and/or COX-2 preferential FANS for the prevention and/or treatment of pathologies related to the loss of macrophages functions and deficiencies of cell-mediated immunity.

### Background of the invention

The enhancement of host-cell-mediated immuno-competence and cytotoxic-cytolytic effector cells in different diseases as in tumours, with the intent to eradicate disease has been the focus of a great deal of basic and clinical research for nearly two decades (Itoh K, Tilden AB, Balch CM, Interleukin 2 activation of cytotoxic T-lymphocytes infiltrating into human metastatic melanomas Cancer Res. 1986; 46:3011-3017; Medler RJ, Whiteside TL, Vuyanovic NL, Hiserodt JC, Herberman RB, Human adherent lymphokine-activated killer cells (a new approach to generating antitumor effectors for adoptive immunotherapy), Cancer Res. 1988;48:3461-3469; Mule JJ, Yang J, Soyu S, Rosenberg SA The antitumor efficacy of lymphokine activated killer cells and recombinant interleukin 2 in vivo (direct correlation between reduction of established metastases and cytolytic activity of lymphokine activated killer cells), J Immunol, 1986;136:3899-3909; Rosenberg SA, Lotze MT, Muul LM, et al., Observations on the systemic administration of autologous LAK cells and rIL-2 to patients with metastatic cancer, N Engl J Med. 1985;313:1485; Rosenberg SA, Lotze MT, Mule JJ, New approaches to the immunotherapy of cancer using interleukin-2, Ann Intern Med, 1988;108:853; Schiltz PM, Beutel LD, Nayak SK, Dillman RO, Characterization of tumor-infiltrating lymphocytes derived from human tumors for use as adoptive immunotherapy of cancer, J Immunother., 1997;20:377-386; Topalian SL, Muul LM, Solomon D, Rosenberg SA, Expansion of human tumor infiltrating lymphocytes for use in immunotherapy trials, J Immunol Methods, 1987;102:127-141; West WH, Tauer KW, Yannelli JR, et al., Constant-infusion recombinant interleukin-2 in adoptive immunotherapy of advanced cancer, N Engl J Med, 1987;316:898; Nijhuis EWP, Kemenade EW, Figdor CG, van Lier RAW, Activation and expansion of tumor-infiltrating lymphocytes by anti-CD3 and anti-CD28 monoclonal antibodies, Cancer Immunol Immunother, 1990;32:245-250; Schmidt-Wolf IGH, Negrin RS, Kiem H, Blume KG, Weissman IL, Use of a SCID mouse/human lymphoma model to evaluate cytokine-induced killer cells with potent antitumor cell activity, J Exp Med, 1991;174:139-149; Topalian SL, Solomon D, Rosenberg SA, Tumor-specific cytolysis by lymphocytes infiltrating human melanomas, J Immunol, 1989;142:3714-3725; Takahashi H, Nakada T, Puisieux I, Inhibition of human colon cancer growth by antibody-directed human LAK cells in SCID mice, Science, 1993;259:1469). Since the original descriptions of lymphocyte-mediated cytotoxicity against human tumour cells in vitro, there have been numerous attempts to exploit such observations for therapeutic use in humans, with decidedly mixed results (Schiltz PM, Beutel LD, Nayak SK, Dillman RO, Characterization of tumor-infiltrating lymphocytes derived from human tumors for use as adoptive immunotherapy of cancer. J Immunother, 1997;20:377-386; Rosenberg SA, Lotze MT, Mule JJ, New approaches to the immunotherapy of cancer using interleukin-2, Ann Intern Med, 1988;108:853; Rosenberg SA, Lotze MT, Muul LM et al., Observations on the systemic administration of autologous LAK cells and rIL-2 to patients with metastatic cancer, N Engl J Med, 1985;313:1485; Lytle GH, Immunotherapy of breast cancer (a review of the development of cell-specific therapy) Semin Surg Oncol, 1991;7:211-216). Clinical applications of host-mediated cellular immunotherapy have included the treatment of patients with various diseases (i.e. HIV, Hepatitis, etc.), including malignancies (such as melanoma) by using interleukin (IL)-2-stimulated lymphokine-activated killer cells derived from autologous peripheral blood. Similarly, tumour-infiltrating lymphocytes, first isolated from primary tumours and subsequently cultured and expanded ex vivo, have also been administered clinically. Although historically a great deal of emphasis has been placed on the role of either natural killer (NK) cells or cytotoxic CD8+ T cells as the primary mediators of anti-protozoan, antiviral and antitumor cytotoxicity (Yannelli JR, Hyatt C, McConnell S et al., Growth of tumor-infiltrating lymphocytes from human solid cancers (summary of a 5-year experience), Int J Cancer, 1996;65:413-421; Bloom MB, Perry-Lalley D, Robbins PF et al., Identification of tyrosinase-related protein 2 as a tumor rejection antigen for the B16 melanoma, J Exp Med, 1997;185:453-459; Colella TA, Bullock TN, Russell LB, et al., Self-tolerance to the murine homologue of a tyrosinase-derived melanoma antigen (implications for tumor immunotherapy), J Exp Med, 2000;191:1221-1232; Dudley ME, Nishimura MI, Holt AK, Rosenberg SA, Antitumor immunization with a minimal peptide epitope (G9-209-2M) leads to a functionally heterogeneous CTL response, J Immunother, 1999;22:288-298; Fleischhauer K, Tanzarella S, Russo V, et al., Functional heterogeneity of HLA-A*02 subtypes revealed by presentation of a MAGE-3-encoded peptide to cytotoxic T cell clones, J Immunol, 1997;159:2513-2521; Kawakami Y, Robbins PF, Wang X et al., Identification of new melanoma epitopes on melanosomal proteins recognized by tumor infiltrating T lymphocytes restricted by HLA-A1, -A2, and -A3 alleles, J Immunol, 1998;161:6985-6992; Kim CJ, Parkinson DR, Marincola F, Immunodominance across HLA polymorphism (implications for cancer immunotherapy), J Immunother, 1998;21:1-16; Mateo L, Gardner J, Chen Q et al., An HLA-A2 polyepitope vaccine for melanoma immunotherapy, J Immunol . 1999;163:4058-4063). It is known the immune therapy for cancer with Bacillus Calmette Guerin (BCG) or BCG-cell-wall skeleton (BCG-CWS) (Seya T, Matsumoto M, Tsuji S, Begum NA, Nomura M, Azuma I, Hayashi A, Toyoshima K, Two receptor theory in innate immune activation: studies on the receptors for bacillus Culmet Guillen-cell wall skeleton.Arch Immunol Ther Exp (Warsz), 2001;49 Suppl 1:S13-21). Until now little attention has been paid to the role of human γδ T cells in this capacity. In fact, microbial components as BCG-cell-wall skeleton can exert immuno-stimulatory and toxic effects in vivo. The immuno-modulatory properties of mycobacterial constituents, including the cell wall and CpG DNA, have long been known as adjuvants and successfully applied in different agent-induced disease as in anticancer immunotherapy and efficient vaccination (Azuma I, Seya T, Development of immunoadjuvants for immunotherapy of cancer, Int Immunopharmacol. 2001 Jul;1(7):1249-59; Hayashi F, Yanagawa Y, Onoé K, Iwabuchi K, Dendritic cell differentiation with prostaglandin E results in selective attenuation of the extracellular signal-related kinase pathway and decreased interleukin-23 production, Immunology, 2010 Sep;131(1):67-76. Epub 2010 Apr 12; Ayashi, A, O Doi, I Azuma and K. Toyoshima, 1998, Immuno-friendly use of BCG-cell wall skeleton remarkably improves the survival rate of various cancer patients, Proc Jpn Acad, 74:50-55; Shimada S, O Yano, H Inoue, E Kuramoto, T Fukuda, H Yamamoto, T Kataoka, and T Tokunaga, 1985, Antitumor activity of the DNA fraction from Mycobacterium bovis BCG. II Effects on various syngeneic mouse tumors, JNCI 74:681-688). However, the mechanisms by which microbial adjuvants are recognized by the immune system had remained obscure until identification of the Toll-like receptor (TLR) family (Medzhitov R, 2001, Toll-like receptors and innate immunity, Nat Rev Immunol, 1:135-145) and rapid advances in the field of innate immunity. In recent years, a series of microbial constituents have been identified as specific TLR ligands and designated pathogen-associated molecular patterns (PAMPs) (Tsuji S, M Matsumoto, O Takeuchi, S Akira, I Azuma, A Hayashi, K Toyoshima and T. Seya, 2000, Maturation of human dendritic cells by cell wall skeleton of Mycobacterium bovis bacillus Calmette-Guérin: involvement of Toll-like receptors, Infect Immun, 68:6883-6890). Gram-negative bacterial lipopolysaccharide (LPS) is a TLR4 ligand, and peptidoglycan (PGN) and lipopeptides of gram-positive bacteria are ligands for TLR2. Different studies demonstrated that the induction of tumour necrosis factor alpha (TNF-α) production by Mycobacterium bovis BCG or by BCG-CWS was impaired in macrophages derived from both TLR2 and TLR4 knockout mice, which indicated the involvement of TLR2 and -4 pathways in the effects of BCG-CWS (Tsuji S, M Matsumoto, O Takeuchi, S Akira, I Azuma, A Hayashi, K Toyoshima, and T Seya, 2000, Maturation of human dendritic cells by cell wall skeleton of Mycobacterium bovis bacillus Calmette-Guérin: involvement of Toll-like receptors, Infect Immun, 68:6883-6890). All TLR receptors bear an interleukin-1 (IL-1) receptor-like cytoplasmic signalling domain, Toll-interleukin receptor homology domain (TIR), but their signalling domains are not identical and recruit different sets of adapter molecules (Horng T, GM Barton, RA Flavell, and R Medzhitov, 2002, The adaptor molecule TIRAP provides signalling specificity for Toll-like receptors, Nature, 420:329-333; Oshiumi H, M Matsumoto, K Funami, T Akazawa, and T Seya, 2003, TICAM-1, an adaptor molecule that participates in Toll-like receptor 3-mediated interferon-beta induction, Nat Immunol, 4:161-167; Oshiumi H, M Sasai, K Shida, T Fujita, M Matsumoto, and T Seya, 2003, TIR-containing adapter molecule (TICAM)-2, a bridging adapter recruiting to Toll-like receptor 4 TICAM-1 that induces interferon-β, J Biol Chem, 278:49751-49762; Yamamoto M, S Sato, H Hemmi, H Sanjo, S Uematsu, T Kaisho, K Hoshino, O Takeuchi, M Kobayashi, T Fujita, K Takeda, and S Akira, 2002, Essential role for TIRAP in activation of the signalling cascade shared by TLR2 and TLR4, Nature, 420:324-329). Their intracellular signalling cascades and cross talk are just beginning to be elucidated. Furthermore, TLRs work in a combinatorial manner, thereby recognizing diverse ligands and probably also eliciting complex intracellular signalling (Akira S, K Takeda, and T Kaisho, 2001, Toll-like receptors: critical proteins linking innate and acquired immunity, Nat Immunol, 2:675-680; Imler JL and JA Hoffmann, 2003, Toll signaling: TIReless quest for specificity, Nat Immunol, 4:105-106; Underhill DM, and A Ozinsky, 2002, Toll-like receptors: key mediators of microbe detection, Curr Opin Immunol, 14:103-110). Thus, the analysis of immune cells' transcriptional responses toward structurally defined microbial ligands is currently of growing interest to clarify adjuvant activity and its signalling pathways (Doyle S, S Vaidya, R O'Connell, H Dadgostar, P Dempsey, T Wu, G Rao, R Sun, M Haberland, R Modlin and G. Cheng, 2002, IRF3 mediates a TLR3/TLR4-specific antiviral gene program, Immunity 17:251-263; Huang Q, D Liu, P Majewski, LC Schulte, JM Korn, RA Young, ES Lander, and N Hacohen, 2001, The plasticity of dendritic cell responses to pathogens and their components, Science, 294:870-875; Toshchakov V, BW Jones, PY Perera, K Thomas, MJ Cody, S Zhang, BR Williams, J Major, TA Hamilton, MJ Fenton, and SN Vogel, 2002, TLR4, but not TLR2, mediates IFN-beta-induced STAT1alpha/beta-dependent gene expression in macrophages, Nat Immunol, 3:392-398; Wang ZM, C Liu and R Dziarski, 2000, Chemokines are the main proinflammatory mediators in human monocytes activated by Staphylococcus aureus, peptidoglycan and endotoxin, J Biol Chem, 275:20260-20267). Since BCG-CWS is responsible for part of the BCG adjuvant activity and lacks LPS-like toxic potential in a dose effectively inducing immunostimulatory activity (Azuma I, Seya T, Development of immunoadjuvants for immunotherapy of cancer, Int Immunopharmacol, 2001 Jul;1(7):1249-59; Hayashi F, Yanagawa Y, Onoé K, Iwabuchi K, Dendritic cell differentiation with prostaglandin E results in selective attenuation of the extracellular signal-related kinase pathway and decreased interleukin-23 production, Immunology, 2010 Sep;131(1):67-76. Epub 2010 Apr 12; Ayashi A, O Doi, I Azuma and K Toyoshima, 1998, Immuno-friendly use of BCG-cell wall skeleton remarkably improves the survival rate of various cancer patients, Proc Jpn Acad, 74:50-55), it has been used postoperatively for adjuvant immunotherapy to treat patients with cancer. Thus, BCG-CWS binds multiple receptors and exerts some effects that are overlapping and others that are distinct from those of LPS on antigen-presenting cells (APCs). IL-12p70 (the p40-p35 complex) is expressed in monocyte-derived DCs (APCs) and acts as a crucial factor on lymphocytes for IFN-γ production (Trincheri G, 2003, Interleukin-12 and the regulation of innate resistance and adaptive immunity, Nat Rev Immunol, 3:133-143) and differential gene expression analysis showed that IL-12p40, but not IL12p35, transcripts were markedly induced by BCG or BCG-CWS. This is consistent with recent finding (Matsumoto M, T Seya, S Kikkawa, S Tsuji, K Shida, M Nomura, M Kurita-Taniguchi, H Ohigashi, M Higashiyama, H Yokouchi, K Takami, A Hayashi, I Azuma, T Masaoka, K Kodama, and K Toyoshima, 2001, Interferon gamma-producing ability in blood lymphocytes of patients with lung cancer through activation of the innate immune system by BCG cell wall skeleton, Int Immunopharmacol, 1:1559-1569.) and a previous report (Re F and JL Strominger, 2001, Toll-like receptor 2 (TLR2) and TLR4 differentially activate human dendritic cells, J Biol Chem, 276:37692-37699) that BCG-CWS induced the release of large amounts of IL12p40 in vitro and IL-23p19 is co-expressed with IL-12p40 by BCG and other TLR2 agonists. It is also known that IL-23 may participate in a distinct mode of protective immunity regulating autoimmune responses (Cua D J, J Sherlock, Y Chen, AC Murphy, B Joyce, B Seymour, L Lucian, W To, S Kwan, T Churakova, S Zurawski, M Wiekowski, SA Lira, D Gorman, RA Kastelein, and JD Sedgwick, 2003, Interleukin-23 rather than interleukin-12 is the critical cytokine for autoimmune inflammation of the brain, Nature, 421:744-748). IL-12 can induce chemokine receptors CCR5 and CXCR3 in lymphocytes and encourage the effector lymphocytes to kill target cells expressing MIP-1 and IP-10 (Fujiwara H and T. Hamaoka, 2001, Coordination of chemokine and adhesion systems in intratumoral T cell migration responsible for the induction of tumor regression, Int Immunopharmacol, 1:613-620). Although there are many DC subsets that differentially respond to PAMPs to reveal distinct cytokine profiles (Matsumoto M, K Funami, M Tanabe, H Oshiumi, M Shingai, Y Seto, A Yamamoto and T Seya, 2003, Subcellular localization of human Toll-like receptor 3 in human dendritic cells, J Immunol, 171:3154-3162), maturation of myeloid or monocyte-derived DCs would be crucial for APC function. BCG or BCG-CWS serves as a ligand for TLR2 and TLR4, while LPS serves as a ligand for TLR4. TLR2 and TLR4 partly share their signalling pathways since their adapter molecules are MyD88 and TIRAP (also named as Mal), leading to activation of NF-κB (Horng T, GM Barton, RA Flavell and R Medzhitov, 2002, The adaptor molecule TIRAP provides signalling specificity for Toll-like receptors, Nature, 420:329-333; Yamamoto M, S Sato, H Hemmi, H Sanjo, S Uematsu, T Kaisho, K Hoshino, O Takeuchi, M Kobayashi, T Fujita, K Takeda and S Akira, 2002, Essential role for TIRAP in activation of the signalling cascade shared by TLR2 and TLR4, Nature, 420:324-329). TLR4 additionally recruits TICAM-2 and TICAM-1, which activate IRF-3 potentially and NF-κB weakly (Oshiumi H, M Matsumoto, K Funami, T Akazawa and T Seya, 2003, TICAM-1, an adaptor molecule that participates in Toll-like receptor 3-mediated interferon-beta induction, Nat Immunol, 4:161-167). There are no all-or-none difference in the NF-κB-dependent genes expressed by the two TLR ligands. Cytokines such as TNF-α, IL-6, IL-8, and IL-1β or chemokine like MIP and MCP are NF-κB dependent and are commonly induced by BCG-CWS and LPS. Thus, in terms of gene expression, there are minimal differences in NF-κB-dependent gene expression between BCG-CWS and LPS. However, the point is that there are several genes specifically expressed by BCG-CWS. IL-23p19, LIV-1, and HKE-4 are examples of such genes and are suggestive of the presence of additional signalling pathways for the BCG-CWS response. LPS, but not the TLR2 agonists, activates IRF-3 and induces expression of IFN-inducible genes in a MyD88-independent fashion, and BCG-CWS-mediated TLR4 activation barely induces activation of IRF-3 and expression of IFN-inducible genes. LPS-TLR4 signalling may be toxic because of simultaneous activation of MyD88 and TICAM-1. LPS-dependent TLR4 signalling somewhat resembles TLR3 signalling (Matsumoto M, K Funami, M Tanabe, H Oshiumi, M Shingai, Y Seto, A Yamamoto and T. Seya, 2003, Subcellular localization of human Toll-like receptor 3 in human dendritic cells, J Immunol, 171:3154-3162) and is distinct from BCG mediated TLR4 signalling. The BCG-CWS-mediated gene regulatory profile common to TLR2 and TLR4 is important for therapeutic potential in adjuvant therapy. MyD88 is an adapter common to TLR2 and TLR4 and is essential for effective cytotoxic T lymphocyte induction with MyD88-deficient mice (Akazawa T, H Masuda, Y Saeki, M Matsumoto, K Takeda, S Akira, I Azuma, K Toyoshima, and T Seya, Adjuvant-mediated tumor regression and tumor-specific CTL induction are impaired in MyD88-deficient mice, Cancer Res, 2010). Intracutaneous application is the usual mode of clinical BCG application; mast cells, Langerhans cells, and iDCs underneath the skin are the immune cells that first encounter the administered BCG. Several in vitro studies demonstrated that BCG-CWS is efficiently taken up by iDCs and promoted DC maturation (Tsuji, S, M Matsumoto, O Takeuchi, S Akira, I Azuma, A Hayashi, K Toyoshima, and T Seya, 2000, Maturation of human dendritic cells by cell wall skeleton of Mycobacterium bovis bacillus Calmette-Guerin: involvement of Toll-like receptors, Infect Immun, 68:6883-6890). BCG products also induce the expression of various co-stimulatory molecules and elicit an extensive mixed-lymphocyte reaction (Tsuji S, M Matsumoto, O Takeuchi, S Akira, I Azuma, A Hayashi, K Toyoshima and T Seya, 2000, Maturation of human dendritic cells by cell wall skeleton of Mycobacterium bovis bacillus Calmette-Guérin: involvement of Toll-like receptors, Infect Immun, 68:6883-6890), The mode of the DC and Mph response is critical in determining whether NK or T cells are preferentially activated via cytokines and adhesion molecules (Colucci F, MA Caligiuri and JP DiSanto, 2003, What does it take to make a natural killer?, Nat Rev Immunol, 3:413-425). The BCG administration induces in area of injection and, in a minor manner, a systemic inflammatory response that culminate in micro-granuloma formation. This inflammatory process is the most important moment of immunity system activation and, at the same time, the moment of activation of some enzymatic pathway, as COX2 way, and of production of some molecules as PGE2 that negatively modulate the macrophage activity and the phlogosis process. Prostaglandin (PG) E(2), via the EP2 receptor-dependent signalling pathway, inhibits the expression of CD36 in peritoneal macrophages, resulting in reduced phagocytic ability. PGE(2)-mediated inhibition of macrophage phagocytic capability was restored by ectopic expression of CD36. In contrast, blockade of PGE(2) production by cyclooxygenase inhibitors (anti-COX2) enhanced the phagocytic ability of peritoneal macrophages. The above evidences, taken together, reveal an immune dysfunction during the BCG inoculation that partially inhibits the strong Mph and DC activation performed by BCG or BCG-CWS. It is demonstrated that a balance between interleukin (IL)-12 and IL-23 production by dendritic cells (DCs) is crucial for the induction of appropriate immune responses. Additionally a low dose of IL-4 during the early stages of cell-mediated immuno-response is fundamental to adequately stimulate the CD8+ lymphocyte subset. The evaluation of the effect of PGE(2) treatment of DCs during differentiation on IL-12 and IL-23 production in response to Toll-like receptor (TLR) stimulation, shows that upon TLR stimulation, IL-23 production by PGE2 treated DCs is markedly decreased compared with that by control DCs. However, no significant differences are detected in IL-12 production between these types of DC (Hayashi F, Yanagawa Y, Onoé K, Iwabuchi K, Dendritic cell differentiation with prostaglandin E results in selective attenuation of the extracellular signal-related kinase pathway and decreased interleukin-23 production, Immunology, 2010,Sep;131(1):67-76. Epub 2010 Apr 12). Similarly, studies performed on specific evaluation of Mph and DCs activity reduction after trauma, stressors and burning induced-lesions demonstrated that very early after chest trauma, inflammatory mediators are present in the intra-alveolar compartment, particularly PGE2 that substantially increased as early at 10 min. This fact change alveolar Mph activity to release cytokines and chemokines. Then, blunt chest trauma also changes the phagocytic and chemotactic activity of alveolar Mph. These functional changes of Mph might enable them to better ward off potential pathogens in the course after trauma. (Knöferl MW, Liener UC, Perl M, Brückner UB, Kinzl L, Gebhard F, Blunt chest trauma induces delayed splenic immunosuppression, Shock, 2004, Jul;22(1):51-6) In fact, severe blunt chest trauma is frequently associated with multiple organ failure and sepsis. Post traumatic immunosuppression seems to play a major role in their development (Ulrich C Liener, Mario Perl, Markus S Huber-Lang, Daniel H Seitz, Uwe B Brückner, Florian Gebhard, Markus W Knöferl, Is the function of alveolar macrophages altered following blunt chest trauma?, Langenbecks Arch Surg, 2010, Apr 15). A study performed in a mouse model, demonstrated the influence of pulmonary contusion on lymphocytes and splenic macrophages. The results indicated that at 2 hours after blunt chest trauma, plasma TNF-alpha and IL-6 were markedly increased. At the same time, no differences in splenocyte cytokine production were detectable. However, at 24 hours a significantly depressed cytokine release was observed in trauma animals. Furthermore, splenic macrophages showed a significantly decreased production of TNF-alpha, IL-10, and IL-12 at 24 hours and markedly increased release of IL-18 at 2 hours after trauma. The results indicate that blunt chest trauma causes severe immuno-dysfunction of lymphocytes and splenic macrophages, due to an early and severe increase of PGE2 levels at the site of injury. Thus, lung contusion as a localized type of trauma causes dysfunction of immuno-competent cell populations, which are located distant from the site of injury.(Knöferl MW, Liener UC, Perl M, Brückner UB, Kinzl L, Gebhard F, Blunt chest trauma induces delayed splenic immunosuppression, Shock, 2004, Jul;22(1):51-6). Similarly, major thermal injury induces the activation of an inflammatory cascade that contributes to the development of subsequent immunosuppression, increased susceptibility to sepsis and multiple organ failure. The productive capacity of macrophages for inflammatory mediators, (i.e., nitric oxide, prostaglandins, TNF-alpha, IL-6 etc.), is profoundly increased post-burn, thereby implicating macrophages in the development of the post-burn immunosuppression. Prostanoids have profound immunosuppressive properties (Bomalaski JS, Williamson PK, Zurier RB, 1983, Prostaglandins and the inflammatory response, Clin Lab Med, 3, 695-717; Griffiths RJ, 1999, Prostaglandins and inflammation. In Inflammation: Basic Principles and Clinical Correlates,Jl Gallin, R Snyderman eds, Philadelphia: Lippincott Williams & Wilkins, 349-360) and macrophage-derived PGE2 has been implicated as the mediator responsible for T lymphocyte dysfunction after thermal injury (Yang L, Hsu B, 1992, The role of macrophages (Mf) and PGE-2 in postburn immunosuppression, Burns, 18, 132-136; Grbic JT, Mannick JA, Gough DB, Rodrick ML, 1991, The role of prostaglandin E2 in immune suppression following injury, Ann Surg, 214, 253-263; Schwacha MG, Samy TSA, Cantania RA, Chaudry IH, 1998, Thermal injury alters macrophage responses to prostaglandin E2: contribution to the enhancement of inducible nitric oxide synthase activity, J Leukoc Biol, 64, 740-746). Suppression of PGE2 production by inhibition of cyclooxygenase activity improves immune function and survival post-burn (Horgan PG, Mannick JA, Dubravec DB, Rodrick ML, 1990, Effect of low dose recombinant interleukin 2 plus indomethacin on mortality after sepsis in a murine burn model, Br J Surg, 77, 401-404). Furthermore, PGE2 can inhibit many aspects of macrophage function (Snyder DS, Beller Dl, Unanue E, 1982, Prostaglandins modulate macrophage la expression, Nature, 299, 163-165; Tannenbaum CS, Hamilton TA, 1989, Lipopolysaccharide induced gene expression in murine peritoneal macrophages is selectively suppressed by agents that elevate intracellular cAMP, J Immunol, 142, 1274-1282), and thermal injury also alters macrophage responses to PGE2 (Molloy RG, O'Riordain M, Holtzeimer R, Nestor M. Collins K, Mannick, JA, Rodrick ML, 1993, Mechanisms of increased tumor necrosis factor production after thermal injury. Altered sensitivity to PGE2 and immunomodulation by indomethacin, J Immunol, 151, 2142-2149; Schwacha MG, Samy TSA, Cantania RA, Chaudry IH, 1998, Thermal injury alters macrophage responses to prostaglandin E2: contribution to the enhancement of inducible nitric oxide synthase activity, J Leukoc Biol, 64, 740-746). Consistent with previous findings (Yang, L, Hsu B, 1992, The role of macrophages (Mf) and PGE-2 in postburn immunosuppression, Burns 18, 132-136; Miller-Graziano CL, Fink M, Wu WY, Szabo G, Kodys K, 1988, Mechanisms of altered monocyte prostaglandin E2 production in severely injured patients, Arch Surg, 123, 293-299; Ogle CK, Mao JX, Wu JZ, Ogle JD, Alexander JW, 1994, The production of tumor necrosis factor, interleukin 1, interleukin 6, and prostaglandin E2 by isolated enterocytes and gut macrophages: effect of lipopolysaccaride and thermal injury, J. Burn Care Rehabil, 15, 470-477), macrophages from injured mice produced elevated amounts of PGE2. However, the lack of gamma/delta T lymphocytes did not modify the elevated PGE2 response by macrophages post-burn, suggesting that the suppression in pro-inflammatory cytokine production observed in TCR d-/- mice is independent of control by PGE2. The previous mentioned studies evidenced that Prostaglandin E2 produced endogenously (by cyclooxygenases-way) can regulate macrophage phagocytosis. Cyclooxygenase activity reduction (mainly through inhibition of inducible Cox-2) can induce PGE2 synthesis depression and can activate the phagocytosis process (Schwacha MG, Chaudry IH, The cellular basis of post-burn immunosuppression: macrophages and mediators, Int J Mol Med, 2002, Sep;10(3):239-43). This fact is well explicated by the action of some conjugated linoleic acid dienes (trans-10, cis-12 CLA and cis-9, trans-11 CLA) in change the phagocytic activity potential of human macrophages. In fact both CLA isomers increased macrophage phagocytosis through inhibition of Cox-2 expression (might by inactivation the NF-kB pathway). The inhibition of mRNA Cox-2 expression contributed (particularly with respect to trans-10, cis-12 CLA) to a decrease in protein Cox-2 synthesis and to reduction of prostaglandin E2 content in the cell. The inhibition of PGE2 synthesis (by CLA treatment or in another way) enhances the phagocytosis process in macrophages (Stachowska E, Baskiewicz-Masiuk M, Dziedziejko V, Adler G, Bober J, Machaliriski B, Chlubek D, Conjugated linoleic acids can change phagocytosis of human monocytes/macrophages by reduction in Cox-2 expression, Lipids, 2007, Aug;42(8):707-16. Epub 2007 Jun 15). One reason proposed for the failure of Mycobacterium bovis Bacillus Calmette Guérin (BCG) vaccination to adequately control the spread of tuberculosis is a limited ability of the vaccine to induce effective CD8 T cell responses (Ryan AA, Nambiar JK, Wozniak TM, Roediger B, Shklovskaya E, Britton WJ, Fazekas de St Groth B, Triccas JA, Antigen load governs the differential priming of CD8 T cells in response to the bacille Calmette Guerin vaccine or Mycobacterium tuberculosis infection, J Immunol, 2009 Jun 1;182(11):7172-7).

A broad range of immune responses and clinical manifestations have been described in CanL. Leishmania infection in dogs may be manifested as a subclinical infection, a self-limiting disease (Bottero E, Poggi M, Viglione M, Lesioni papulari indotte da Leishmania spp. in 8 cani giovani, Veterinaria 1, 2006,33-36), or a non-self-limiting and severe illness. In dogs, the two opposite extremes of this clinical spectrum are characterized by: protective immunity that is CD4 T cell mediated by the release of γ-interferon, IL-2 and TNFα that induce macrophage anti-Leishmania activity, and disease susceptibility that is associated with the production of a marked humoral non-protective immune response and a reduced or depressed cell mediated immunity with a mixed Th1 and Th2 cytokines response (Alvar J, Canavate C, Molina R, Moreno J, Nieto J, Canine leishmaniasis, Adv Parasitol, 57 (2004),1-88; Baneth G, Koutinas AF, Solano-Gallego L, Bourdeau P, Ferrer L, Canine leishmaniosis new concepts and insights on an expanding zoonosis: part one, Trends Parasitol, 24, 2008, 324-330). Within this spectrum, clinical disease can range from a mild papular dermatitis associated with specific cellular immunity and low humoral responses (Ordeix L, Solano-Gallego L, Fondevila D, Ferrer L, Fondati A, Papular dermatitis due to Leishmania spp. infection in dogs with parasite-specific cellular immune responses, Vet. Dermatol. 16 (2005), 187-191) to a severe disease characterized by renal damage with glomerulonephritis due to immune complex deposition associated with a massive humoral response and high parasite loads (Costa FA, Goto H, Saldanha LC, Silva SM, Sinhorini IL, Silva TC, Guerra JL, Histopathologic patterns of nephropathy in naturally acquired canine visceral leishmaniasis, Vet Pathol, 40, 2003, 677-684). These two important observations are strictly related with two other observations in Leishmania immunology: the demonstration of a dramatic dichotomy in T helper 1 versus T helper 2 subset expansion leading to protection versus disease exacerbation; and analysis of the macrophage activation pathways leading to enhanced intracellular killing of parasites, in particular the tumour necrosis factor alpha (TNF alpha)-dependent sustained induction of the inducible nitric oxide synthase gene (Nos2) leading to the generation of large amounts of nitric oxide (NO). In Pinelli E, Gonzalo RM, Boog CJ, Rutten VP, Gebhard D, Del Real G, Ruitenberg EJ, Leishmania infantum-specific Tcells lines derived from asymptomatic dogs that lyse infected macrophages in a major histocompatibility complex-restricted manner, Eur J Immunol, 25 (6), 1995, 1594-1600, has been demonstrated that in dog as in human and in mice CD8+ lymphocytes showed a fundamental role in interferon gamma production and in direct and indirect lysis of parasitized macrophages. Lysis of infected macrophages by T cell lines is major histocompatibility complex restricted. Characterization of parasite-specific cytotoxic T cell lines revealed that the responding cells are CD8+. However, for some animals, CD4+ T cells that lyse infected macrophages were also found. In contrast to asymptomatic dogs, lymphocytes from symptomatic dogs failed to proliferate and produce interferon-gamma after Leishmania antigen stimulation in vitro and were not capable of lysing infected macrophages. These results suggest that both the production of strong concentrations of interferon-gamma, by the way of Th1 response stimulation and good NO production by parasitized macrophages, and the destruction of the parasitized host cells by Leishmania-specific T cells, maximally of CD8+ phenotype, play an important role in resistance to leishmaniasis. Different factors influence the dog resistance or susceptibility status to the CanL: factors such as age, gender, nutrition, host genetics, co-infections and/or concomitant disease, immunosuppressive conditions, parasitic burden, virulence of Leishmania strain, previous infections and method of transmission can affect the polarity of clinical manifestations of Leishmania infection. But all these factors are important because can influenced cytokine environment and to exacerbate the Th1/Th2 dichotomy. In spite of a recent study demonstrates that IL-4, IL-10 and TNF-alpha mRNA levels significantly increased only in symptomatic dogs at 8 months P.I. in comparison to the levels found in asymptomatic or control dogs (Panaro MA, Brandonisio O, Cianciulli A, Cavallo P, Lacasella V, Paradies P, Testini G, De Caprariis D, Mitolo V, Otranto D, Cytokine espression in dogs with natural Leishmania infantum infection, Parasitology, 136 (8), 2009, 823-831), different studies demonstrates that mild levels of II-4 are necessaries to induce a good CD8+ response. Different studies suggest that the strong cytokine response induced by Leishmania and Plasmodium infection affects host T cell responses, inhibiting protective CD8+ T cells against the intracellular-stage of the disease (Ocaña-Morgner C, Wong KA, Lega F, Dotor J, Borras-Cuesta F, Rodriguez A, Role of TGF-beta and PGE2 in T cell responses during Plasmodium yoelii infection, Eur J Immunol. 2007 ,Jun;37(6):1562-74).

During an acute blood-stage malaria infection, T cell responses to malaria and other bystander antigens are inhibited. Plasmodium infection induces strong cytokine responses that facilitate parasite clearance but may interfere with T cell functions, as some of the soluble immune mediators induced are also general inhibitors of T cell responses. Some authors (Ocaña-Morgner C, Wong KA, Lega F, Dotor J, Borras-Cuesta F, Rodriguez A, Role of TGF-beta and PGE2 in T cell responses during Plasmodium yoelii infection, Eur J Immunol, 2007 Jun;37(6):1562-74) using a malaria mouse model, have analysed the cytokines produced by dendritic cells in response to P. yoelii infection that have potential T cell inhibitory activity. Results demonstrated that during acute infection DC migrate to the spleen and secrete TGF-beta, prostaglandin E2 (PGE2) and IL-10. The role of these T cell inhibitors, particularly PGE2, in a particular T cell response is evident and important during malaria infections: the CD8+ T cells generated against the liver-stage of the disease are suppressed. In fact, during blood-stage infection, inhibition of the activity of TGF-beta and PGE2 restores the CD8+ T cell responses generated by sporozoites, increasing protection against re-infection. These findings suggest that the strong cytokine response induced by blood-stage P. yoelii infection affects host T cell responses, inhibiting protective CD8+ T cells against the liver-stage of the disease (Ocaña-Morgner C, Wong KA, Lega F, Dotor J, Borras-Cuesta F, Rodriguez A. Role of TGF-beta and PGE2 in T cell responses during Plasmodium yoelii infection, Eur J Immunol, 2007, Jun;37(6):1562-74). In fact, Malaria starts with Plasmodium sporozoites infection of the host's liver, where development into blood stage parasites occurs. It is not clear why natural infections do not induce protection against the initial liver stage and generate low CD8+ T cell responses. Some Authors (Ocaña-Morgner C, Mota MM, Rodriguez A. Malaria blood stage suppression of liver stage immunity by dendritic cells, J Exp Med, 2003 Jan 20;197(2):143-51) have demonstrated that Plasmodium blood stage infection suppresses CD8+ T cell immune responses that were induced against the initial liver stage. Blood stage Plasmodium affects dendritic cell (DC) functions, inhibiting maturation and the capacity to initiate immune responses and inverting the interleukin (IL)-12/IL-10 secretion pattern. The interaction of blood stage parasites with DCs induces the secretion of soluble factors that inhibit the activation of CD8+ T cells in vitro, particularly secretion of high levels of PGE2, and the suppression of protective CD8+ T cell responses against the liver stage in vivo. This evasion mechanism leaves the host unprotected against reinfection by inhibiting the immune response against the initial liver stage of the disease. CD8+ T cells have been implicated as critical effector cells in protection against pre-erythrocytic stage malaria, including the potent protective immunity of mice and humans induced by immunization with radiation-attenuated Plasmodium spp. sporozoites. This immunity is directed against the Plasmodium spp. parasite developing within the host hepatocyte and for a number of years has been presumed to be mediated directly by CD8+ CTL or indirectly by IFN-gamma released from CD8+ T cells. Experiments performed in BALB/c mice established that, after immunization with irradiated sporozoites or DNA vaccines, parasite-specific CD8+ T cells trigger a novel mechanism of adaptive immunity dependent on T cell- and non-T cell-derived cytokines (in particular IFN-γ and IL-12) and requires NK cells but not CD4+ T cells (Doolan DL, Hoffman SL, IL-12 and NK cells are required for antigen-specific adaptive immunity against malaria initiated by CD8+ T cells in the Plasmodium yoelii model, J Immunol, 1999, Jul 15;163(2):884-92). The absolute requirement for CD8+ T cells to initiate such an effector mechanism, and the requirement for IL-12 and NK cells in such vaccine-induced protective immunity, are unique and underscore the complexity of the immune responses that protect against malaria and other intracellular pathogens. However, the relative capacity of the BCG vaccine and virulent Mycobacterium tuberculosis to induce activation of CD8 T cells is well characterized. Using a TCR transgenic CD8 T cell transfer model, some authors demonstrated significant activation of Ag-specific CD8 T cells by BCG, but responses were delayed and of reduced magnitude compared with those following infection with M. tuberculosis. The degree of CD8 T cell activation was critically dependent on the level of antigenic stimulation, as modifying the infectious dose to achieve comparable numbers of BCG or M. tuberculosis in draining lymph nodes led to the same pattern of CD8 T cell responses to both strains. Factors specific to M. tuberculosis infection did not influence the priming of CD8 T cells, as co-delivery of M. tuberculosis with BCG did not alter the magnitude of BCG-induced T cell activation (Morris SC, Heidorn SM, Herbert DR, Perkins C, Hildeman DA, Khodoun MV, Finkelman FD, Endogenously produced IL-4 nonredundantly stimulates CD8+ T cell proliferation, J Immunol, 2009, Feb 1;182(3):1429-38). CD8+ lymphocytes, as general T cell proliferation and survival, are regulated by the cytokine receptor common gamma-chain-associated cytokines IL-2, IL-7, and IL-15, while IL-4, another gamma-chain-associated cytokine, is thought to primarily affect T cell quality rather than quantity. Some experiments (Morris SC, Heidorn SM, Herbert DR, Perkins C, Hildeman DA, Khodoun MV, Finkelman FD, Endogenously produced IL-4 nonredundantly stimulates CD8+ T cell proliferation, J Immunol, 2009 Feb 1;182(3):1429-38) reveal that endogenously produced IL-4 is a direct, non-redundant, and potent stimulator of CD8(+) T cell proliferation in Ag- and pathogen-induced CD8(+) T cell responses. These stimulatory effects of IL-4 are observed in both BALB/c and C57BL/6 mice and activate both naive and memory/activated phenotype CD8(+) T cells, although the former are stimulated less than are the latter. IL-4 effects are IL-7- and IL-15-independent, but MHC class I-dependent stimulation appears to be required for the mitogenic effect of IL-4 on naive phenotype CD8(+) T cells. Thus, endogenously produced IL-4 is an important regulator of quantitative as well as qualitative aspects of T cell immunity. IL-4 receptor (IL-4R)-deficient CD8+ T cells specific for the circumsporozoite protein of Plasmodium yoelii develop a severely impaired memory response after priming with parasites. Memory CD8+ T cells lacking the IL-4R are unable to establish a stable population residing in non-lymphoid organs, although they develop normally in lymphoid organs. Because memory cells from non-lymphoid organs disappear shortly after immunization, the protective anti-parasitic activity of this T cell response also is lost (Morrot A, Hafalla JC, Cockburn IA, Carvalho LH, Zavala F, IL-4 receptor expression on CD8+ T cells is required for the development of protective memory responses against liver stages of malaria parasites, J Exp Med, 2005 Aug 15;202(4):551-60, Epub 2005 Aug 8). These results demonstrate that IL-4/IL-4R interactions on CD8+ T cells play a critical role in modulating primary response to the hepatic stages of Plasmodium spp. and the development and tissue distribution of memory cells induced by parasite infection/immunization. They also indicate that memory cells residing in non-lymphoid tissues are critical for protective immunity against malaria parasites.

Cancer immunosuppression that favours tumour progression and metastasis evolves by constitution of an immunosuppressive network, which is mediated by several tumour-derived soluble factors (TDSFs), such as prostaglandin E2 (PGE2), interleukin-10 (IL-10), transforming growth factor-β (TGF-β) and vascular endothelial growth factor (VEGF), and which extends from the primary tumour site to secondary lymphoid organs and peripheral vessels (Zou W, Immunosuppressive networks in the tumour environment and their therapeutic relevance, Nat Rev Cancer, 2005;5:263-27; Yang L, Carbone DP, Tumor-host immune interactions and dendritic cell dysfunction, Adv Cancer Res, 2004;92:13-27). Tumour-derived VEGF acts as a strong chemo-attractant that induces immature myeloid cells (iMCs) from the bone marrow into peripheral vessels, where they are recruited to the tumour site through chemokines and chemokine receptors (Kusmartsev S, Gabrilovich Dl, Immature myeloid cells and cancer-associated immune suppression. Cancer Immunol Immunother, 2002;51:293-8). The iMCs, which include immature dendritic cells (iDCs) and macrophages, are functionally and biochemically modulated in the tumour microenvironment into tumour-associated iDCs (TiDCs) and macrophages (TAMs) that are re-circulated to regional lymph nodes, spleen and peripheral vessels for immune evasion. The immunosuppressive iMCs and increased concentrations of reactive oxygen species (ROS) inhibit T-cell activation in tumour-specific immune responses (Kusmartsev S, Nefedova Y, Yoder D, Gabrilovich D,. Antigen-specific inhibition of CD8+ T cell response by immature myeloid cells in cancer is mediated by reactive oxygen species, J Immunol, 2004;172:989-99). Despite the fact that tumour cells generate pro-inflammatory conditions, as the production of prostaglandins as PGE2, the immune cells are regulated in an anti-inflammatory environment, which is mediated by impaired clearance of apoptotic cells by macrophages during the turnover of tumour cells. Although apoptotic cells are removed by phagocytosis of macrophages through the interaction of phosphatidylserine (PS) and phosphatidylserine receptor (PSR) (Fadok VA, Bratton DL, Rose DM, Pearson A, Ezekewitz RA, Henson PM, A receptor for phosphatidylserine-specific clearance of apoptotic cells, Nature, 2000;405:85-90; Li MO, Sarkisian MR, Mehal WZ, Rakic P, Flavell RA, Phosphatidylserine receptor is required for clearance of apoptotic cells, Science, 2003;302:1560-3), the soluble form of PS, sPS, can interact with the PSR in macrophages and DCs, resulting in the promotion of an anti-inflammatory response by the released mediators IL-10, TGF-β and in Mph inhibition by prostaglandin E2 (PGE2) relapse, which inhibit subsequent activation of DCs and T cells (Kim R, Emi M, Tanabe K, Cancer cell immune escape and tumor progression by exploitation of anti-inflammatory and pro-inflammatory responses, Cancer Biol Ther, 2005;4:924-33). The impaired clearance of apoptotic cells induces anti-DNA antibodies to self-antigens that lead to a pseudo-autoimmune status, which provokes a pro-inflammatory response in tumour progression (Kim R, Emi M, Tanabe K, Cancer cell immune escape and tumor progression by exploitation of anti-inflammatory and pro-inflammatory responses, Cancer Biol Ther, 2005;4:924-33). The increased concentration of autoantibodies and iDCs can induce the production of CD4+ CD25+ regulatory T cells (Tregs) that inhibit T-cell function. Thus, it is likely that cancer immunosuppression is produced by TDSF-induced iMCs, an anti-inflammatory response to immune cells triggered by a defective apoptotic cell clearance, and increased concentration of Treg cells.

Systemic lupus erythematosus (SLE) is a prototype of autoimmune disease. It is characterized by defective tolerance to self-antigens and increased concentration of autoantibodies to ribonucleoproteins, double-stranded DNA (dsDNA) and other cellular constituents, which provoke pro-inflammatory responses (Deshmukh US, Gaskin F, Lewis JE, Kannapell CC, Fu SM, Mechanisms of autoantibody diversification to SLE-related autoantigens, Ann N Y Acad Sci, 2003;987:91-8). Given that the complements C1q, C3 and C4 are involved in the recognition of apoptotic cells for phagocytosis by macrophages, the hereditary deficiency of these complement factors in SLE causes impaired clearance of apoptotic cells, which promotes the production of anti-DNA antibodies (Taylor PR, Carugati A, Fadok VA et al., A hierarchical role for classical pathway complement proteins in the clearance of apoptotic cells in vivo, J Exp Med, 2000;192:359-66; Bijl M, Reefman E, Horst G, Limburg PC, Kallenberg CG, Reduced uptake of apoptotic cells by macrophages in systemic lupus erythematosus (SLE): correlates with decreased serum levels of complement, Ann Rheum Dis, 2006;65:57-63). Indeed, the impaired clearance of apoptotic cells plays a critical role in producing autoimmune disease (Hanayama R, Tanaka M, Miyasaka K, Aozasa K, Koike M, Uchiyama Y, Nagata S, Autoimmune disease and impaired uptake of apoptotic cells in MFG-E8-deficient mice, Science, 2004;304:1147-50). The accumulated autoantibodies stimulate a pro-inflammatory response in the cells which leads to tissue damage. Nevertheless, the production of autoantibodies cannot be inhibited by Treg cells because they are functionally deficient (Wraith DC, Nicolson KS, Whitley NT. Regulatory CD4+ T cells and the control of autoimmune disease, Curr Opin Immunol, 2004;16:695-701). Concerning the clinical situation associated with a link between cancer and autoimmune disease, recent reports indicate that several autoimmune paraneoplastic syndromes, which are distinct from autoimmune diseases, associated with the production of autoantibodies, were observed in various types of cancer patients (Solans-Laque R, Perez-Bocanegra C, Salud-Salvia A, Fonollosa-Pla V, Rodrigo MJ, Armadans L, Simeon-Aznar CP, Vilardell-Tarres M, Clinical significance of antinuclear antibodies in malignant diseases: association with rheumatic and connective tissue paraneoplastic syndromes, Lupus, 2004;13:159-64; Sommer C, Weishaupt A, Brinkhoff J, Biko L, Wessig C, Gold R, Toyka KV, Paraneoplastic stiff-person syndrome: passive transfer to rats by means of IgG antibodies to amphiphysin, Lancet, 2005;365:1406-11).

During the past two decades, several modalities for cancer immunotherapy have been used, and some considerable developments have been observed in the discovery of tumour antigens and tumour-associated antigens, which induce tumour-specific immune responses. These antigens are crucial for the success of the emerging cancer vaccines. Nevertheless, the results of clinical trials on cancer vaccination are not satisfactory (Rosenberg SA, Yang JC, Restifo NP, Cancer immunotherapy: moving beyond current vaccines, Nat Med, 2004;10:909-15). The reason for the disappointing results may be one of several factors involved in tumour immune evasion. However, given that a cancer immunosuppressive network initiated from the primary tumour site produces immunological ignorance and tolerance in step with tumour progression, it is unlikely that immunotherapy alone would be able to disrupt the tolerance and ignorance in advanced cancer. Most of the cases given immunotherapy have failed previous treatment with anticancer drugs, and the immunosuppressive network is established. Rather, cancer immunotherapy should be used as a primary treatment targeting a reduced number of cancer cells or residual cells in combination with surgery and chemo-radiation. Removal of the primary site followed by disruption of the immunosuppressive network is necessary and critical for improving the tumour immune response in cancer patients. Indeed, neo-adjuvant chemotherapy for breast cancer (Demaria S, Volm MD, Shapiro RL, Yee HT, Oratz R, Formenti SC, Muggia F, Symmans WF, Development of tumor-infiltrating lymphocytes in breast cancer after neoadjuvant paclitaxel chemotherapy, Clin Cancer Res, 2001;7:3025-30) and neo-adjuvant chemo-radiation therapy for cervical cancer (Fattorossi A, Battaglia A, Ferrandina G, Coronetta F, Legge F, Salutari V, Scambia G, Neoadjuvant therapy changes the lymphocyte composition of tumor-draining lymph nodes in cervical carcinoma, Cancer, 2004;100:1418-28) showed tumour-infiltrating lymphocytes and an enhancement of the immune competency of tumour-draining lymph nodes in which induction of massive tumour cell death might be a trigger for provoking a tumour-specific immune response. The effectiveness of BCG or BCG-CWS (cell-wall skeleton fraction) for the cancer immunotherapy in patients was shown in several clinical trials. On the action mechanism of BCG on host immune cells, has been evidenced that dendritic cells and macrophages express two sorts of receptors, Toll-like receptors, TLR-2 and TLR-4, and a putative binding receptor for BCG-CWS, whose signalling pathways lead to a sufficient antigen-presenting state in the activation of the innate immune system (Nadler R, Luo Y, Zhao W, Ritchey JK, Austin JC, Cohen MB, O'Donnell MA, Ratliff TL, Interleukin 10 induced augmentation of delayed-type hypersensitivity (DTH) enhances Mycobacterium bovis bacillus Calmette-Guérin (BCG) mediated antitumour activity, Clin Exp Immunol, 2003 Feb;131 (2):206-16). Intra-vescical BCG therapy, for example, is effective in the treatment of superficial bladder cancer. Both clinical and experimental results suggest a role for cytokines and delayed-type hypersensitivity (DTH) in BCG-induced anti-tumour immunity. In animal models, some authors characterized the modulatory effects of BCG on bladder cytokine expression and determined the relationship between DTH and BCG anti-tumour activity. Mice, instilled through a catheter intra-vescically with BCG, showed bladder tissue RNA and urine elevated levels of IFN-gamma and TNF-alpha, the two major cytokines associated with DTH (Nadler R, Luo Y, Zhao W, Ritchey JK, Austin JC, Cohen MB, O'Donnell MA, Ratliff TL, Interleukin 10 induced augmentation of delayed-type hypersensitivity (DTH) enhances Mycobacterium bovis bacillus Calmette-Guérin (BCG) mediated antitumour activity, Clin Exp Immunol, 2003, Feb;131(2):206-16). IL10, an important down-regulator of DTH, was also induced by BCG. Constitutive levels of IL4 and IL5 were observed, and IL4 is slightly also modulated by BCG; as previously described, this is an important passage for CD8+ lymphocytes activation. Similar results were observed in the kinetic analysis of urinary cytokines in patients after intra-vescical BCG therapy. Production of Th1 (T helper type 1) cytokines (IFN-gamma, IL2 and IL12) preceded that of the Th2 (T helper type 2) cytokine IL10 and IL4 (Azuma I, Seya T, Development of immunoadjuvants for immunotherapy of cancer, Int Immunopharmacol, 2001 Jul;1 (7):1249-59). A tendency toward higher ratios of IFN-gamma versus IL10 for BCG responders also was observed. In animal studies the absence of IL10 abrogated either by antibody inhibition or the use of genetically modified, IL10 deficient (IL10-/-) mice resulted in enhanced DTH responses. Under conditions of enhanced DTH, a significant enhancement in anti-tumour activity was observed. These data demonstrate the anti-tumour activity of intra-vescical BCG therapy that acts as DTH and mononuclear infiltration great stimulator (Azuma I, Seya T, Development of immunoadjuvants for immunotherapy of cancer, Int Immunopharmacol, 2001, Jul;1(7):1249-59).

Immunotherapy with intra-vescical ECG was first described in 1976 by Morales et al., Intracavitary bacillus Calmette-Guerin in the treatment of superficial bladder tumors, J Urol, 167: 891, 2002. Bohle, et al., Intravescical bacillus Calmette-Guerin versus mitomycin C for superficial bladder cancer: a formal meta-analysis of comparative studies on recurrence and toxicity, J Urol, 169: 90, 2003, reported the effectiveness in tumor ablation of carcinoma in situ and prophylaxis against tumor recurrence. Dovedi SJ, Kirby JA, Atkins H, Davies BR, Kelly JD, 2005, cyclooxygenase-2 inhibition: a potential mechanism for increasing the efficacy of bacillus calmette-guerin immunotherapy for bladder cancer, the Journal of Urology, Vol. 174, 332-337, disclosed the ECG therapy for treating high risk, non-invasive urothelial carcinoma and carcinoma in situ of the bladder and the effect of in situ administration of ECG on the local T-helper type 1 lymphocytes immune response was investigated. Dovedi SJ, Kirby JA, Davies BR, Leung H, Kelly JD, 2008, Celecoxib has Potent Antitumour Effects as a Single Agent and in Combination with BCG Immunotherapy in a Model of Urothelial Cell Carcinoma, European Urology, 54,621 - 630, disclosed the intra-vescical immunotherapy with BCG and celecoxib for the treatment of high-grade non-muscle-invasive urothelial cell carcinoma (UCC) and carcinoma in situ (CIS); the effect of the combination is investigated in a ortho-topic model of urothelial carcinoma in immuno-competent mouse. BCG is administered by direct instillation into the bladder and acts coming into contact with cancer cells inducing a local response of the mucosa, reducing the invasiveness and metastatic potential of neoplastic cells. The results on the therapy with intra-vescical ECG in bladder/urothelial carcinoma showed that BCG local instillation stimulates in situ lymphocytic infiltration within the cancer area of bladder, while celecoxib retains his anti-tumor efficacy.

### Technical problem

The present invention represents an important innovation in the therapeutic approach of many important diseases.

The aim of the invention is the expansion of the immuno-modulating activity of BCG (Bacillus Calmette Guerin) which is a non-invasive non-pathogen mutate strain, used for a long time for the Tuberculosis's prophylaxis. BCG exalts the cell-mediated response and it is usually used as "alive" adjuvant in many vaccines. It confers host protection through increasing the phagocytosis ability of the macrophages by the way of a great synthesis of nitrogen monoxide (NO) and a great production of cytokines as IFN-γ and IL-2, determining lymphocytes and macrophages recruitment. The limits of action of BCG consist in the fact that BCG administration operates inevitably stimulating a phlogosis. Therefore, If on one side BCG exalts cell-mediated response, on the other side it indirectly induces the production of PGE2 which down-rule the system. It is clear from the state of the art that the activation of the innate immune system is a prerequisite for the maturation of dendritic cells (DC) and macrophages (Mph) followed by the clonal expansion of lymphocytes and the targeting of cells expressing "non-self" antigens. Microbes as other "noxae", being something exerting a harmful effect on the body, have a component being competent to activating DC/Mph for antigen presentation. This component is named as adjuvant, or recently defined as "pathogen-associated molecular pattern-PAMP" or modulin based on its molecular identification. It has been proposed that DC/Mph express receptors for PAMP, whose signalling pathways lead to a sufficient antigen (Ag)-presenting state. In bacterial infection, Toll-like receptors (TLRs) and uptake receptors participate to DC maturation and Mph activation. Likewise, in infections of a number of viruses or protozoa, receptors having short consensus repeats (SCR), immunoglobulin-like domains or chemokine receptor-like motifs induce functional modulation of DC/Mph. In immune therapy for cancer, in malaria and Leishmania therapy, the primary activation of innate system is essential for tumour Ag-specific T cell augmentation. Cancer cells or protozoa express tumour-associated Ag but barely co-express PAMP; this situation does not allow for the activation of innate immune responses. Moreover, the inadequate CD8 T cell response is due to the detrimental effect of PGE2 on macrophages activity, in terms of antigen binding and NO production.

It is also known from the prior art the treatment of bladder carcinoma by intra-vescical administration of BCG also in combination with celecoxib resulting in a limited local stimulation of lymphocytic infiltration within the cancer area. A systemic effect of the combination in diseases different bladder cancer and carcinomas which are non-invasive neoplasia with a nipple-like structure (papillary) and a peculiar biological behaviour has never been demonstrated, therefore the data obtained in bladder cancer cannot be translated to other kind of much more aggressive cancer and to immuno-based disease with a systemic character.

The above technical problem is solved by combining Bacillus Calmette Guerin (BCG) with at least one anti-COX-2 selective molecule and/or at least one COX-2 preferential NSAID (FANS) as in the claims.

This combination can be used for effectively preventing and/or treating the pathologies being related to a loss of a macrophage function and deficiency of cell-mediated immunity.

Anti-COX-2 selective molecules and COX-2 preferential NSAIDs (FANS) according to the claims show strong selective inhibiting power towards the inducible form of COX enzyme (indicated as COX-2).

The expression of COX enzyme, allows the synthesis of some prostaglandins, particularly the PGE2, beginning from the arachidonic acid. In turn prostaglandins, particularly PGE2, exalts some activities of the inflammatory process, as the neo-angiogenesis, the production of tromboxans, the coagulation pathway, the vasopermeability and the vasoconstriction; depresses macrophage phagocytosis, the ability of presentation of the antigen in the context of the major histocompatibility complex of class I and II (MHCl, and II) and then, the function of T lymphocytes CD8+, and NKs cells, the production of cytokines typical of the Th1 response (IFN-γ, IL-2, IL-6, IL-12, etc.), exalting those of the Th2 response (TGF-β, IL-10, IL-4, etc.).

The combined action of BCG with and Anti-COX-2 selective molecules and/or COX-2 preferential FANS give a notable expansion of the cytotoxic and cell-mediate effect with an increase of the activity of the immunity system towards all those pathologies that require a mechanism of cell-mediated response, prevalently of Th1 immuno-phenotype.

In the combination of the present invention, the effect of BCG administration on Mph activity in terms of antigen-binding, nitric oxide production and antigen killing is enhanced by the Anti-COX-2 selective molecules and/or COX-2 preferential FANS. In addition the inadequate CD8 T cell response, due to the detrimental effect of PGE2 on macrophages activity, in terms of antigen binding and NO production, is restored by Anti-COX-2 selective molecules and/or COX-2 preferential FANS.

The combination of the present invention induces IL-12 and IL-4 response operate by BCG stimulation, that enhance a good Th1 and CD8+ activity and the anti-PGE2 activity restores the CD8+ T cell responses generated by sporozoites, neoplastic cells, fungi or bacteria, increasing protection against re-infection or tumour recurrence.

Therefore the combination of the present invention restores a correct cell-mediated response, in particular an early NK and CD8+ lymphocytes response, in all neoplastic condition in which a strong antigenic component is produced by neoplastic cells, such as in melanoma, injection site sarcoma, some mucins secreting adenocarcinomas.

The inhibition of PGE2, trough Anti-COX-2 selective molecules and/or COX-2 preferential FANS enhance the activity of phagocytosis of DC and Mph with strong antigen presentation and CD4+ (Th1), CD8+ recruitment, associated also with strong NKs activation.

Despite of the fact that the prior art discloses the combination of BCG locally administered and celecoxib in the treatment of bladder cancer/carcinoma, the person skilled in the field would not be incentivized to administer systemically Bacillus Calmette Guerin (BCG) with at least one anti-COX-2 selective molecules and/or at least one COX-2 preferential FANS as in the present invention.

The results in the Bladder cancer did not show any systemic effect was not showed, because the direct instillation into the bladder mucosa acts on the cancer cells and leads merely to a localised response of the mucosa not affecting the cell-mediated systemic immune response.

Moreover, the data reported on the Immunotherapy with intra-vescical ECG are strictly related to Bladder carcinoma which is a non-invasive neoplasia with a nipple-like structure (papillary) remaining on the surface of the mucosa, usually treated locally or by chirurgical ablation.

The systemic synergistic effect of the claimed combination was unexpected because the effects of Bacillus Calmette Guerin (BCG) exerted on the immune system are opposite to those exerted by anti-COX-2 selective molecules and COX-2 preferential FANS. Bacillus Calmette Guerin (BCG) was designed to enhance host reactivity towards antigens to produce an exacerbated immune response leading to an inflammatory response. On the contrary, anti-COX-2 selective molecules and COX-2 preferential FANS block the inflammatory cascade.

In the present invention, the synergistic effect of the systemically administered combination of Bacillus Calmette Guerin (BCG) with anti-COX-2 selective molecules and/or COX-2 preferential FANS leads to a systemic controlled inflammatory response due to the effect of anti-COX-2 selective molecules and COX-2 preferential FANS counteracting the negative effect of Bacillus Calmette Guerin (BCG).

### Object of the Invention

The above technical problem is solved by the objects of the present invention.

Object of the present invention is the combination of Bacillus Calmette Guerin (BCG) with at least one anti-COX-2 selective molecule and/or at least one COX-2 preferential NSAID (FANS) according to the claims.

Object of the present invention is the systemic administration of the combination of Bacillus Calmette Guerin (BCG) with at least one anti-COX-2 selective molecules and/or at least one COX-2 preferential NSAID (FANS) according to the claims.

Another object of the present invention is the use of the combination of Bacillus Calmette Guerin (BCG) with at least one anti-COX-2 selective molecules and/or at least one COX-2 preferential NSAID (FANS) according to the claims for the prevention or treatment of pathologies related to the loss of macrophages functions and deficiencies of cell-mediated immunity.

A further object of the present invention is the use of the above combination for the preparation of pharmaceutical composition by adding suitable pharmaceutically acceptable diluents and/or adjuvants and/or excipients and/or vehicles according to the claims.

The pharmaceutical composition for the prophylactic use may be under the form of a vaccine or a drug for strengthening the immune system.

Bacillus Calmette Guerin (BCG), anti-COX-2 selective molecules and/or COX-2 preferential FANS can be administered together, separately and consequently.

Also disclosed is a kit of parts, for unitary, separate or sequential administration, comprising at least one dose of Bacillus Calmette Guerin (BCG), at least one dose of anti-COX-2 selective molecules and/or COX-2 preferential FANS, instructions for administering to the subject the dose.

It is still another object of the present invention the use of the combination of Bacillus Calmette Guerin (BCG) with at least one anti-COX-2 selective molecules and/or at least one COX-2 preferential NSAID (FANS) according to the claims in the treatment of pathologies related to the loss of macrophages functions and deficiencies of cell-mediated immunity by administration once a week for 5 times (loading phase), followed by administration once a month for a year (maintenance phase).

The anti-COX-2 selective molecules are selected from the group consisting of celecoxib, deracoxib, etoricoxib, firocoxib, firecoxib, lumiracoxib, mavacoxib, parecoxib, robenacoxib, rofecoxib, valdecoxib and vaxamine.

The COX-2 preferential FANS are selected from the group consisting of meloxicam, nimesulide, tolfenamic, carprofen, etodolac, nabumetone.

The pathologies related to the loss of macrophages functions and deficiencies of cell-mediated immunity are selected from the group consisting of Leishmaniasis, Vaccine Associated Feline Sarcoma, feline injection site sarcoma (FISS) Feline Infectious Peritonitis, dog idiopathic inflammatory bowel disease, human idiopathic inflammatory bowel disease, Feline Diarroic Syndromes, Malaria, Tuberculosis, Allergic Encephalitis, Atopic Dermatitis, HIV/AIDS, Crohn disease, Ulcerative colitis, Psoriasis, Feline Chronic lymphocytic plasmacytic gingivostomatitis, Feline odontoclastic resorptive lesions (FORLs), human Inflammatory Mammary Carcinoma, dog Inflammatory Mammary Carcinoma, Metastatic Melanoma, Avian Aspergillosis, Feline Squamous Carcinoma, Toxoplasmosis, Immune Complex Diseases, Avian Proventricular Dilatation Disease (PDD), Multiple Sclerosis, Canine demodicosis, Canine Systemic Lupus, Canine Leucocyte Adhesion Deficiency / Canine Granulocytopathy Syndrome, Combined Immunodeficiency, Complement (C3) Deficiency, Cyclic Haematopoiesis of Grey Collies, Canine Growth hormone deficiency, IgA Deficiency, Lethal Acrodermatitis in Bull Terriers, Pelger-Huet Anomaly, Feline Immunodeficiency Virus, Feline viral leucosis, Psittacine Beak and Feather Disease (PBFD), Polyomavirus infections, Secondary or acquired immunodeficiency syndromes.

The concentration of the single components in the combination and in the pharmaceutical composition and kits thereof is not critical for obtaining the desired properties.

The dosages of the dosage regimens can be determined by the person skilled in the art.

Preferably the dosage of Bacillus Calmette Guerin (BCG) is in the range of 0,01 -1 ml for mammals, including humans, dogs, cats and laboratory animal models, such as mice, mouse and More preferably, the dosage regimen of Bacillus Calmette Guerin (BCG) is 0,1 ml/head for mammals, including humans, dogs, cats and laboratory animal models, such as mice, mouse and Preferably the dosage regimen of anti-COX-2 selective molecules and/or COX-2 preferential FANS is from 1 mg/kg to 5 mg/kg for mammals, including humans, dogs, cats and laboratory animal models, such as mice, mouse and rat.

More preferably, the dosage regimen of anti-COX-2 selective molecules and/or COX-2 preferential FANS is 2 mg/kg or 5 mg/kg.

A further object of the present invention is a composition for treating mammal patients suffering from a disease related to the loss of macrophages functions and deficiencies of cell-mediated immunity by following the administration protocol wherein:
the protocol provides a weekly administration for 5 times (loading phase), followed by monthly administrations for the next year (maintenance phase).

Preferably the dosage regimen of Bacillus Calmette Guerin (BCG) is in the range of 0,01 -1 ml for mammals, including humans, dogs, cats and laboratory animal models, such as mice, mouse and
More preferably, the dosage regimen of Bacillus Calmette Guerin (BCG) is 0,1 ml/head for mammals, including humans, dogs, cats and laboratory animal models, such as mice, mouse and
Preferably the dosage regimen of anti-COX-2 selective molecules and/or COX-2 preferential FANS is from 1 mg/kg to 5 mg/kg for mammals, including humans, dogs, cats and laboratory animal models, such as mice, mouse and rat.

More preferably, the dosage regimen of anti-COX-2 selective molecules and/or COX-2 preferential FANS is is 2 mg/kg or 5 mg/kg.

The anti-COX-2 selective molecules and/or COX-2 preferential FANS are given from two days before BCG injection till two days after, for a total time of four days.

In the same way of BCG vaccine, this protocol provides weekly administration for 5 times (for an overall number of 20 administrations means 4 days per week for 5 times), followed by monthly administrations for the next year (for an overall number of 40 administrations means 4 days per month for 10 months).

the anti-COX-2 selective molecules and/or COX-2 preferential FANS are administered weekly for 5 weeks, 4 days per week; from 2 days before BCG inoculation till 2 days after, followed by a suspension for 4 weeks. From the 10^{th} week, the anti-COX-2 selective molecules and/or COX-2 preferential FANS is administered monthly, 5 days per month; from 2 days before BCG inoculation till 2 days after, up to one year of the therapy, and the anti-COX-2 selective molecules and/or COX-2 preferential FANS are given from two days before BCG injection till two days after, for a total time of four days. The protocol provides weekly administration for 5 times, for an overall number of 20 administrations, being 4 days per week for 5 times, followed by monthly administrations for the next year, for an overall number of 40 administrations, being 4 days per month for 10 months.

Further features and characteristics of the present invention would be clear from the following detailed description and examples, with reference to the figures wherein:

### Brief description of the drawings

Figure 1 shows a flowchart of canine leishmaniasis experimental treatment clinical study (example1).

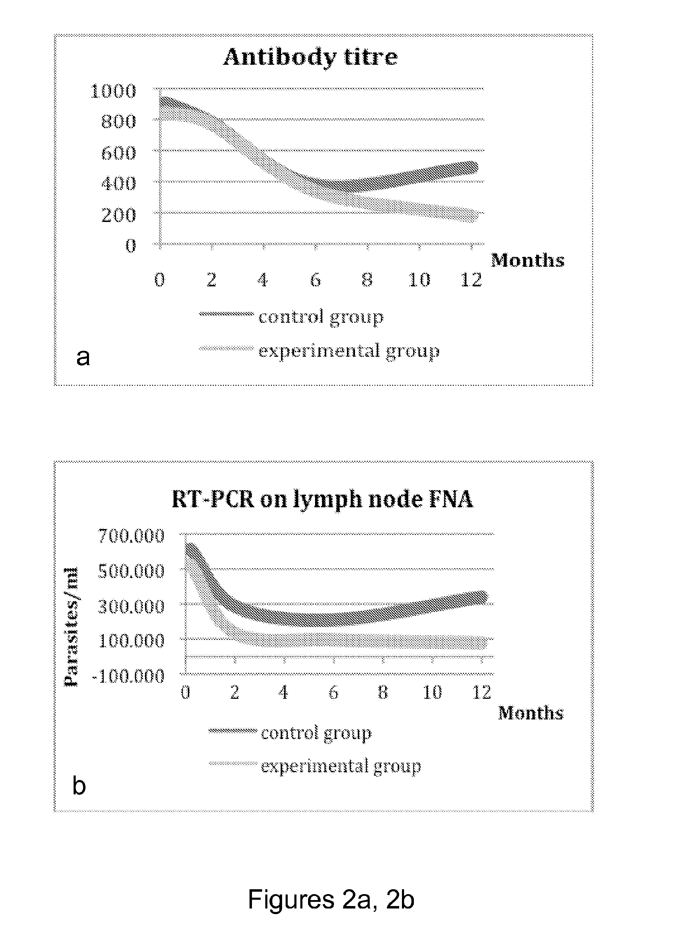

Figure 2A refers to example 1 and shows the trend of antibody of the two groups of dogs: a similar trend of Ab titre in the first period of the trial (until six months). After this period the group B (Glucantime Group) the antibody title increase.
Figure 2B refers to example 1 and shows PCR results with a similar trend of figure 2A, after two months of treatment the levels of DNA copies is very low or negative in the BCG+antiCOX2 treated dogs, and this trend is maintained also after one year. Dogs treated with Glucantime, after a period of decreased PCR title, evidenced a progressive increase of parasite DNA in lymphnodes and bone-marrow.

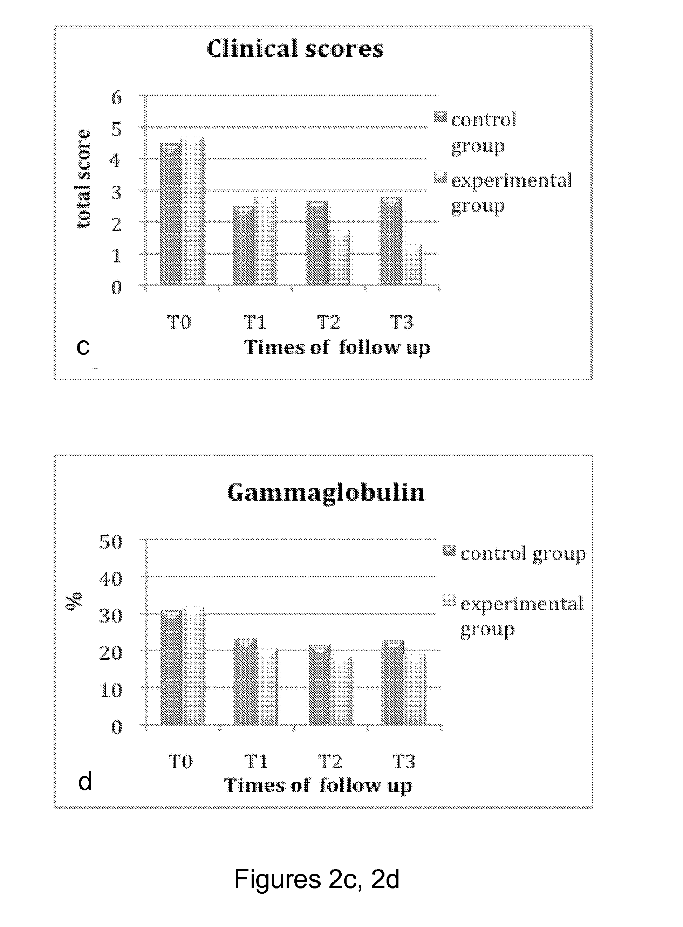

Figure 2C refers to example 1 and shows clinical scores evaluated in the two groups of Dogs of example 1: progressive clinical improvement of Group A at the time of the T2 and T3 follow-up.
Figure 2D refers to example 1 and shows electrophoresis: electrophoresis evidenced a progressive reduction og gamma-globulins levels in sera of dogs beloging to the group A, while in group B after a first period of decreasing trend, the gamma globulins trend increase.

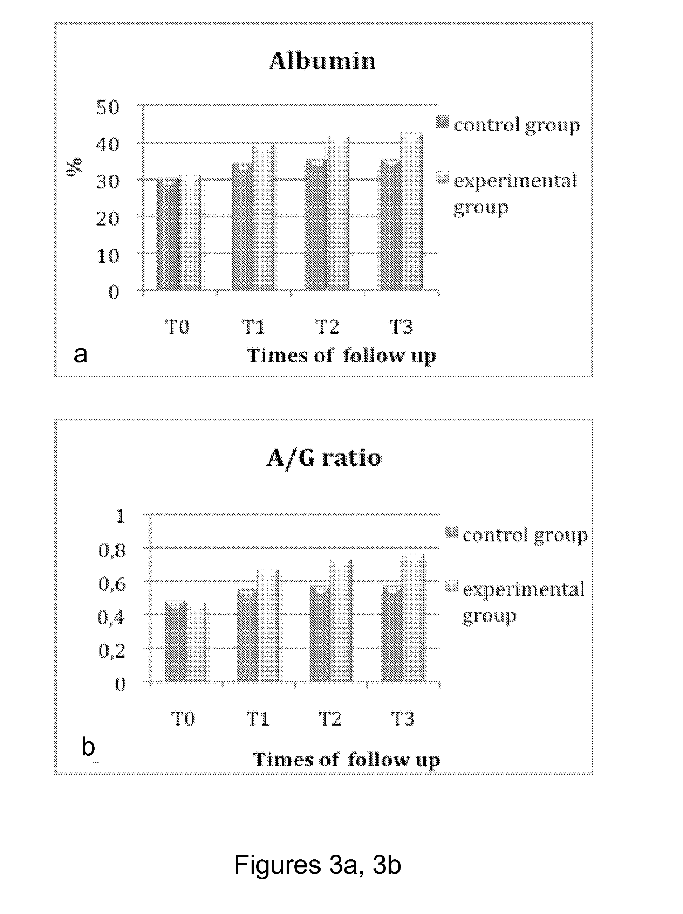

Figure 3A refers to example 1 and shows the serological trend of the albumin.
Figure 3B refers to example 1 and shows the Albumin/Globulin ratio, which are very similar in the two Groups of dogs of example 1, the progressive improvement of Albumin levels and the regularization of A/G ratio in Group A.

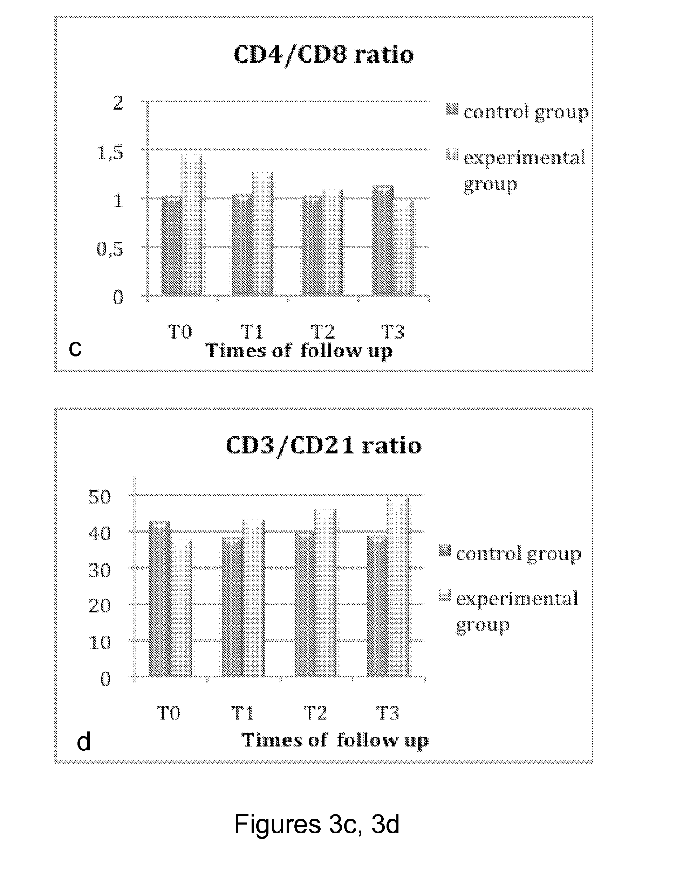

Figure 3C refers to example 1 and shows the CD4/CD8 ratio: progressive increase of CD8 levels and, consequentially, a progressive decrease of CD4/CD8 ratio. The increased level of CD8 is associate with an increased anti-leishmanicidal activity associated to decreased level of Th2 related immunoglobulin production.
Figure 3D refers to example 1 and shows the CD3/CD21 trend: a progressive CD3 increase, related to an enhancement of the cell-mediated immuno-response and a decrease of the humoral response CD21 mediated.

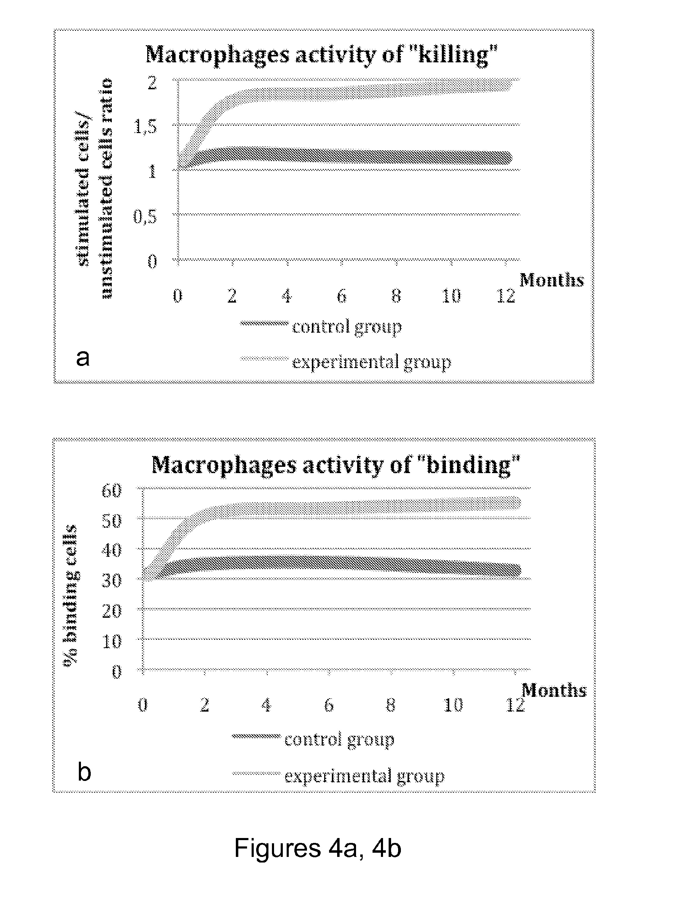

Figure 4A refers to example 1 and shows Macrophages activity: killing activity, measured as levels of NO production and consequently of NBT reduction, indicates a very strong increase of this activity in macrophages of BCG+α-COX2 treated dogs (Group A). The levels of NO production is constantly low in Glucantime treated dogs (Group B).
Figure 4B refers to example 1 and shows the macrophages "binding" of the antigen: similar results are obtained in terms of macrophages "binding" of the antigen. The direct count of macrophages that contained yeast cells, indicates an higher number of phagocytizing cells per 40HPF in Group A to respect Group B.
Fig. 5A refers to example 2 and shows the histological aspect of FISS: the characteristic cell arrangement and the high mitotic index of the tumour (H&E, 20X).
Figure 5B refers to example 2 and shows the strong immunohistochemical expression of COX2 in sarcomatous fibroblasts. (ABC method, Meyer haematoxylin nuclear counterstain, 10X).
Figure 6A refers to example 2 and shows the diffuse immuno-histochemical expression of NF-κBp65 sub-unit in neoplastic cells, the best stained areas of the analyzed tumors showed a surrounding large mononuclear cells reactive infiltrate (ABC method, Meyer haematoxylin nuclear counterstain, 10X).
Figure 6B refers to example 2 and shows high power magnification evidenced some nuclear positivities for NF-κBp65 (arrows). (ABC method, Meyer haematoxylin nuclear counterstain, 20X).
Figure 7A refers to example 2 and shows after SDS-Page migration, proteic profiles of normal and neoplastic fibroblasts, FISS cells differ by the presence of a three proteins, 40.738; 17.538, and 14.689 kDa of weight respectively. In particular FISS cells showed 40.738 kDa and 14.689 kDa proteins (black arrows), but lack of 17.538 kDa protein (grey arrows).
Figure 7B refers to example 2 and shows humoral response of two cats to FISS autologous tumor cells. Western blot analysis of pre-immune sera (T0) and post-immune sera (one yr after excision; T1) from patients receiving the immunomodulating protocol showed that cats had strong reactivity to multiple tumor-specific antigens (arrows).
Figure 8 refers to example 2 and shows histology of a FISS local recurrence after 7 months of surgery in a immunomodulate cat. The pathologic tissue appears characterized by strong perineoplastic lymphoplasmacytic infiltration, strong tumour demarcation and diffuse aspect of granulomatous lesions with large central areas of necrosis (arrows).
Figure 9 refers to example 2 and shows a general overview of follow up in cats enrolled in the study.

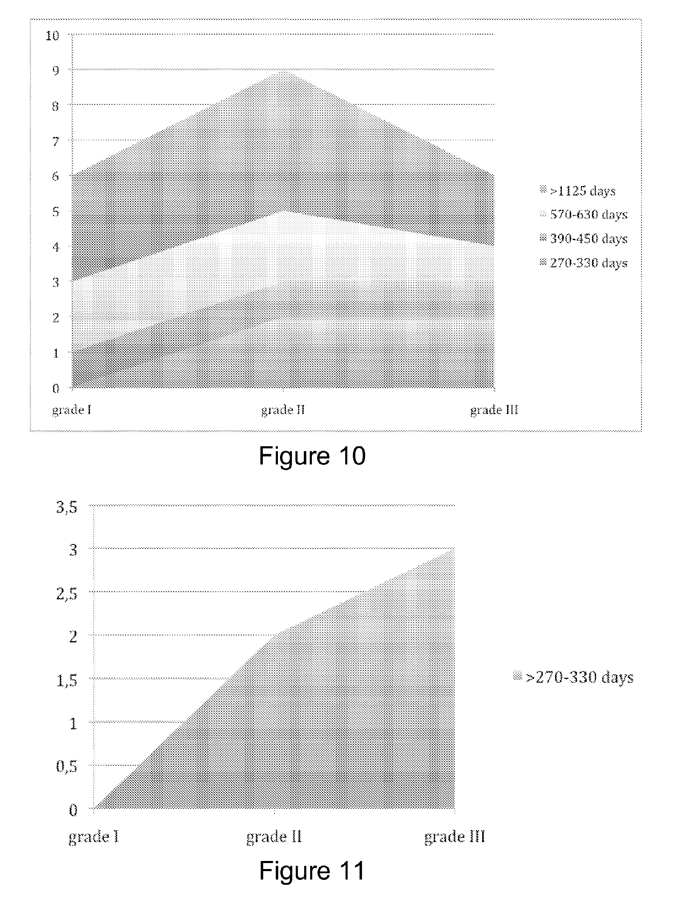

Figure 10 refers to example 2 and shows the distribution of cats free from recurrence in relationship to tumour degree.
Figure 11 refers to example 2 and shows the distribution of cats with recurrence in relationship to tumour degree.

### Detailed description of the invention

### Definitions

Within the content of the present invention Bacillus Calmette Guerin (BCG) means strain of the attenuated live bovine tuberculosis bacillus, Mycobacterium bovis or any fragments thereof capable of exerting the adjuvant effect.

Within the content of the present invention anti-COX-2 selective molecule means any active molecule being capable of inhibiting the enzyme cyclooxygenase-2.

Within the content of the present invention COX-2 preferential FANS means any non-steroidal anti-inflammatory drug being capable of inhibiting the enzyme cyclooxygenase-2.

Within the content of the present invention pathology related to the loss of macrophages functions and deficiencies of cell-mediated immunity means any disease caused by an inadequate activation of the Cell-mediated immunity.

Within the content of the present invention mammal means any member of the mammal class including humans, dogs, cats and any laboratory animal models, such as mice, mouse and rat.

Within the content of the present invention combination means that the active ingredients can be administered together, separately or sequentially.

Within the content of the present invention antibody titer means the amount of antibodies in blood.

Within the content of the present invention RT-PCT means Reverse transcription polymerase chain reaction.

Within the content of the present invention emocytometric essay means complete blood count (CBC), full blood count (FBC) or full blood exam (FBE) or blood panel.

Within the content of the present invention onychogryphosis is a hypertrophy that may produce nails resembling claws or a ram's horn.

Within the content of the present invention respiratory burst means oxidative burst, the release of reactive oxygen species from cells (NBT, PMA, SOD).

Within the content of the present invention biochemical profile means the chemical analysis of the serum portion of the blood.

Within the content of the present invention lymphocytes sub-population means classification of lymphocytes based on structurally or functionally different populations of cells.

An embodiment of the present invention is the combination of Bacillus Calmette Guerin (BCG) with at least one anti-COX-2 selective molecule and/or at least one COX-2 preferential NSAID (FANS) according to the claims.

An embodiment of the present invention is the systemic administration of the combination of Bacillus Calmette Guerin (BCG) with at least one anti-COX-2 selective molecule and/or at least one COX-2 preferential NSAID (FANS) according to the claims.

Another embodiment of the present invention is the use of the combination of Bacillus Calmette Guerin (BCG) with at least one anti-COX-2 selective molecule and/or at least one COX-2 preferential NSAID (FANS) according to the claims.

for the prevention of pathologies related to the loss of macrophages functions and deficiencies of cell-mediated immunity.

Another embodiment of the present invention is the use of the combination of Bacillus Calmette Guerin (BCG) with at least one anti-COX-2 selective molecule and/or at least one COX-2 preferential NSAID (FANS) according to the claims for the treatment of pathologies related to the loss of macrophages functions and deficiencies of cell-mediated immunity.

Bacillus Calmette Guerin (BCG), anti-COX-2 selective molecules and/or COX-2 preferential FANS can be administered together, separately and sequentially.

A further embodiment of the present invention is the use of the above combination for the preparation of pharmaceutical composition by adding suitable pharmaceutically acceptable diluents and/or adjuvants and/or excipients and/or vehicles, for the prevention of pathologies related to the loss of macrophages functions and deficiencies of cell-mediated immunity.

In one preferred embodiment of the present invention the above pharmaceutical composition for the prophylactic use may be under the form of a vaccine or a drug for strengthening the immune system.

A further embodiment of the present invention is the use of the above combination for the preparation of pharmaceutical composition by adding suitable pharmaceutically acceptable diluents and/or adjuvants and/or excipients and/or vehicles, for the treatment of pathologies related to the loss of macrophages functions and deficiencies of cell-mediated immunity.

The skilled person know of such pharmaceutical composition and their preparation.

The pharmaceutical composition may be in the form of solution, suspension, tablets, capsules, aerosol, inhalants, suppositories or the like.

The pharmaceutical composition may be adapted for oral, inhalation, parenteral, intravenous, topical administration, wherein, according to the general skills oral, inhalation, parenteral, intravenous, topical administration are intended as being systemic administration so that the entire body is affected.

Also disclosed is is a kit of parts, for unitary, separate or sequential administration, comprising at least one dose of Bacillus Calmette Guerin (BCG), at least one dose of anti-COX-2 selective molecules and/or COX-2 preferential FANS, instructions for administering to the subject the dose.

In the combination of the present invention and in the pharmaceutical compositions and kit thereof the concentration of the single components are not critical for obtaining the desired effects.

It is still another embodiment of the present invention the use of the combination of Bacillus Calmette Guerin (BCG) with at least one anti-COX-2 selective molecule and/or at least one COX-2 preferential NSAID (FANS) according to the claims.

for the manufacture of a medicament for use in the treatment of pathologies related to the loss of macrophages functions and deficiencies of cell-mediated immunity by administration once a week for 5 times (loading phase), followed by administration once a month for a year (maintenance phase).

In one preferred embodiment of the present invention the anti-COX-2 selective molecules are selected from the group consisting of celecoxib, deracoxib, etoricoxib, firocoxib, firecoxib, lumiracoxib, mavacoxib, parecoxib, robenacoxib, rofecoxib, valdecoxib, vaxamine.

In one preferred embodiment of the present invention the COX-2 preferential FANS are selected from the group consisting of meloxicam, nimesulide, tolfenamic, carprofen, etodolac, nabumetone.

The pathologies related to the loss of macrophages functions and deficiencies of cell-mediated immunity are selected from the group consisting of Leishmaniasis, Vaccine Associated Feline Sarcoma, feline injection site sarcoma (FISS) Feline Infectious Peritonitis, dog idiopathic inflammatory bowel disease, human idiopathic inflammatory bowel disease, Feline Diarroic Syndromes, Malaria, Tuberculosis, Allergic Encephalitis, Atopic Dermatitis, HIV/AIDS, Crohn disease, Ulcerative colitis, Psoriasis, Feline Chronic lymphocytic plasmacytic gingivostomatitis, Feline odontoclastic resorptive lesions (FORLs), human Inflammatory Mammary Carcinoma, dog Inflammatory Mammary Carcinoma, Metastatic Melanoma, Avian Aspergillosis, Feline Squamous Carcinoma, Toxoplasmosis, Immune Complex Diseases, Avian Proventricular Dilatation Disease (PDD), Multiple Sclerosis, Canine demodicosis, Canine Systemic Lupus, Canine Leucocyte Adhesion Deficiency / Canine Granulocytopathy Syndrome, Combined Immunodeficiency, Complement (C3) Deficiency, Cyclic Haematopoiesis of Grey Collies, Canine Growth hormone deficiency, IgA Deficiency, Lethal Acrodermatitis in Bull Terriers, Pelger-Huet Anomaly, Feline Immunodeficiency Virus, Feline viral leucosis, Psittacine Beak and Feather Disease (PBFD), Polyomavirus infections, Secondary or acquired immunodeficiency syndromes.

The dosages of the dosage regimens can be determined by the person skilled in the art.

In one preferred embodiment the dosage regimen of Bacillus Calmette Guerin (BCG) is in the range of 0,01 -1 ml for mammals, including humans, dogs, cats and laboratory animal models, such as mice, mouse and rat.

In one more preferred embodiment the dosage regimen of Bacillus Calmette Guerin (BCG) is 0,1 ml/head for mammals, including humans, dogs, cats and laboratory animal models, such as mice, mouse and rat.

In one preferred embodiment the dosage regimen of anti-COX-2 selective molecule and/or COX-2 preferential FANS is from 1 mg/kg to 5 mg/kg for mammals, including humans, dogs, cats and laboratory animal models, such as mice, mouse and rat.

In one more preferred embodiment the dosage regimen of anti-COX-2 selective molecules and/or COX-2 preferential FANS is is 2 mg/kg or 5 mg/kg.

A further embodiment of the present invention is a method for treating mammal patients suffering from a disease related to the loss of macrophages functions and deficiencies of cell-mediated immunity by following the administration protocol wherein:

Said method provides a protocol of administration comprising weekly administration for 5 times (loading phase), followed by monthly administrations for the next year (maintenance phase).

In one preferred embodiment the dosage regimen of Bacillus Calmette Guerin (BCG) is in the range of 0,01 -1 ml for mammals, including humans, dogs, cats and laboratory animal models, such as mice, mouse and rat.

In one more preferred embodiment the dosage regimen of Bacillus Calmette Guerin (BCG) is 0,1 ml/head for mammals, including humans, dogs, cats and laboratory animal models, such as mice, mouse and rat.

In one preferred embodiment the dosage regimen of anti-COX-2 selective molecules and/or COX-2 preferential FANS is from 1 mg/kg to 5 mg/kg for mammals, including humans, dogs, cats and laboratory animal models, such as mice, mouse and rat.

In one more preferred embodiment the dosage regimen of anti-COX-2 selective molecules and/or COX-2 preferential FANS is is 2 mg/kg or 5 mg/kg.

In one preferred embodiment the anti-COX-2 selective molecules and/or COX-2 preferential FANS is given from two days before BCG injection till two days after, for a total time of four days.

In the same way of BCG vaccine, the protocol provides weekly administration for 5 times (for an overall number of 20 administrations means 4 days per week for 5 times), followed by monthly administrations for the next year (for an overall number of 40 administrations means 4 days per month for 10 months).
Or
the anti-COX-2 selective molecules and/or COX-2 preferential FANS is administered weekly for 5 weeks, 4 days per week; from 2 days before BCG inoculation till 2 days after, followed by a suspension for 4 weeks. From the 10^{th} week, the anti-COX-2 selective molecules and/or COX-2 preferential FANS is administered monthly, 5 days per month; from 2 days before BCG inoculation till 2 days after, up to one year of the therapy, and
the anti-COX-2 selective molecules and/or COX-2 preferential FANS is given from two days before BCG injection till two days after, for a total time of four days. The protocol provides weekly administration for 5 times, for an overall number of 20 administrations, being 4 days per week for 5 times, followed by monthly administrations for the next year, for an overall number of 40 administrations, being 4 days per month for 10 months.

According to the present invention Bacillus Calmette Guerin (BCG) and anti-COX-2 selective molecules and/or COX-2 preferential FANS may be administered in a separate form or in the form of an unitary dosage.

When Calmette Guerin (BCG) and anti-COX-2 selective molecules and/or COX-2 preferential FANS are administered in a separate form said active ingredients are administered in the same moment or in a sequential manner.

A most preferred embodiment of the present invention provides a method for the treatment of leishmaniasis wherein the administration schedule provides a weekly administration for 5 times (loading phase), followed by monthly administrations for the next year (maintenance phase). The BCG dose is always 0,1 ml of the reconstituted vaccine. The injection site should be the distal part of a limb, each time choosing a separate limb on the previous.

In a preferred embodiment is administered Firecoxib at the dosage of 5mg/kg or Robenacoxib employed at the dosage of 2mg/kg. Anti COX2 anti-inflammatory drugs are administered from two days before BCG injection till two days after, for a total time of four days per each BCG intra-dermal administration. The protocol provides weekly administration for 5 times (for an overall number of 20 administrations means 4 days per week for 5 times), followed by monthly administrations for the next year (for an overall number of 40 administrations means 4 days per month for 10 months).

A further most preferred embodiment of the present invention provides a method for the treatment of Feline injection-site sarcomas (FISS) wherein Bacillus Calmette-Guérin (BCG) vaccine is administered by intradermal injection.

The protocol provides a weekly administration for 5 times (loading phase), followed by monthly administrations for the next year (maintenance phase). The protocol provides an intradermal inoculation of 0.1 ml of BCG administered weekly for 5 weeks, associate with administration of anti-COX2 drug at the dose of 1.5mg/kg per os S.I.D., 4 days per week; from 2 days before BCG inoculation till 2 days after, followed by a suspension for 4 weeks. From the 10th week, the protocol (BCG+anti-COX2) is administered monthly. The anti-COX2 is administered, 5 days per month; from 2 days before BCG inoculation till 2 days after, up to one year of the therapy. Anti COX2 is administered from two days before BCG injection till two days after, for a total time of four days. The protocol provides weekly administration for 5 times, for an overall number of 20 administrations, being 4 days per week for 5 times, followed by monthly administrations for the next year, for an overall number of 40 administrations, being 4 days per month for 10 months.

A further most preferred embodiment of the present invention provides the treatment of Feline Infectious Peritonitis (FIP) wherein the protocol provides a weekly administration for 5 times (loading phase), followed by monthly administrations for the next year (maintenance phase). The protocol provides an intradermal inoculation of 0.1 ml of BCG administered weekly for 5 weeks, associate with administration of anti-COX2 drug at the dose of 1.5mg/kg per os S.I.D., 4 days per week; from 2 days before BCG inoculation till 2 days after, followed by a suspension for 4 weeks. From the 10th week, the protocol (BCG+anti-COX2) is administered monthly. The anti-COX2 is administered, 5 days per month; from 2 days before BCG inoculation till 2 days after, up to one year of the therapy. Anti COX2 is administered from two days before BCG injection till two days after, for a total time of four days. The protocol provides weekly administration for 5 times, for an overall number of 20 administrations, being 4 days per week for 5 times, followed by monthly administrations for the next year, for an overall number of 40 administrations, being 4 days per month for 10 months.

A further most preferred embodiment of the present invention provides the treatment of Feline Chronic Gingivostomatitis (FCGS) wherein the protocol provides a weekly administration for 5 times (loading phase), followed by monthly administrations for the next year (maintenance phase). The protocol provides an intradermal inoculation of 0.1 ml of BCG administered weekly for 5 weeks, associate with administration of anti-COX2 drug at the dose of 1.5mg/kg per os S.I.D., 4 days per week; from 2 days before BCG inoculation till 2 days after, followed by a suspension for 4 weeks. From the 10th week, the protocol (BCG+anti-COX2) is administered monthly. The anti-COX2 is administered, 5 days per month; from 2 days before BCG inoculation till 2 days after, up to one year of the therapy. Anti COX2 is administered from two days before BCG injection till two days after, for a total time of four days. The protocol provides weekly administration for 5 times, for an overall number of 20 administrations, being 4 days per week for 5 times, followed by monthly administrations for the next year, for an overall number of 40 administrations, being 4 days per month for 10 months.

A further most preferred embodiment of the present invention provides the treatment of Proventricular Dilatation Disease (PDD) wherein the protocol provides a weekly administration for 5 times (loading phase), followed by monthly administrations for the next year (maintenance phase). The protocol provides an intradermal inoculation of 0.1 ml of BCG administered weekly for 5 weeks, associate with administration of anti-COX2 drug at the dose of 1.5mg/kg per os S.I.D., 4 days per week; from 2 days before BCG inoculation till 2 days after, followed by a suspension for 4 weeks. From the 10th week, the protocol (BCG+anti-COX2) is administered monthly. The anti-COX2 is administered, 5 days per month; from 2 days before BCG inoculation till 2 days after, up to one year of the therapy. Anti COX2 is administered from two days before BCG injection till two days after, for a total time of four days. The protocol provides weekly administration for 5 times, for an overall number of 20 administrations, being 4 days per week for 5 times, followed by monthly administrations for the next year, for an overall number of 40 administrations, being 4 days per month for 10 months.

A further most preferred embodiment of the present invention provides the treatment of Canine Inflammatory bowel disease (IBD) wherein the protocol provides a weekly administration for 5 times (loading phase), followed by monthly administrations for the next year (maintenance phase). The protocol provides an intradermal inoculation of 0.1 ml of BCG administered weekly for 5 weeks, associate with administration of anti-COX2 drug at the dose of 1.5mg/kg per os S.I.D., 4 days per week; from 2 days before BCG inoculation till 2 days after, followed by a suspension for 4 weeks. From the 10th week, the protocol (BCG+anti-COX2) is administered monthly. The anti-COX2 is administered, 5 days per month; from 2 days before BCG inoculation till 2 days after, up to one year of the therapy. Anti COX2 is administered from two days before BCG injection till two days after, for a total time of four days. The protocol provides weekly administration for 5 times, for an overall number of 20 administrations, being 4 days per week for 5 times, followed by monthly administrations for the next year, for an overall number of 40 administrations, being 4 days per month for 10 months.

### Examples

### Example 1 - Leishmaniasis

85 dogs with severe form of leishmaniasis where treated with the following protocol, without any adjunctive drug with leishmanicidal activity. The Bacillus Calmette-Guérin (BCG) vaccine consists of live attenuated Mycobacterium bovis bacteria. It should be kept in refrigeration and transported at +2° to +8°C. It is a freeze-fried vaccine and needs to be reconstituted. A sterile needle and sterile syringe must be used for adding the diluent to the frozen powder and the reconstituted vaccine must be stored at +4°C. Usually, BCG vaccine should be discarded after 6 hours after reconstitution, but this protocol allows to use reconstituted BCG up to 6 months, because it is based on the properties of Mycobacterium bovis and not on its viability. This last one is essential for the first injection only, so at the beginning of the therapy just reconstituted BCG vaccine must be administrate. BCG is given as an intradermal injection, so a 25-27 gauge 5/8" (16 mm) needle is recommended. This protocol provides a weekly administration for 5 times (loading phase), followed by monthly administrations for the next year (maintenance phase). The BCG dose is always 0,1 ml of the reconstituted vaccine. The injection site should be the distal part of a limb, each time choosing a separate limb on the previous. For this experiment Firecoxib employed at the dosage of 5mg/kg and Robenacoxib employed at the dosage of 2mg/kg were used indifferently as specific anti-COX2 inhibitor. Anti COX2 anti-inflammatory drugs were administered from two days before BCG injection till two days after, for a total time of four days per each BCG intra-dermal administration. In the same way of BCG vaccine, this protocol provides weekly administration for 5 times (for an overall number of 20 administrations means 4 days per week for 5 times), followed by monthly administrations for the next year (for an overall number of 40 administrations means 4 days per month for 10 months). The protocol efficacy in this study was evaluated by parasitological and immunological criteria, because no obvious clinical signs of canine Leishmaniasis are observed in infected dogs as long as 14 months after infections. The Control group consisted in 31 dogs treated with a conventional protocol based on S.C. administration of Meglumine antimoniate at the dose of 100mg/kg/SID for 30 days, according to all the official protocols.

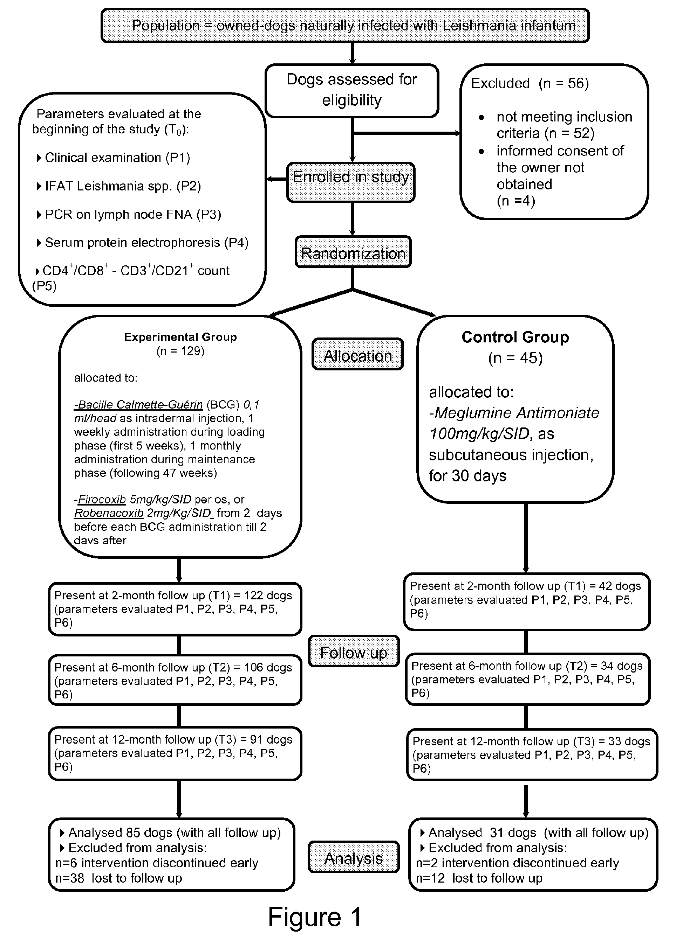

As reported in the Flux Diagram of figure 1 all the enrolled cases were dogs with evident clinical signs of Leishmania infection, in which a serological (IFAT) examination showed high antibody title. All the animals enrolled were also tested for direct detection and quantification of the parasite, by using a PCR analysis performed on lymph-node fine needle aspiration (FNA). The 116 dogs used were allocated into two groups. Group A and B constituted respectively of 85 and 31 dogs with a clinical diagnosis of canine Leishmaniosis (CL), that was subsequently confirmed by positive LN PCR, according to a validated protocol (Ciaramella P, Corona M, 2003, Canine leishmaniasis: clinical and diagnostic aspects, Comp Cont Educ Pract Vet, 25: 358-368; Chulay JD, Bryceson ADM, 1983, Quantitation of amastigotes of Leishmania donovani in smears of splenic aspirates from patients with visceral leishmaniasis, Am J Trop Med Hyg, 32: 475-479), using primers T2 and B4 to amplify a 250-bp fragment of the rRNA gene of Leishmania spp. (Boelaert M, Rijal S, Regmi S, Singh R, Karki B, Jaquet D, Chappuis F, Campino L, Desjeux P, Le Ray D, Koirala S, van der Stuyft P, 2004, A comparative study of the effectiveness of diagnostic tests for visceral leishmaniasis, Am J Trop Med Hyg, 70: 72-77). PCR amplification was carried out according to the guidelines described by Kwok and Higuchi (Ozbel Y, Oskam L, Ozensoy S, Turgay N, Alkan MZ, Jaffe CL, Ozcel MA, 2000, A survey on canine leishmaniasis in western Turkey by parasite, DNA and antibody detection assays, Acta Trop, 74: 1-6), and DEPC-treated H₂O was used, instead of DNA, as a negative control, to exclude the possibility of contamination. All these dogs were admitted to the clinic due to their disease manifestations. In Group A and B the most common CL-compatible clinical signs included peripheral lymphadenomegaly, poor body condition, masticatory muscle atrophy, and exfoliative dermatitis. Of the 116 dogs enrolled in Group A and B, all were serologically positive using indirect immunofluorescence antibody testing (IFAT, cutoff titer ≥1/200). One popliteal or pre-scapular LN was sampled from all dogs with a non-aspiration fine-needle biopsy technique using a 21-gauge needle attached to a 10-mL syringe; smears prepared with the squash method were Giemsa-stained (Soto M, Requena JM, Quijada L, Gomez LC, Guzman F, Patarroyo ME, Alonso C, 1996, Characterization of the antigenic determinants of the Leishmania infantum histone H3 recognized by antibodies elicited during canine visceral leishmaniasis, Clin Exp Immunol, 106: 454-461). Bone marrow was obtained only in 15.5% of enrolled dogs (18 cases): in these cases the sample was obtained by iliac crest puncture with a Rosenthal needle and a 10-mL syringe containing 0.2 mL sterile 3% EDTA/isotonic saline solution. One smear was immediately prepared and stained, as described for LN, with the exception of the doubled staining time (Roura X, Sanchez A, Ferrer L, 1999, Diagnosis of canine leishmaniasis by a polymerase chain reaction technique, Vet Rec, 144: 262-264).

Light microscopy examination of LN and BM smears was carried out blindly (i.e., without knowledge of the animals' Group), in monolayer areas of adequate cellularity and staining quality. Leishmania amastigotes were recognized as round to oval organisms, with a diameter of 2-5 µm, an eccentric nucleus, a kinetoplast with more intense basophilic staining compared with the nucleus, and a visible cellular membrane (Davies CR, Kaye P, Croft SL, Sundar S, 2003, Leishmaniasis: new approaches to disease control, Brit Med J, 326: 377-382). Microscopic examination included a total of 10 to 1,000 oil immersion fields (OlFs, × 1,000), depending on the detection and density of amastigotes; the latter was scored, according to a scale proposed by Chulay and Bryceson, (Piarroux R, Gambarelli F, Dumon H, Fontes M, Dunan S, Mary C, Toga B, Quilici M, 1994, Comparison of PCR with direct examination of bone marrow aspiration, myeloculture, and serology for diagnosis of visceral leishmaniasis in immuno-compromised patients, J Clin Microbiol, 32: 746-749) as 0 (no amastigotes per 1,000 OlFs), +1 (1-9 amastigotes per 1,000 OlFs), +2 (1-9 amastigotes per 100 OlFs), +3 (10-99 amastigotes per 100 OlFs), +4 (10-99 amastigotes per 10 OlFs), +5 (100-999 amastigotes per 10 OlFs), and +6 (more than 1,000 amastigotes per 10 OlFs).

Dogs of Group A and B were monitored at T0, T1, T2, T3 by Flow cytometry. Three millilitres of blood from the cephalic vein was collected from each dog in Heparin-tubes, transported at room temperature and processed 48 hours after collection. For the ex vivo analysis, 30µl of blood was incubated for 30 minutes at room temperature, with 30 µl of each one of the following monoclonal antibodies diluted in Facs diluted solution (10% FCS-supplemented PBS buffer): anti-Thy-1 (Rat-IgG2bclone YKIX337.217) (1:800), anti-CD5 (Rat-IgG2a-clone YKIX322.3) (1:800), anti-CD4 (Rat-IgG2a-clone YKIX302.9) (1:12500), and anti-CD8 (Rat-IgG1-clone YCATE55.9) (1:100) anti CD3 (Serotec) and anti CD21(VMRD). Facs diluted solution was used as negative control. After this period, 2ml of PBS-W (PBS buffer with 0.5% bovine serum albumin and 0.1 % sodium azide) were added to each tube and the mixture was homogenised, and centrifuged at 1300 rpm, at room temperature, for 7 minutes. The supernatants were aspirated and pellets homogenised and added to 60_l of anti-rat FITC conjugate (1:200) (Serotec, UK) except for the Facs diluted cell control. At this time, 4_l of the FITC-labelled mouse anti-human-CD21 (Mouse-IgG1-clone IOB1a) monoclonal antibody (Immunotech Co., Marseille, France) was used in a direct immunofluorescence procedure. All suspensions were homogenised, incubated for 30 minutes at room temperature in the dark and treated with 2ml of the 1/10 diluted lysis solution during vortex homogenisation (Becton & Dickinson, USA). The mixtures were further incubated for 10 minutes at room temperature in the dark and further centrifuged at 1300rpm for 7 minutes. Supernatants were discarded and the pellet-containing tubes were inverted on to absorbent paper. All these procedures were repeated twice after the addition of 2ml PBS. The pellets were homogenised carefully and finally fixed with 300_l of 2.8% formaldehyde-PBS. The relative immunofluorescence of cells was counted in a total of 10,000 events measured in a Becton Dickinson Facscalibur apparatus and further analysed using Windows Multiple Document Interface Flow Cytometry Application (WinMDI) Version 2.8 software. As control, FACS analysis of PBMC of nine normal healthy untreated dogs was also performed.

To visualize the alterations in serum proteins SDS-PAGE was performed. 100 µg of total serum proteins were transferred in the wells of 15 % polyacrylamide gel. The serum electrophoresis was conducted in an electrophoresis solution (pH 8.8) containing 1.46 g acrylamide, 0.04 g biscrylamide, 2.5 ml Tris-buffer (1.5 M), 100 µl sodium dodecyl sulphate (SDS, 10 %), 50µl N,N,N',N'-tetramethylethylene diamine (TEMED, 10 %) in 10 ml. Gels were electrophoresed for 45 min at room temperature at 200 V using Mini-Protean gel apparatus (Bio-RAD, Munich/ Germany) for vertical gels. Electrophoresis was conducted in Tris 25 mM, glycine 195 mM and SDS 0.1 % buffer. Bands were visualised by staining Coomassie Brillant Blue R-250 (Biomol, Hamburg/Germany). The specificity of albumin bands were confirmed with the co-migration of dog serum albumin (A-3184 Sigma, Munich/Germany).

Specific antibody determinations were carried out by obtaining serum samples at the different follow-up and by analysing sera for presence of specific anti Leishmania IgG subsets by ELISA. 96 Maxisorb plates (Nunc) were coated with recombinant Leishmania infantum antigens (4 µg/ml) overnight at 4 °C in PBS pH 7.5;1% SDS. Plates were washed with PBS-0.05% Tween-20 (PBS-T) and blocked with 1% BSA in PBS-T (blocking buffer) for at least 1 hour at 37 °C. Serum samples were diluted in blocking buffer at a dilution 1:80, added in 100 µl/well and incubated (1 hour, 37 °C) Plates were then washed three times. Peroxidase conjugated goat anti-dog IgG1 or sheep anti-dog IgG2 (Bethyl Lab. Inc., Montgomery, TX, USA) was added (1 hour, 37 °C). The IgG1-Fc and IgG2-Fc conjugates were diluted in blocking buffer 1/500 and 1/3000, respectively. Plates were added the peroxidase substrate OPD (Sigma) The reaction was stopped 15 minutes later by addition of 100 µl of 3M H₂SO₄. Absorbance values were read at 495 nm in a FluoStar Galaxy system (BMG Labtechnologies). All determinations were carried out by triplicate and the data presented as the mean of the values corresponding to the group of dogs.

Macrophages activation (binding and killing test) was carried out by isolating canine blood monocytes from peripheral blood of all dogs belonging to the two groups (A and B) at the different times (T0, T1, T2, and T3) according to the Bøyum method. Cells were cultured O-N in media containing PMA, LPS, MD or TP. The abilities of non- and PMA-stimulated macrophages to reduce NBT was estimated according to Park with Szczylik modification. Leishmania amastigote lysates and MPT stimulated macrophages were utilized for RT-PCR analysis in attempt to amplify the TNF-α, IFN-γ, TGF-β, IL-1β, IL-4 and IL-12 interleukins transcripts. Quantitative real-time PCR (qRT-PCR) was applied to validate gene expression profiling. For qRT-PCR 10 ng of cDNA, from both control-healthy dogs and HUC-affected dogs, was used in each reaction. qRT-PCR was carried out in triplicate by using Power SYBR Green PCR Master Mix (Applied Biosystem), in 30 µl reaction volume, by using a 7300 Real-Time PCR System (Applied Biosystem). The 2-ΔΔCt method was applied to analyze the relative changes in expression profiling of interest genes, as already reported (Livak and Schmittgen, 2001). Values were normalized to the internal gap dh gene control. Primers for qRT-PCR were designed through Primer Express software (Applied Biosystem). The abilities of non- and PMA-stimulated monocytes of NBT reduction were estimated by the method according to Park with Szczylik modification (Kaminskaia GO, Abdullaev Rlu. Oxidative metabolism changes in respiratory tract cells of guinea pigs during natural development of experimental tuberculosis and under specific chemotherapy. Probl Tuberk. 1996;(2):33-6). Evaluation of phagocytising macrophages was performed by direct microscopical count of yeast-containing cells in 10-HPM randomly selected fields per culture-slide (Chamberslide-Bioptica).

Statistical analysis was carried out by using chi-square and Fisher exact tests to compare different data obtained from the two groups of dogs (sero-prevalence, clinical signs, Macrophage activity, PCR etc.). Analyses were conducted using SPSS software version 13.5, with a probability (P) value of <0.05 as statistically significant.

Table 1 show the results obtained comparing the different parameters examined in the two groups of dogs (A e B): trend of serological, parasitological, clinical and immunological parameters (mean ± standard deviation) in the two groups of L. infantum infected dogs enrolled in the 52-week randomised clinical trial (with p < 0,05).

**Table 1**

| | | **T₀** | **T₁** | **T₂** | **T₃** |
|---|---|---|---|---|---|
| | | (beginning of therapy) | (2 months) | (6 months) | (12 months) |
| **Antibody titre (****Fig.2A****)** | Control group | 908 ± 1240 | 779 ± 1031 | 378 ± 321 | 493 ± 678 |
| | Experimental group | 847 ± 1425 | 772 ± 1090 | 341 ± 395 | 179 ± 187 |
| **RT**-**PCR on lymph node FNA (****Fig.2B****)** | Control group | 613.228 ± 747.653 | 287.482 ± 434.807 | 210.103 ± 364.909 | 340.367 ± 511.898 |
| Parasites/ml | Experimental group | 524.571 ± 916.787 | 133.743 ± 318.876 | 95.703 ± 309.536 | 78.119 ± 264.608 |
| **Clinical scores (****Fig.2C****)** | Control group | 4,4 ±2 | 2,5 ± 1,9 | 2,7 ±2 | 2,8 ± 2,2 |
| | Experimental group | 4,7 ± 1,5 | 2,7 ± 1,2 | 1,7 ± 0,9 | 1,3 ± 0,9 |
| **Serum γ-globulin (****Fig.2D****) (%)** | Control group | 30,67 ± 7,91 | 23,15 ± 7,46 | 21,56 ± 5,86 | 22,74 ± 7,93 |
| | Experimental group | 31,63 ± 9,51 | 20,28 ± 6,35 | 18,65 ± 5,46 | 18,78 ± 6,17 |
| **Serum albumin (****Fig.3A****)(%)** | Control group | 30,08 ± 9,63 | 34,03 ± 9,5 | 35,22 ± 9,35 | 35,26 ±9,61 |
| | Experimental group | 30,86 ± 9,51 | 39,41 ± 7,94 | 41,51 ± 6,83 | 42,23 ± 6,78 |
| **A/G ratio (****Fig.3B****)** | Control group | 0,48 ± 0,23 | 0,55 ± 0,23 | 0,57 ± 0,23 | 0,57 ± 0,22 |
| | Experimental group | 0,47 ± 0,21 | 0,67 ± 0,21 | 0,73 ± 0,20 | 0,76 ± 0,20 |
| **CD4⁺/CD8⁺ ratio (****Fig.3C****)** | Control group | 1,02 ± 0,48 | 1,04 ± 0,46 | 1,02 ± 0,45 | 1,31 ± 0,58 |
| | Experimental group | 1,44 ± 1 | 1,26 ± 0,9 | 1,08 ± 0,75 | 0,97 ± 0,6 |
| **CD3⁺/CD21⁺ ratio (****Fig.3D****)** | Control group | 42,62 ± 21,24 | 38,13 ± 20,56 | 39,85 ± 17,58 | 38,64 ± 19,54 |
| | Experimental group | 37,45 ± 21,26 | 43,00 ± 22,47 | 45,87 ± 24,29 | 49,52 ± 22,32 |
| **Macrophages activity of killing (****Fig.4A****)** | Control group | 1,1 ± 0,1 | 1,2 ± 0,1 | 1,1 ± 0,1 | 1,1 ± 0,1 |
| | Experimental group | 1,1 ± 0,1 | 1,8 ± 0,3 | 1,8 ± 0,3 | 1,9 ± 0,3 |
| **Macrophages activity of binding (****Fig.4B****)** | Control group | 31,84 ± 12,9 | 34,84 ± 14,2 | 35,42 ± 13,68 | 32,77 ± 12,78 |
| | Experimental group | 31,1 ± 10 | 50,81 ± 11,73 | 53,33 ± 10,97 | 55,13 ± 11,16 |

The results demonstrate differences of both humoral and cellular immune responses between stimulated and un-stimulated dogs (control group treated with a conventional leishmanicidal protocol). The combination of BCG with Firocoxib or Robenacoxib was able to induce a strong and efficacy protective cell-mediated response and the role of PGE2 to prevent this protective mechanism was clarified. It appears to be mainly T-cell-mediated and suggest that anti-Leishmania antibodies play little or no role in protection (Locksley RM and JA Louis, Immunology of leishmaniasis, Curr Opin Immunol, 1992, (4) pp. 413-418; Bogdan C and M Rollinghoff, The immune response to Leishmania: mechanisms of parasite control and evasion, Int J Parasitol, 28 1998 (1), 121-134). Although the clinical manifestations of disease are determined by the species of Leishmania and the host immune response, the outcome of the Leishmania infections seems to be dependent on the regulation of the balance between the Th1/Th2 reactive T-cell populations. Analysis of antibody iso-type responses provides a convenient surrogate marker of Th1 and Th2 CD4+ T cell differentiation (Locksley RM and JA Louis, Immunology of leishmaniasis, Curr Opin Immunol, 1992, (4) pp. 413-418; Bogdan C and M Rollinghoff, The immune response to Leishmania: mechanisms of parasite control and evasion, Int J Parasitol, 28 1998 (1), 121-134). The specific iso-type response against Leishmania infection after protocol administration was studied because determination of antibody iso-type profile in treated dogs provided some indication on the stimulation impact on the subset development. Noteworthy, a significant and exclusive increase in IgG2 antibodies to Leishmania infantum infection has been observed only in immuno-stimulated animals both early after protocol administration and as far as 12 months post start-phase of the protocol. These results appear to support those of several authors indicating that in dogs the differential IgG1/IgG2 response is a better indicator of the outcome of infection than total IgG (Bourdoiseau G, C Bonnefont, E Hoareau, C Boehringer, T Stolle, L Chabanne, Specific IgG1 and IgG2 antibody and lymphocyte subset levels in naturally Leishmania infantum-infected treated and untreated dogs, Vet Immunol Immunopathol, 59,1997,(1-2) 21-30; MJ Day, Immunoglobulin G subclass distribution in canine leishmaniosis: a review and analysis of pitfalls in interpretation, Vet. Parasitol, 147 (2007), 2-8). An explanation is that IgG2 responses are associated to Th1-type responses whereas the IgG1 subtype correlates with Th2-type responses. Moreover, specific IgG2 responses in dogs immuno-stimulated could be persist whereas this response could be totally absent in placebo dogs at each time. Indeed, the control of L. major lesion growth in genetically resistant mice has been clearly associated with the expansion of the Th1-type T-cells producing IFN-γ and IL-2 cytokines, whereas induction of the Th2-type cells secreting cytokines such as IL-4 and IL-10 results in the inability to control Leishmania infection and in disease exacerbation (Kurtzhals JA, AS Hey and A. Jardim et al., Dichotomy of the human T cell response to Leishmania antigens. II. Absent or Th2-like response to gp63 and Th1-like response to lipophosphoglycan-associated protein in cells from cured visceral leishmaniasis patients, Clin Exp Immunol, 96 (1994) (3),416-421; Gaafar A, A Kharazmi and A Ismail et al., Dichotomy of the T cell response to Leishmania antigens in patients suffering from cutaneous leishmaniasis; absence or scarcity of Th1 activity is associated with severe infections, Clin Exp Immunol, 100 (1995) (2), 239-245). Moreover, several studies provided evidence for the existence of a Th1/Th2 dichotomy in the T-cell response also in human cutaneous leishmaniasis (Scott P, IFN-gamma modulates the early development of Th1 and Th2 responses in a murine model of cutaneous leishmaniasis, J Immunol, 147 (1991) (9), 3149-3155; Reiner SL, ZE Wang, F Hatam, P Scott and RM Locksley, TH1 and TH2 cell antigen receptors in experimental leishmaniasis, Science, 259 (1993) (5100), 1457-1460; I Okwor and J. Uzonna, Persistent parasites and immunologic memory in cutaneous leishmaniasis: implications for vaccine designs and vaccination strategies, Immunol Res, 41 (2008), 123-136). Killing of Leishmania parasites by infected macrophages is critical in resolving an infection with this intracellular pathogen. Studies of murine experimental infection models have shown that macrophage activation by T-cell-derived cytokines was required to establish the control of cellular infection and progressive disease. In these cells, Leishmania growth inhibition or killing was mediated by NO production from inducible nitric oxide synthase (iNOS) via the I-arginine NO pathway. Transcription of this enzyme was induced and enhanced by cytokines derived from Th1-type lymphocytes as IFN-γ and other synergic cytokines such as tumor necrosis factor (TNF)-α and/or interleukin (IL)-2. CD4+ T cells, in association with CD8+ T cells, appeared to be essential for the resolution of primary immunity and resistance to re.infection in mice infected with L. donovani and in human L. infantum infection. The role of CD8+ T cells in protection could be mediated by the large amount of IFN-γ that is secreted by these cells. Furthermore, previous observations have clearly emphasized the apparent lack of a clear-cut role of Th1 or Th2 cell activation in either disease cure or non-cure in human visceral leishmaniasis (Locksley RM and JA, Louis, Immunology of leishmaniasis, Curr Opin Immunol, 4 (1992) (4), 413-418; Bogdan C and M Rollinghoff, The immune response to Leishmania: mechanisms of parasite control and evasion, Int J Parasitol, 28 (1998) (1), 121-134). IL-4 has a significant role in the induction of tumor-specific and some, but not all, antiviral and antibacterial CD8+T cells responses. Recently, an essential requirement for IL-4 in vaccine induced CD8+T-responses explained why Leishmania vaccines using strong IFN-γ promoting adjuvants have met only partial success compared with vaccines containing Alum. BCG is a strong IFN-γ secretion stimulator, but in a recent work, (Lastra JM, Galindo RC, Gortázar C, Ruiz-Fons F, Aranaz A, de la Fuente J, Expression of immunoregulatory genes in peripheral blood mononuclear cells of European wild boar immunized with BCG, Vet Microbiol, 2009 Mar 2;134(3-4):334-9, Epub 2008 Aug 29) demonstrated a peak of mRNA levels of IFN-gamma (>15-fold increase) at 5 w post BCG treatment, whereas transcripts for IL-4 showed a peak (>2-fold increase) at 13 wpi in BCG-immunized animals when compared to non-immunized controls. This work demonstrates that BCG inoculation enhanced contemporaneously IFN-g and IL-4 levels, with direct CD8+T and NK cells enrolment. In areas of phlogosis induced by the BCG activity, PGE2 production, strongly depressed by Coxib administration, induces cell-mediate immuno-response defects; associated with increased release of prostaglandin E(2) (PGE(2)) as a result of overexpression of cyclooxygenase (COX)-2 by macrophages and other inflammatory cells. Is demonstrated that Prostaglandin E2 stimulated a dose dependent increase in the levels of IL-10 produced by BCG activated DCs. IL-10 significantly decreased IL-12 production and, indirectly, the macrophages phagocytosis and NO production. On the contrary, the inhibition of PGE2 synthesis by COX2 inhibition favoured the production of IL-12 by BCG activated DC. This potentially will result in the generation of a T-helper type 1, polarized T-cell response that may improve the efficacy of BCG therapy. The above results demonstrates that BCG stimulation together with anti-COX2 treatment protect animals inducing the proliferation of IFN-γ-producing specific T-cells and Th2-IL-4-induced expansion. Increase of IFN-γ results in activation of canine macrophages that produced sufficient amounts of NO to display intracellular Leishmania killing by the I-arginine-NO metabolic pathway; IL-4 BCG-induced levels causes a strong CD8+ and NK cells recruitment, a very effective lymphocytes subsets able to free parasites and intracellular Leishmania destruction. In Leishmania, as in malaria, during acute infection DC migrate and secrete TGF-beta, prostaglandin E2 (PGE2) and IL-10. This cytokines background inhibits the Th1 response and, in particular, the CD8+ T cells response generated against the endo-cellular-stage of the diseases. Inhibition of the activity of TGF-beta and PGE2 restores the CD8+ T cell responses generated by amastigotes or sporozoites, increasing protection against re-infection.

### Example 2 - Feline injection-site sarcomas (FISS)

Because of the complex involvement of various factors in vivo, an animal model of mesenchymal neoplasia of inflammatory origin, the feline injection site sarcoma (FISS) was investigated. Feline injection site sarcoma (FISS) is a particular form of soft tissue tumour associated to a chronic-inflammatory process that probably acts a fundamental role in the tumour pathogenesis. Many analogies are observed between FISS and human nodular fasciitis (NF), a pseudo-sarcomatous lesion, and myxo-inflammatory fibroblastic sarcoma (MFS), another characteristic soft tissues tumour of man. All these lesions are morphologically and clinically very similar, the possible causes proposed and investigated include trauma/inflammation, genetic predisposition, exposure to radiation or certain chemicals, all of which may stimulate the tissues to divide more rapidly than normal. In human cases, as recently described in cat, oncogenes, tumour suppressor genes, and other cellular genetic defects have been identified and these exhibit an association with connective tissue sarcomas. Feline and human lesions are also similar for COX-2 protein expression and for NF-κB pathway activation. These similarities suggest that cat may represent a good model to study inflammatory-induced soft tissue proliferations in man and to investigate the role of the cell-mediated Th1 type and Nk immuno-response in the control of tumour spread and recurrences, and in patients outcome. Sarcomas are very common tumours that arise from the soft connective tissues, that support and surround the organs and other structures of the body in man as in animals. The pathogenesis of sarcomas is as yet unknown. No breed, sex or age predisposition has been demonstrated. A number of factors have been implicated as potential causes of tumorigenesis, and the aetiology may be multifactorial and involve co-carcinogenic interaction (Piarroux R, Gambarelli F, Dumon H, Fontes M, Dunan S, Mary C, Toga B, Quilici M, 1994; Comparison of PCR with direct examination of bone marrow aspiration, myelo-culture, and serology for diagnosis of visceral leishmaniasis in immuno-compromised patients J Clin Microbiol 32: 746-749). Possible causes proposed and under investigation include genetic predisposition, exposure to radiation or certain chemicals, trauma, and inflammation, all of which may stimulate the tissues to divide more rapidly than normal. In human cases oncogenes, tumour suppressor genes, and other cellular genetic defects have been isolated that exhibit an association with connective tissue sarcomas (Roninson IB, Oncogenic functions of tumour suppressor p21Waf1/Cip1/Sdi1: association with cell senescence and tumour-promoting activities of stromal fibroblasts, Cancer letters. 2002. 179 (1); 1-14.). For example, a Neurofibromatosis-1 gene (NF1) codes for the development of diffuse fibrous tumours throughout the body in patients with neurofibromatosis, and these lesions can undergo malignant change (Gutmann DH, Parallels between tuberous sclerosis complex and neurofibromatosis 1: Common threads in the same tapestry, Seminars in Pediatric Neurology, 1998; 5(4); 276-286). Much like other tumours that arise after irradiation, human sarcomas can occur in tissues that have been irradiated for other cancers (Fletcher CD, Krishnan Unni K, Mertens F, Pathology and genetics of tumours of soft tissue and bone. In: Kleihues PM, Sobin LH, editors. World Health Organization Classification of Tumours. 4th edition. Lyon, France: IARC Press; 2002, 10-16, 102-103, 120-126.). The radiation may induce genetic mutations that lead to uncontrolled cell division. Additionally, various environmental or industrial chemicals have been linked to the development of sarcomas, including vinyl chloride and arsenic (Cormier JN, Pollock RE, Soft tissue sarcomas, CA: A Cancer Journal for Clinicians, 2004; 54(2):94-109). Actually the relationship between a certain number of human sarcomatous lesions and trauma or chronic phlogosis is diffusely investigated. The cat represent a good model for the pathogenesis studies of certain type of human sarcomas, particularly sarcomas related to a local chronic inflammation process. In particular Feline Injection Site Sarcoma (FISS) represent a group of clinically aggressive neoplasms typically described in cat, that occur at distinct anatomic locations and that clearly showed an inflammatory nature (McEntee MC, Page RL, Feline vaccine-associated sarcomas, J Vet Intern Med, 2001; 15: 176-182). Many studies demonstrated that the insurgence of FISS is strictly related to anatomic districts used for the administration of injections. The main hypothesis is that vaccination, drug administration or local trauma are associated with the development of a local chronic phlogosis that induces FISS (Macy DW, Hendrick MJ, The potential role of inflammation in the development of post-vaccinal sarcomas in cats, Vet Clin North Am Small Anim Pract, 1996; 26:103-109). The reaction leads to uncontrolled proliferation of fibroblasts and myofibroblasts that in a subset of cats undergo malignant transformation. As demonstrated in some articles, in human patients as in cats, the relationship between trauma, inflammation, and recovery are clearly particular and probably differs from other species by characteristic cellular and molecular events responsible for tumorigenesis (Cormier JN, Pollock RE, Soft tissue sarcomas, CA: A Cancer Journal for Clinicians, 2004; 54(2):94-109). Growth factors are essential for regulation of the cellular events involved in granulation tissue formation and wound healing. When growth factors are added to fibroblast cultures, the cells assume a neoplastic phenotype (Cormier JN, Pollock RE, Soft tissue sarcomas, CA: A Cancer Journal for Clinicians, 2004; 54(2):94-109). Many tumours in humans have been associated with autocrine stimulation (i.e., tumours have receptors for growth factors, which they themselves produce in an abnormal or autonomous fashion). Many oncogenes cause cancer by coding for and causing overexpression of growth factors or their receptors (v-sis codes for platelet-derived growth factor [PDGF]; verb codes for epidermal growth factor [EGF] receptor) (Yarbro JW, Oncogenes and cancer suppressor genes, Seminars in Oncology Nursing, 1992; 8 (1); 30-39). Several studies are focusing on immuno-histochemical identification and localization of growth factors and their receptors in certain types of human traumatic/inflammatory induced sarcoma, as similar studies are conducted in different feline sarcomatous injection-associated lesions (Macy DW, Hendrick MJ, The potential role of inflammation in the development of post-vaccinal sarcomas in cats, Vet Clin North Am Small Anim Pract, 1996; 26:103-109). In our study we have evaluate the possibility to reduce the primary tumour, to prevent the metastatization process and to prevent local recurrences of the tumour in the primary site of insurgence in a population of thirty (Price S, Kahn LB, Saxe N, Dermal and intravascular fasciitis: unusual variants of nodular fasciitis, Am J Dermatopathol, 1993, 15:539-543) FISS-affected cats.

Anti-COX-2 specific antibody were used to evaluate the expression of COX2 enzyme by the tumour cells in 30 cats showing FISS. The majority of neoplastic cells showed elevated levels of COX2 production and, even if some cancer cells do not express COX-2, they were found to have expression of PG receptors and PG-related downstream signaling molecules associated to cell viability and our observation suggests that these cells can be influenced by PGs derived from stromal, normal tissues.

Bacillus Calmette-Guérin (BCG) vaccine consists of live attenuated Mycobacterium bovis bacteria, kept in refrigeration and transported at +2° to +8°C and to be reconstituted. A sterile needle and sterile syringe used for adding the diluent to the frozen powder and the reconstituted vaccine stored at +4°C. BCG is administered by intradermal injection. The protocol provides a weekly administration for 5 times (loading phase), followed by monthly administrations for the next year (maintenance phase). The BCG dose is always 0,1 ml of the reconstituted vaccine. To treat muco-cutaneous tumours, the first application is intra-lesional. Initial BCG dose of 0,1 ml diluted in 1 ml of saline fluid. The solution is used to perform micro-injections (0,05-0,1 ml) around the lesion, both in case of inoperable tumour and in case of post-surgical excision. Following injections are performed on the limbs. To treat visceral tumours and metastasis the injection site is the distal part of a limb, each time choosing a separate limb on the previous. The protocol for FISS treatment consisted in an intradermal inoculation of 0.1 ml of BCG administered weekly for 5 weeks, associate with administration of anti-COX2 drug at the dose of 1.5mg/kg per os S.I.D., 4 days per week; from 2 days before BCG inoculation till 2 days after, followed by a suspension for 4 weeks. From the 10th week, the protocol (BCG+anti-COX2) is administered monthly. The anti-COX2 is administered, 5 days per month; from 2 days before BCG inoculation till 2 days after, up to one year of the therapy. Anti COX2 is administered from two days before BCG injection till two days after, for a total time of four days. The protocol provides weekly administration for 5 times, for an overall number of 20 administrations, being 4 days per week for 5 times, followed by monthly administrations for the next year, for an overall number of 40 administrations, being 4 days per month for 10 months.

In 1974, Prof. E. Lederer and his coworkers, University of Paris, have reported that N-acetyl muramyl-L-alanyl-D-isoglutamine (MDP) was the minimum adjuvant-active structure of bacterial cell wall in BCG (Azuma I, Biochemical and immunological properties of the fractions of tubercle bacilli, Kekkaku, 1998 Feb;73(2):65-70). They have synthesized several hundreds of MDP derivatives and selected, MDP-Lys(L18), romurtide, as the candidate of cytokine inducer for the clinical application. Romurtide is applied in cancer patients for the recovery of the number and functions of monocytes, neutrophils and platelets (Azuma I, Biochemical and immunological properties of the fractions of tubercle bacilli, Kekkaku, 1998 Feb;73(2):65-70). These results suggest that the tubercle bacilli, especially, CWS and related synthetic MDP derivatives, are effective for the potentiation of host resistance against infectious diseases and cancer. By the use of the combination of the present invention the size of primary tumor in FISS is reduced, also in cases in which is not possible to surgically remove the primary neoplasm. The disease was stabilized, the tumoral development blocked and, in cases in which the surgical ablation of the primary mass is possible, the post-surgical relapses definitively prevented in the 85% of the treated cases of FISS.

For this protocol of cancer treatment, thirty cats affected by FISS were selected, the tumour surgically removed and histologically graded according to the histological grading system proposed by Couto (Couto SS, Griffey, M, Duarte PC, Madewell BR, Feline vaccine-associated fibrosarcoma: morphological distinctions, Vet Pathol, 2002; 39: 33-41). Seven cases were classified as grade I, thirteen as grade II and ten as grade III. Serial sections of tumours were immuno-stained by avidin-biotin technique, as reported in previous works (Magi GE, Rossi G, Bertani C, Renzoni G, PTEN, PI3K and NFk-B expression in feline post-vaccinal fibrosarcoma, Proceedings of 26th Annual Meeting of ESVP, 17-21 Sept. Dubrownik Croatia, 2007; 142-143.) for COX-2 (Santa Cruz, M-19, goat polyclonal antibody) and NF-κB p65 (Abcam, rabbit polyclonal antibody). Staining was evaluated semi quantitatively for percentage and intensity. The score for each antibody represented the product of percentage of positive tumour cells and intensity. Scores from 1 to 3 were considered as low expression, from 4 to 6 as intermediate expression, and scores ≥ 7 as high expression. Specificity of COX-2 antibody was validated using an immunizing peptide blocking experiment.

In all cases an analysis of immune response of cats affected by FISS when immuno-stimulated, after the excision of the tumour, by the administration of BCG+anti-COX2 association was performed. Cats received the BCG/anti-COX2 protocol (as previously reported), to obtain a cell-mediated immune response stimulation. At time of excision (T0), and after one year from the tumour excision (T1), sera of these cats were tested. Western blot analysis, a semi-quantitative measurement, was used to evaluate expression of different protein pattern in tumour cells compared to fibroblasts belonging to the dermis of a healthy cats, and to monitor the development of antibodies to these tumour cell antigens in immuno-stimulated animals. In this assay, 5x106 total tumour cells or normal fibroblasts were lysed using 1 ml of a mammalian lysis buffer (Mammalian Cell-PE LB, Geno-Tech, St. Louis, MO). The cell lysates were then clarified by centrifugation and the supernatant harvested and stored. The lysates were subjected to 10% SDS-Polyacrylamide gel electrophoresis at 200 V for 45 minutes. Transfer of the separated proteins from the gel to PVDF membrane was carried out using a BioRad Semi-dry Transfer System at 100 mA for 60 minutes. Following transfer the blot/PVDF membrane was washed overnight using 100 ml of TTBS with 1% non-fat dried milk. The PVDF/blot was then transferred to a hybridization plate and 2 ml of authologus serum of each tested cat, used at a dilution of 1:5,000 was added. The PVDF/blot was then incubated and rotated in a hybridization oven at 37°C for 30 minutes. After this incubation step, the PVDF/blot was washed 3 times in 15 ml of TTBS buffer. Following the final wash, 20 ml of a 1:20,000 dilution of a biotinylated Goat Anti-Cat IgG antibody (Vector laboratories, Burlingame, UK) was added and the PVDF/blot incubated for a further 30 minutes at 37°C. The PVDF/blot was then washed 3 times in TTBS. Detection of the bound antibody was carried out observing labelled bands.

At the same time histological sections performed from cutaneous biopsies taken in the area of neoplasm excision, one year after the surgery (T0), were tested to immunophenotyping the lymphocytes infiltrate around peripheral areas of the old surgery. These lymphocytes subsets were characterized also in two cases of abortive local recurrence of the tumour. The panel of antibodies employed for lymphocytes characterization comprehend: anti CD3 (Sigma, monoclonal, diluted 1:10); anti CD21 (VMRD, monoclonal diluted 1:50), anti CD8 (VMRD, monoclonal diluted 1:10), anti CD36 (VMRD , specific for NK cells, monoclonal diluted 1:10), anti Lisozyme (Sigma, monoclonal, 1:100), anti CD4 (Sigma, monoclonal diluted 1:10), and CD25 (Sigma, monoclonal diluted 1:50). Serial sections were stained with these antibodies to evaluate the typology of cell mediated immunoresponse to the tumour.

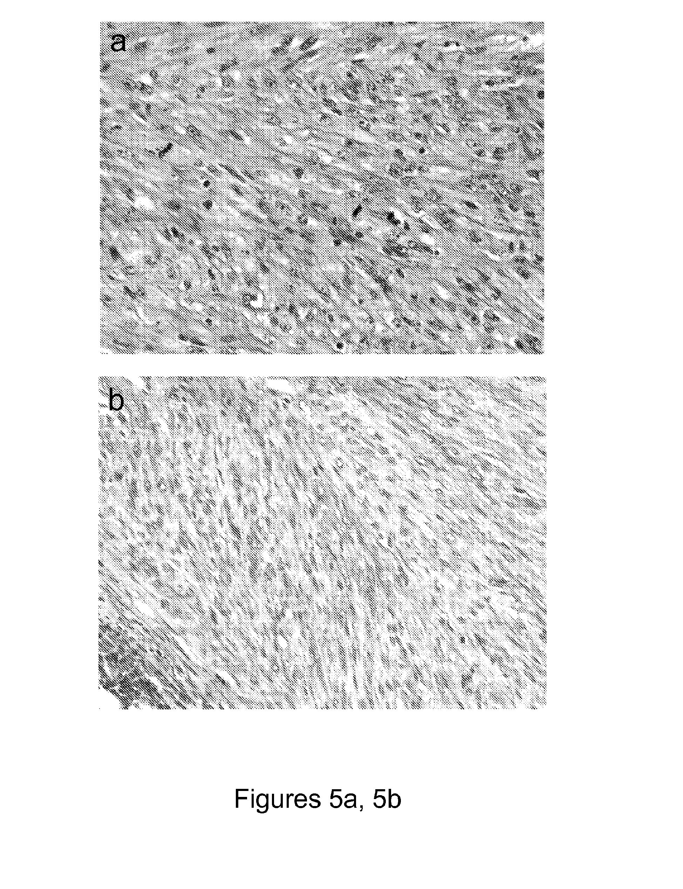

For COX-2 thirty FISS (97%) were immunopositive. The score level varied from low (12/30; 40%) to intermediate (9/30; 30%) and high (9/30; 30%) (figure 5A and 5B). No correlation was observed between COX-2 score and histological grade, in fact we did not found a higher COX-2 expression in the higher grade FISS (grade III). Within the FISS neoplastic multinucleated giant cells were immunoreactive for COX-2.

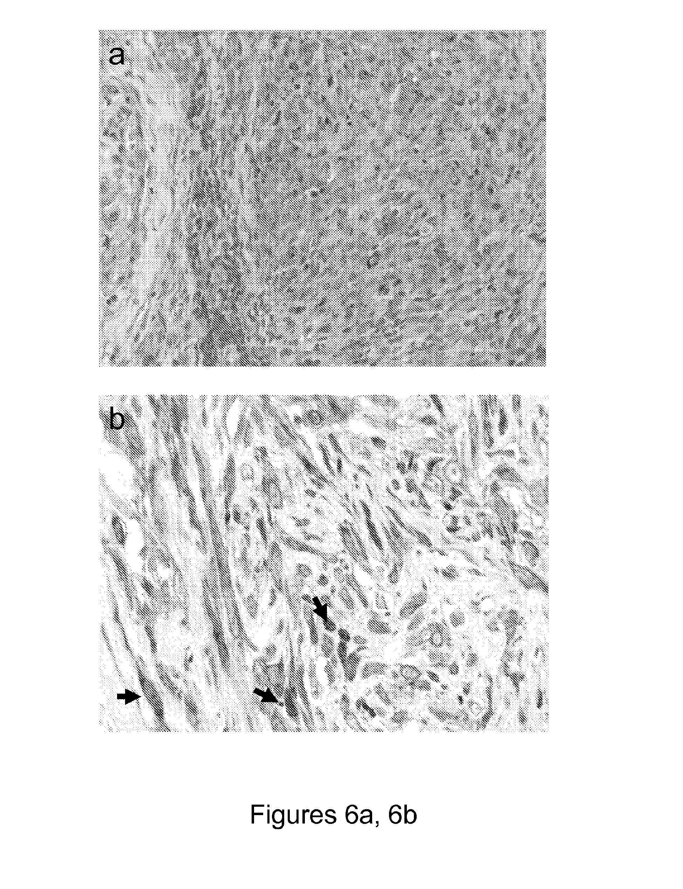

For NF-κB p65 we identified 31 of 31 (100%) FISS specimens immunoreactive. The score was low in 4 cases (13%), intermediate in 16 cases (52%) and high in 11 cases (35%) (figure 6A and 6B). In all cases cytoplasmic immuno-staining was observed but also in 9 cases nuclear positivity was present (figure 6B). Regarding the grade no correlation was observed between score level and histological grade.

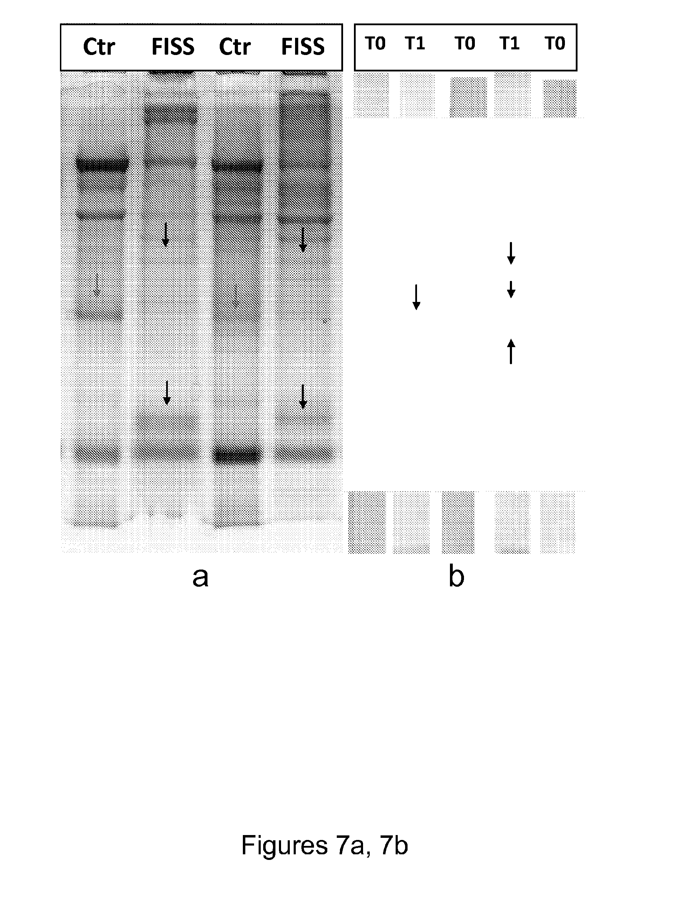

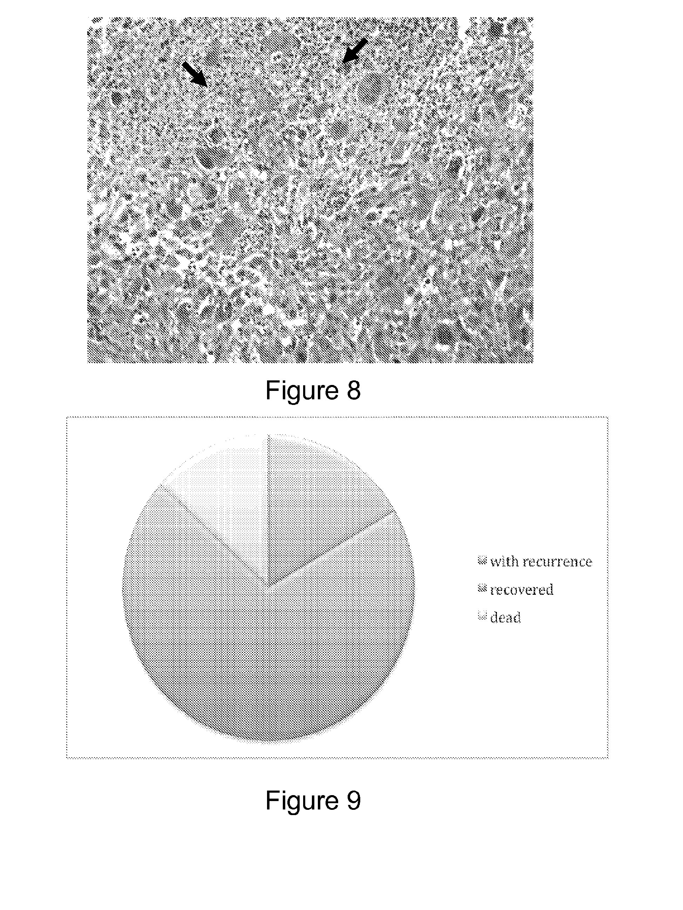

Pre-immune sera (T0) and immune sera (T1) one year after the immunomodulant protocol were compared by standard PAGE Western blot analysis. Figure 7B shows the result of a Western blot analysis of a cat. In all cats, pre-immune sera showed some reactivity to autologous tumour cell lysates but also to normal fibroblasts. Neoplastic cells differ from normal fibroblasts by the presence of a two proteins, 40.738, and 14.689 kDa of weight respectively, but they lack of 17.538 kDa protein (figure 7A). The reactivity of the preimmune sera was primarily with high molecular weight proteins. The sera from 1 year showed significant reactivity with multiple tumor cell antigens of varying molecular weights, and constant reaction with proteins observed only in neoplastic cells (see the previous reported molecular weights in kDa) was observed in all the cats of this study. Clinically, only in three immunomodulate cats a tumour recurrence was reported, 6, 7 and 9 months after the first excision but the relapses histologically demonstrated a very different aspect to respect the primary tumour, characterized by strong perineoplastic lymphoplasmacytic infiltration, strong tumour demarcation and diffuse aspect of granulomatous lesions with some areas of fibroblasts morphologic atypia and a diffuse areas of necrosis (figure 8). Immunophenotypical analysis of lymphocyte subsets that infiltrate the surrounding area of the tumor, demonstrate that a strong percentage of CD8+ and CD4+/Th1 lymphocytes and activated macrophages are present after our BCG+antiCOX2 treatment. This particular pattern of inflammatory cells is strongly indicative of a cell-mediated and cytotoxic response, and indicate also a strong macrophages activation to respect tumour antigens.

Table 2 evidenced the follow up of treated cats, in term of time of surviving, recurrence of the tumour or general spread of the neoplasia in treated cats. Is interesting to note that in untreated cats (in which the Immunomodulation protocol is not applied) the mean time for a recurrence of the tumour is approximately 3.5 months. In our cases we have that also the recurrences showed a mean time of 7.3 months for relapse.

The inflammatory origin of a particular group of fibrosarcomas or sarcoma-like lesions that occur in man is strongly suspected and in low number of studies was also proposed (Fletcher CD, Krishnan Unni K, Mertens F, Pathology and genetics of tumours of soft tissue and bone, In: Kleihues PM, Sobin LH, editors, World Health Organization Classification of Tumours, 4th edition. Lyon, France: IARC Press; 2002, 10-16, 102-103, 120-126). Nodular (pseudo-sarcomatous) fasciitis for example, is a reactive vascular and fibro-proliferative response to injury that occur in the head and neck region (DiNardo LJ, Wetmore RF, Potsic WP, Nodular fasciitis of the head and neck in children. A deceptive lesion, Arch Otolaryngol Head Neck Surg,1991; 117:1001-1002; Price EP Jr, Siliphant WM, Shuman R, Nodular fasciitis: a clinic-pathologic analysis of 65 cases, Am J Clin Pathol 1961; 35:122. 85; Soule EH, Proliferative (nodular) fasciitis, Arch Pathol, 1962; 73:437; Werning JT, Nodular fasciitis of the oro-facial region, Oral Surg Oral Med Oral Pathol. 1979; 48:441-446.). The lesion is benign but has a rapid rate of growth and a histopathologic appearance which can be quite alarming. Histologically this pseudosarcomatous lesion presents haphazardly arranged bundles of fibroblasts in a myxoid or mucoid background, with a fine capillary network arranged in a radial pattern around a larger central vessel or vessels (Montgomery EA, Meis JM, Nodular fasciitis. Its morphologic spectrum and immunohistochemical profile, Am J Surg Pathol, 1991; 15:942-945; Price S, Kahn LB, Saxe N, Dermal and intravascular fasciitis: unusual variants of nodular fasciitis, Am J Dermatopathol, 1993; 15:539-543; Werning JT, Nodular fasciitis of the orofacial region, Oral Surg Oral Med Oral Pathol. 1979; 48:441-446). The fibroblasts are typically large and plump, similar to those of granulation tissue. Pleomorphic fibroblasts may be present, and mitoses are common but not plentiful or abnormal. A variable amount of collagen and acid mucopolysaccharide are seen in the intercellular matrix, and scattered chronic inflammatory cells are typically present in small to moderate numbers of cases (Montgomery EA, Meis JM, Nodular fasciitis. Its morphologic spectrum and immunohistochemical profile, Am J Surg Pathol, 1991; 15:942-945). Interestingly, long-standing lesions may demonstrate foamy histiocytes and osteoclast-like multinucleated giant cells with 2-6 nuclei, very similar to pleomorphyc giant cells observed in FIIS (Lai FM-M, Lam WY, Nodular fasciitis of the dermis, J Cutan Pathol, 1993; 20:66-69). When striated muscle is involved (intramuscular fasciitis), it is completely replaced by the fibrovascular proliferation, unlike proliferative myositis, which infiltrates between muscle fibres (Montgomery EA, Meis JM, Nodular fasciitis. Its morphologic spectrum and immuno-histochemical profile, Am J Surg Pathol, 1991; 15:942-945). The spindle-shaped fibroblasts in this lesion tend to be arranged in long fascicles which are slightly curved, whorled or S-shaped, and they seem especially prone to extension along the fibrous septa of hypoderm. Similarly to the feline FISS, the lesion is not often encapsulated but is usually well demarcated from surrounding tissues (Montgomery EA, Meis JM, Nodular fasciitis. Its morphologic spectrum and immuno-histochemical profile, Am J Surg Pathol, 1991; 15:942-945). As in FISS, ultra-structural studies have confirmed the presence of myofibroblasts in nodular fasciitis, with a basic fibroblast appearance but with peripherally located bundles of myofilaments with dense patches similar to those of smooth muscle cells (Diaz-Flores L, Martin Herrera Al, Garcia Montelongo R, Gutierrez Garcia R, Proliferative fasciitis: ultrastructure and histogenesis, J Cutan Pathol, 1989; 16:85-92), immunoreactive to vimentin, smooth muscle actin, and muscle-specific actin, but not for desmin. The presence of inflammatory cells also help to differentiate the lesion from true fibrosarcoma, lipo- or rhabdomiosarcomas or sarcomas of hystiocitic nature. As in FISS, occasional lesions will demonstrate very small foci of metaplastic bone or cartilage (ossifying fasciitis, fasciitis ossificans, parosteal fasciitis), tempting the human pathologist to diagnose the case as osteosarcoma (Diaz-Flores L, Martin Herrera Al, Garcia Montelongo R, Gutierrez Garcia R, Proliferative fasciitis: ultrastructure and histogenesis, J Cutan Pathol.1989; 16:85-92). Despite its often aggressive microscopic appearance, nodular fasciitis is a self-limiting lesion which is readily treated by simple local excision, but recurrence rates vary from 1-6% with this treatment and some lesions have been reported to have multiple recurrences (Lai FM-M, Lam WY. Nodular fasciitis of the dermis, J Cutan Pathol, 1993; 20:66-69). Recently another mesenchymal neoplasia, classified as myxoinflammatory fibroblastic sarcoma, have been described in man. This tumour of the subcutaneous soft tissue, commonly diagnosed in adults, can arise on the trunk but most commonly occurs in the distant extremities (Meis-Kindblom JM, Kindblom LG, Acral myxoinflammatory fibroblastic sarcoma: a low-grade tumour of the hands and feet. Am J Surg Pathol, 1998; 22:911 -924; Montgomery EA, Devaney KO, Giordano TJ, Weiss SW, Inflammatory myxohyaline tumour of distal extremities with virocyte or Reed-Sternberg-like cells: a distinctive lesion with features simulating inflammatory conditions, Hodgkin's disease, and various sarcomas, Mod Pathol, 1998; 11:384 -391). As FISS, also myxoinflammatory fibroblastic sarcoma has a relatively good prognosis with a long life expectancy despite frequent local recurrence (Meis-Kindblom JM, Kindblom LG, Acral myxoinflammatory fibroblastic sarcoma: a low-grade tumour of the hands and feet, Am J Surg Pathol, 1998; 22:911 -924; Lambert I, Debiec-Rychter M, Guelinckx P, Hagemeijer A, Sciot R, Acral myxoinflammatory fibroblastic sarcoma with unique clonal chromosomal changes, Virchows Arch, 2001; 438:509 -512). According to the literature, the local recurrence rate in patients with myxoinflammatory fibroblastic sarcoma ranges from 22% to 67% (Meis-Kindblom JM, Kindblom LG, Acral myxoinflammatory fibroblastic sarcoma: a low-grade tumour of the hands and feet, Am J Surg Pathol, 1998; 22:911 -92; Montgomery EA, Devaney KO, Giordano TJ, Weiss SW, Inflammatory myxohyaline tumour of distal extremities with virocyte or Reed-Sternberg-like cells: a distinctive lesion with features simulating inflammatory conditions, Hodgkin's disease, and various sarcomas, Mod Pathol, 1998; 11:384 -391), with an uncertain metastasis rate; metastases have been reported to develop in only a few cases (Meis-Kindblom JM, Kindblom LG, Acral myxoinflammatory fibroblastic sarcoma: a low-grade tumour of the hands and feet, Am J Surg Pathol, 1998; 22:911 -924). In all of our patients, excisional biopsy for definitive diagnosis and primary therapy was performed. As feline injection induced sarcoma, grossly, myxoinflammatory fibroblastic sarcoma forms a poorly circumscribed mass that extend into the dermis and skeletal muscle. Microscopically, the tumour is characterized by solid nests of atypical spindle and epithelioid cells in a myxoid stroma and dense inflammatory infiltrates. The immunophenotype is positive for vimentin, with variable immunoreactivity for CD34, CD68, cytokeratin, and smooth-muscle actin (Meis-Kindblom JM, Kindblom LG, Acral myxoinflammatory fibroblastic sarcoma: a low-grade tumour of the hands and feet, Am J Surg Pathol, 1998; 22:911 - 924; Pohar-Marinsek Z, Flezar M, Lamovec J, Acral myxoinflammatory fibroblastic sarcoma in FNAB samples: can we distinguish it from other myxoid lesions?, Cytopathology, 2003; 14:73 -78). Microscopic findings of myxoinflammatory fibroblastic sarcoma also closely resemble those of giant cell tumours of the tendon sheath, proliferative fasciitis, acral fibromyxoma, myxoid liposarcoma, and myxofibrosarcoma (Llauger J, Palmer J, Monill JM, Franquet T, Bague S, Roson N, MR imaging of benign soft-tissue masses of the foot and ankle, RadioGraphics,1998; 18:1481 -1498; Munk PL, Sallomi DF, Janzen DL, et al, Malignant fibrous histiocytoma of soft tissue imaging with emphasis on MRI, J Comput Assist Tomogr.1998; 22:819 -826). The morphological and clinical similarities between inflammatory-induced soft tissues proliferations in man and feline ISS are also accompanied by similar pathogenic mechanisms. In fact many tumours in humans have been associated with autocrine stimulation (i.e., tumours have receptors for growth factors, which they themselves produce in an abnormal or autonomous fashion). Many oncogenes cause cancer by coding for and causing overexpression of growth factors or their receptors (v-sis codes for platelet-derived growth factor [PDGF]; verb codes for epidermal growth factor [EGF] receptor).Several studies are focusing on immunohistochemical identification and localization of growth factors and their receptors in injection-associated lesions of cat. Injection-associated sarcomas have been found to be immunoreactive for PDGF and its receptor, EGF and its receptor, and transforming growth factor, whereas non-injection -associated fibrosarcomas are negative or faintly positive.(Hendrick MJ, Feline vaccine-associated sarcomas: Current studies on pathogenesis, J Am Vet Med Assoc, 1998; 213:1425-1426; Hendrick MJ, Feline vaccine-associated sarcomas, Cancer Invest, 1999; 17:273-277). Additionally, lymphocytes in injection-associated sarcomas are positive for PDGF, but lymphocytes in non-vaccine-associated sarcomas and in normal lymph nodes or aggregated lymphoid follicles (Peyer's patches) are negative. Macrophages in the area also stain positively for PDGF receptor. The neoplastic cells in vaccine-associated sarcomas that are closest to the lymphocytes have the strongest staining for PDGF receptor. These findings led to a hypothesis that lymphocytes in vaccine-associated lesions may secrete PDGF to recruit macrophages and cause fibroblast proliferation (Hendrick MJ, Feline vaccine-associated sarcomas, Cancer Invest, 1999; 17:273-277). Additional work is being done to characterize the lymphocyte populations within these nodules.(Hendrick MJ, Feline vaccine-associated sarcomas: Current studies on pathogenesis, J Am Vet Med Assoc, 1998; 213:1425-1426; Hendrick MJ, Feline vaccine-associated sarcomas. Cancer Invest. 1999; 17:273-277). Injection-associated sarcomas express c-jun, a proto-oncogene coding for the transcriptional protein AP-1 that is associated with cellular proliferation and oncogenesis in vitro. Non-vaccine-associated sarcomas do not express c-jun. (Hendrick MJ, Feline vaccine-associated sarcomas, Cancer Invest, 1999; 17:273-277). Increased growth factor stimulation of fibroblasts by lymphocytes and ultimately via autocrine stimulation has been hypothesized to lead to induction and overexpression of c-jun. As in man, feline post-injection sarcomas are associated with the presence of p53 gene mutations.(Mayr B, Reifinger M, Alton K, Schaffner G, Novel p53 tumour suppressor mutations in cases of spindle cell sarcoma, pleomorphic sarcoma and fibrosarcoma in cats, Vet Res Commun, 1998; 22:249-255; Mayr B, Blauensteiner J, Edlinger A, et al., Presence of p53 mutations in feline neoplasms, Res Vet Sci, 2000; 68:63-69) that induce an alteration or absence of the gene-relative product; the cell cycle regulating protein p53. Cells in which p53 is absent or mutated are allowed to proceed unregulated through the cell cycle, giving rise to aberrant clones, which may go on to result in malignancy. Our study primarily show that NF-κB and COX-2 are highly expressed in FIIS. NF-κB transcription factor pathway as well as COX-2 seem to be important in cancer related inflammatory genesis and represent a novel molecular targets for treatment of FISS. Inappropriate NF-κB can stimulate cell transformation and growth, in addition NF-κB seems to have a role in tumours with high immunohistochemical component.

On the basis of the immunohistochemical results of this study, it was found that NF-κB p65 sub-unit was expressed in all the cases studied and that COX-2 was expressed in 97% of d cases, with a score level varied from low (12/30; 40%) to intermediate (9/30; 30%) and high (9/30; 30%). Interestingly, the absence of correlation observed between COX-2 score and histological grade of FIIS analysed may be indicate that COX2 expression is highly maintained also in undifferentiated form of FISS; these forms could be sensible as well differentiated forms when chemotherapic protocols employing anti-COX2 drugs are used. Our results are in striking contrast to those obtained in a previous study (Beam SL, Rassnick KM, Moore AS, McDonough SP, An immunohistochemical study of cyclooxygenase-2 expression in various feline neoplasms, Vet Pathol, 2003 Sep;40(5):496-500) in which FISS showed a constant negativity for COX2 expression. Lack of detectable COX-2 expression in the FISS of previous study, may have been caused by sample size, failure of the antigen retrieval method used, or other confounding variables such as the type of fixative or prolonged fixation. The polyclonal antibody used in this study has detected COX-2 in feline and canine tissues in previous studies (Khan KNM, Stanfield KM, Trajkovic D, Knapp DW. Khan KNM, Stanfield KM, Trajkovic D, Knapp DW, Expression of cyclooxygenase-2 in canine renal cell carcinoma, Vet Pathol, 2001; 38:116-119; Mohammed SI, Bennett PF, Craig BA, Effects of the cyclooxygenase inhibitor, piroxicam, on tumour response, apoptosis, and angiogenesis in a canine model of human invasive urinary bladder cancer, Cancer Res, 2002; 62:356-358.) as well as in control canine tissues used in this study. Determination of prostaglandin E2 (PGE2) concentration is commonly used as a marker for COX-2 activity. (Mohammed SI, Coffman K, Glickman NW, Prostaglandin E2 concentrations in naturally occurring canine cancer, Prostaglandins Leukot Essent Fatty Acid, 2001; 64:1-4.; Brideau C, Van Staden C, Chan CC, In vitro effects of cyclooxygenase inhibitors in whole blood of horses, dogs, and cats, Am J Vet Res, 2001; 62:1755-1760) Prostaglandins, produced by COX-2 activity, increase cell proliferation, inhibit apoptosis, promote angiogenesis, alter cellular adhesion that allows for metastasis, inhibit immune surveillance, and may activate xenobiotics to reactive substances that are carcinogenic (Mohammed SI, Coffman K, Glickman NW, Prostaglandin E2 concentrations in naturally occurring canine cancer, Prostaglandins Leukot Essent Fatty Acid, 2001; 64:1-4; Mohammed SI, Bennett PF, Craig BA, Effects of the cyclooxygenase inhibitor, piroxicam, on tumour response, apoptosis, and angiogenesis in a canine model of human invasive urinary bladder cancer, Cancer Res. 2002; 62:356-358; Dempke W, Rie C, Grothey A, Cyclooxygenase-2: a novel target for cancer chemotherapy?, J Cancer Res Clin Oncol, 2001; 127:411-417; Shaheen NJ, Straus WL, Sandler RS, Chemoprevention of gastrointestinal malignancies with nonsteroidal anti-inflammatory drugs, Cancer, 2002; 94:950-963). In numerous studies on humans, the chronic use of NSAIDs is linked to a lower incidence of several types of neoplasms and COX-2 inhibitors cause reductions in neoplastic cell numbers in vitro and tumour regression in mouse tumour models (Dempke W, Rie C, Grothey A, Cyclooxygenase-2: a novel target for cancer chemotherapy?, J Cancer Res Clin Oncol, 2001; 127:411-417; Shaheen NJ, Straus WL, Sandler RS, Chemoprevention of gastrointestinal malignancies with nonsteroidal anti-inflammatory drugs, Cancer, 2002; 94:950-963). The results demonstrated also that neoplastic tissue belonging to cats affected showed some protein bands, with the 40.738; 17.538, and 14.689 kDa of weight respectively. These bands are present in the neoplastic cell membrane lysates but no in cytoplasm-derived proteins. These proteins are strongly reactive with cats sera after one year of induced immunomodulation, demonstrating a strong immunogenicity for the host. Although limited information is available on the use of acemannan or other immunostimulants products used in combination with surgery and radiation therapy in the treatment of feline IIS (King GK, Yates KM, Greenlee PG, The effect of acemannan immunostimulant in combination with surgery and radiation therapy on spontaneous canine and feline fibrosarcomas, J Am Anim Hosp Assoc, 1995;31:439-447), the results demonstrated that an enhancement of cell-mediated immune response, obtained by BCG+ anti- COX2 protocol, increases the cat reactivity to the plasmalemmal-associated neoplastic cells antigens and to induce a control of local recurrences of the tumour. These results are very important also for treatment of human cases of inflammatory-related sarcomas. The suggestion has been made that because of the apparent role of inflammatory mediators in the pathogenesis of FISS, as in human MIS and NF, manipulation of the inflammatory and immunologic mechanisms as COX2 silentiation or Th-1 and NKs response enhancement, should be explored.(Hendrick MJ, Feline vaccine-associated sarcomas: Current studies on pathogenesis, J Am Vet Med Assoc, 1998; 213:1425-1426).

In the following table 2 are reported the results of the follow-up of treated cats, after surgical ablation of the tumour. In 9/30 cats we observe no recurrence after 1125d (3years) from T0; in 5/30 cats no recurrence after 570/630d from T0 (2 years), 3/30 no recurrence after 390/450d from T0 (1 years), In 5/30 cats we have recurrence after 280/300 days, while actually, in 4/30 any recurrence is observed after a period of 270/330d from T0. Finally, 4/30 cats died during the treatment for different causes (i.e. trauma or other diseases) independently to the tumour or to the protocol.

**Table 2**

| **Cat n°** | **Histological grade** | **Follow up** | **Notes** |
|---|---|---|---|
| 1 | I | no recurrence after 1125d (3ys) from T0 | 9/30 no recurrence after 1125d (3ys) from T0 |
| 2 | I | no recurrence after 1125d (3ys) from T0 | 5/30: no recurrence after 570/630d from T0. |
| 3 | I | no recurrence after 570/630d from T0. | 3/30: no recurrence after 390/450d from T0 |
| 4 | I | no recurrence after 570/630d from T0. | 5/30: recurrence after 280/300d |
| 5 | I | no recurrence after 390/450d from T0 | 4/30: death during the treatment (different causes). |
| 6 | I | no recurrence after 1125d (3ys) from T0 | 4/30: no recurrence after 270/330d from T0. |
| 7 | I | death during the treatment (different causes). | |
| 8 | II | no recurrence after 570/630d from T0. | |
| 9 | II | no recurrence after 1125d (3ys) from T0 | |
| 10 | II | no recurrence after 390/450d from T0 | |
| 11 | II | no recurrence after 570/630d from T0. | |
| 12 | II | no recurrence after 1125d (3ys) from T0 | |
| 13 | II | no recurrence after 270/330d from T0. | |
| 13 | II | death during the treatment (different causes). | |
| 15 | II | no recurrence after 1125d (3ys) from T0 | |
| 16 | II | no recurrence after 270/330d from T0. | |
| 17 | II | recurrence after 280/300d | |
| 18 | II | no recurrence after 1125d (3ys) from T0 | |
| 19 | II | death during the treatment (different causes). | |
| 20 | II | recurrence after 280/300d | |
| 21 | III | no recurrence after 390/450d from T0 | |
| 22 | III | no recurrence after 270/330d from T0. | |
| 23 | III | recurrence after 280/300d | |
| 24 | III | death during the treatment (different causes). | |
| 25 | III | recurrence after 280/300d | |
| 26 | III | no recurrence after 270/330d from T0. | |
| 27 | III | no recurrence after 570/630d from T0. | |
| 28 | III | no recurrence after 1125d (3ys) from T0 | |
| 29 | III | no recurrence after 1125d (3ys) from T0 | |
| 30 | III | recurrence after 280/300d | |

### Example 3 - Feline Infectious Peritonitis (FIP)

Feline infectious peritonitis (FIP) is an immunologically mediated lethal disease that occurs in cats infected with virulent feline coronavirus (Fc) strains. Differing to the virulent strains, the avirulent coronaviruses infect fewer macrophages, produce lower virus titers, are less able to sustain viral replication, and spread less efficiently to other susceptible macrophages. Additionally data indicate a decrease in peripheral blood lymphocyte counts in infected cats, and a particularly marked decrease in T cells including CD4+ and CD8+cells. In particular, in vitro studies suggest that TNF-alpha, produced by virus-infected macrophages, is responsible for induction of apoptosis in uninfected T cells, primarily CD8+ T lymphocytes. Some in vitro and in vivo evidences that the protocol enhance the macrophages activity in terms of antigen binding and killing, by the way of NO production, enhancing also some interleukins production, typical of the Th1 and Th2 response, as high levels of IFN-γ and IL-4. This interleukins combination is very effective in CD8+ recruitment and activation. In the absence of therapies or immunizing protocols, has been demonstrated that the immunomodulating protocol is very effective to treat all the clinical forms of FIP, particularly the dry uneffusive, to enhance the quality of life, prolong the life span and possibly resolve the infection in affected cats.

In a preliminary study, and involving six cats with dry form and two cats with wet form of FIP, the animals have been treated with the immunomodulatory protocol (IP) as disclosed in examples 1 and 2 . Actually 3 cats with dry form (50%) and one cat with wet form (50%) treated are alive and well, three years after diagnosis. Tests performed monthly on macrophages isolated, cultured and in vitro stimulated, belonging to these animals show an increase metabolic activity and high concentration of NO production, compared to values obtained from cells of same cats at the beginning of therapy. Additionally data are represented by an increase CD4+/CD8+ T lymphocytes ratio, with high levels of CD8+ lymphocytes producing IFN-γ. This increase CD8+ T cells activity in peripheral blood of the four treated cats is probably due to an increased level of IL-4, typically induced by BCG stimulation of macrophages, as reported by Ravindran R, Bhowmick S, Das A, Ali N, Comparison of BCG, MPL and cationic liposome adjuvant systems in leishmanial antigen vaccine formulations against murine visceral leishmaniasis, BMC Microbiology, 2010, 10:181.

Thus, in treated cats, the IP enhances the production of two interleukins typical of Th1 and Th2 response: IFN-γ and IL-4 respectively. This interleukins combination is very effective in CD8+ recruitment and activation. Further, once it is diagnosed, cat life expectancy drops to two years. About this, there are only a few reports on the survival time of cats with FIP, most of them are anecdotal reports, which do not define clearly the survival time after diagnosis of FIP (Robison RL, Holzworth J, Gilmore CE, Naturally occurring feline infectious peritonitis: Signs and clinical diagnosis, J Am Vet Med Assoc, 1971, 158:981-986; McReynolds C, Macy D, Feline infectious peritonitis. Part I. Etiology and diagnosis, Comp Cont Educ Pract Vet, 1997; 19:1007-1016). Only two studies about FelFN-ω administration after the diagnosis of FIP reported the survival time of cats. In a first study, 4 of 12 cats treated with FelFN-ω survived longer than 2 years (Ishida T, Shibanai A, Tanaka S, Use of recombinant feline interferon and glucocorticoid in the treatment of feline infectious peritonitis, J Feline Med Surg 2004; 6:107-109.).On the contrary, in a second study the longest survival time of treated cats was 200 days, and FelFN-ω treatment didn't produce any significant improvement in treated cats in compared to cats treated with placebo; the only difference between the groups consisted in a significantly lower lymphocyte count in treated cats (Ritz S, Egberink H, Hartmann K, Effect of Feline Interferon-Omega on the Survival Time and Quality of Life of Cats with Feline Infectious Peritonitis, J Vet Intern Med, 2007; 21:1193-1197). From these premises, the need to approach the disease differently has arisen. As FIP is an immune-mediated disease, treatment is primarily aimed at controlling the immune response triggered by infection with Feline Coronavirus (FCoV). Some veterinarians prescribe immune modulators to treat cats with FIP with no documented controlled evidence of efficacy. It has been suggested that these agents may benefit infected animals by restoring compromised immune function, thereby allowing the patient to control viral burden and recover from clinical signs. However, a non-specific stimulation of the immune system may be contraindicated as clinical signs develop and progress as a result of an immune-mediated response to the mutated FCoV (Hartmann K, Ritz S, Treatment of cats with feline infectious peritonitis, Vet Immunol Immunopathol, 2008, May 15,123(1-2): 172-5). In this panorama the pecific IP may be an important therapeutic method to treat cats with diagnosis of FIP. Compared to steroid therapy, that usually leads to a survival time ranging from a few days to some weeks, by the use of our proposed protocol, an elongation of the survival time (months) has been demonstrated. Based on our actual knowledge on the pathogenesis of FIP and on the mechanisms of actions of drugs included in the experimental protocol, we have observed a remission of symptoms, as expected. Independently on the survival time or on the remission of symptoms, an improvement of the immune responsiveness in treated cats has been documented. Even in the absence of a complete clinical remission, our results are particularly important in terms of basic knowledge on FCoV-host interaction and of the establishment of preventive plans for eradication of FCoV infection from clinically healthy animals living in FCoV-endemic environments. The study was designed as a randomized double-blind one and two groups of cats, 8 cats included into group A (immunomodulated cats) and 5 cats assigned to the group B (corticosteroids treated cats), for a total of 13 cats. Inclusion criterion to enter the study was the definitive diagnosis of FIP (see table 3). These cats were consecutive cases with confirmed FIP that had owners willing to participate in the study. In cats with effusive forms (2 cats), the diagnosis was confirmed by detection of FCoV antigen in macrophages in the effusion.

**Table 3**

| | |
|---|---|
| Test | Findings consistent with FIP |
| Complete physical examination | effusions consistent with FIP (see below) |
| | neurological signs |
| | uveitis or ocular precipitates |
| | signs of renal insufficiency in young cats not exposed to potentially neurotoxic agents |
| | presence of abdominal masses with cytological features consistent with pyogranulomatous inflammation |
| Analysis of effusion | macroscopically yellowish cloudy, sticky and containing fibrin clots |
| | cytology: mixed inflammation (atoxic neutrophils/lymphocytes/macrophages/mesothelial cells), with proteinaceous granular background |
| | high protein and/or globulin content eventually positive immunocytochemistry |
| Serum protein electrophoresis | increased a2- and g-globulin (polyclonal) |
| Biochemistry | increased α1-acid-glycoprotein (AGP) |
| Hematology | lymphopenia |

Exclusion criteria was: presence of concurrent conditions potentially able to interfere with cell-mediate immunity (e.g. infection with FIV or FeLV, hpyeradrenocorticism, hyperthyroidism); administration of steroid treatments in the months before the first visit. Cats were excluded if they survived fewer than 72 hours after treatment initiation. If the protocol won't have any effect after the first week of treatment, the cats were submitted to a classical therapy based on palliative corticosteroids administration. Cats were randomly subdivided into two sub-groups A and B, as in table 4.

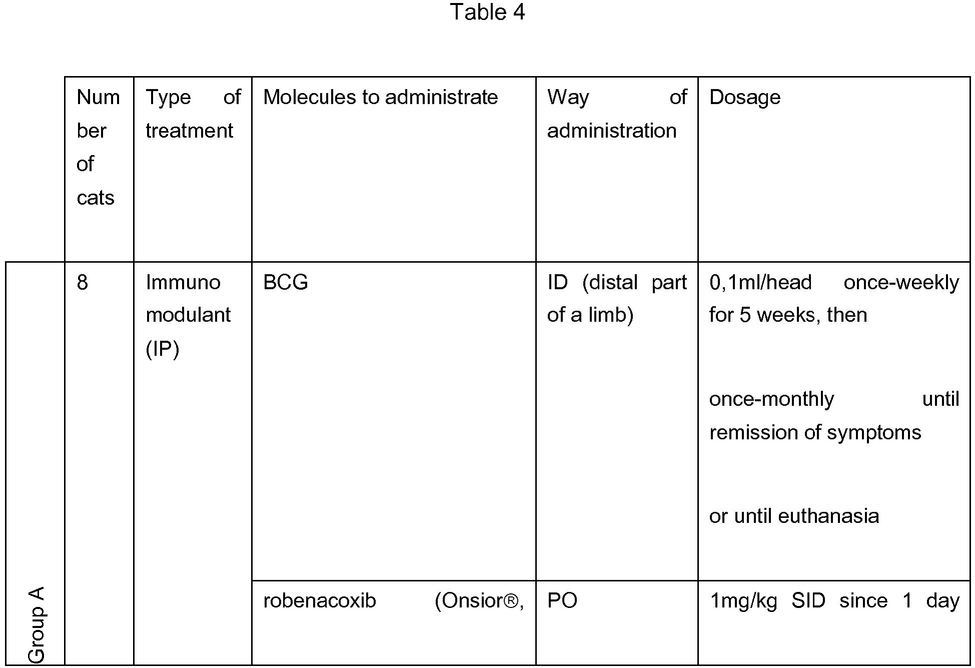

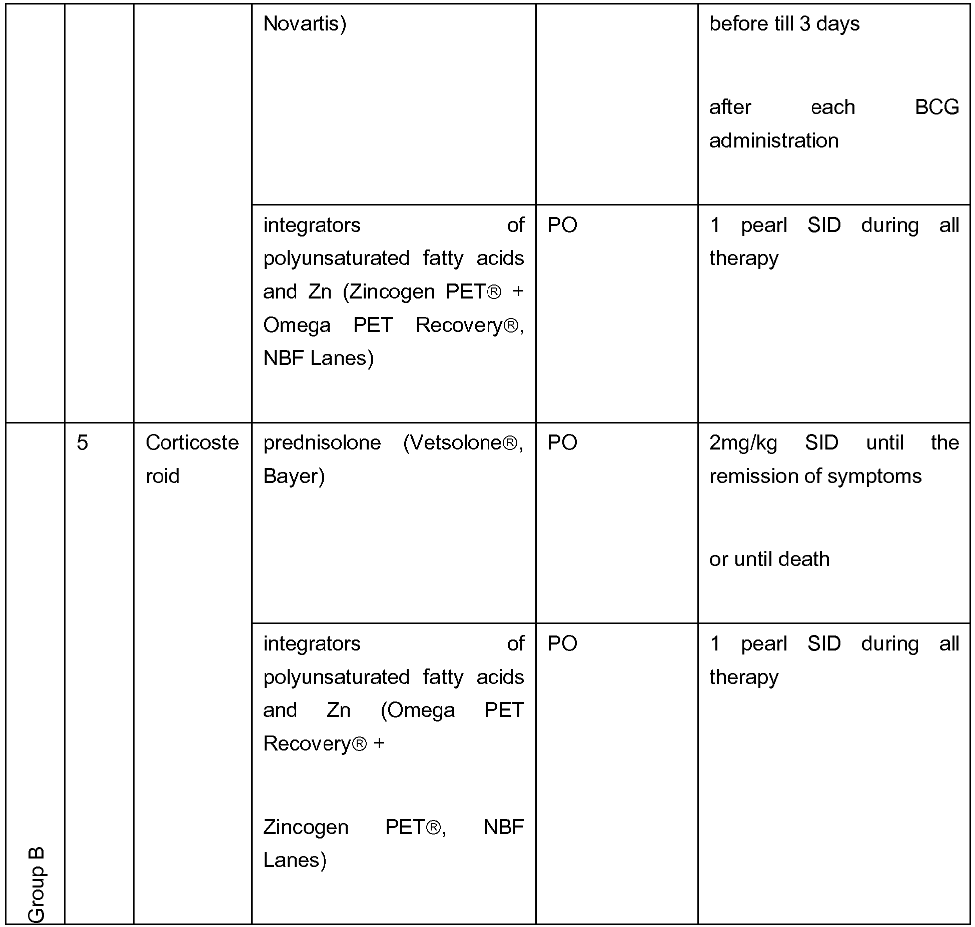

When requested by the clinical condition any additional treatment useful to maintain an adequate quality of life and to avoid unnecessary stress or pain was administered, following the protocols recommended in literature (Pedersen NC, Lin H, Dodd KA, Pesavento PA, Significance of coronavirus mutants in feces and diseased tissues of cats suffering from feline infectious peritonitis, Viruses 2009; 1:166-184; Addie DD, McLachlan SA, Golder M, Ramsay I, Jarrett O, Evaluation of an in-practice test for feline coronavirus antibodies, Journal of Feline Medicine and Surgery 2004; 2:63-68). These additional treatments included (depending on the clinical presentation): drainage of effusions, fluid therapy, administration of hepatoprotective or analectic drugs, specific therapies in the case of neurological signs, other drugs requested by the clinical presentation in the case of peculiar or atypical forms of FIP

Follow up and samplings - Results interpretation - Statistic : To assess the evolution and the intensity of inflammation and of humoral and cell mediated immunity, cats were subjected to daily clinical visits for the first week, then once a week for the next month and then once a month until the end of therapy. In occasion of each visit the following clinical parameters being recorded: body temperature, body weight, diameter of the abdomen. Blood samples were collected which also displays tests will be done at each sampling. In case of severe worsening of clinical condition, additional visits at times different from those scheduled above will be performed. In case of subjects with worsening of the clinical conditions threatening to patient life, cats were humanely euthanized under permission of the owners. In this case, just before the death, an additional sampling will be performed for the entire panel of tests described above. Afterwards, a complete necropsy will be performed, followed by histology and immunohistochemistry with antibodies against the FCoV and against the main feline lymphocyte subsets. Results were analyzed to assess the following clinical and immunological outcomes:
- Survival time: the survival time recorded in both groups was compared using Kaplan Meyer curves
- Time of remission of symptoms: cats were considered free of symptoms when the symptoms traditionally associated with effusive FIP have become no more evident, (especially effusion or fever). Possible presence of additional symptoms potentially associated with FCoV infection (e.g. ocular damages, mild neurological signs, moderate renal failure) were not considered as a direct consequence of "active" infection, because these signs could also depend on sequelae of lesions developed during the acute phase of the disease and they could thus persist also after the remission of the disease. The time of remission of symptoms recorded in the two groups were compared using Kaplan-Meyer curves.
- Time of relapse (time free of disease): relapses (reappearance of effusion or of other signs consistent with an "active" infection) were recorded and the time length between the first time of remission and the relapse recorded in the two groups was compared using Kaplan-Meyer curves.

Statistical analysis were performed with SPSS, Version 13.0.0. Variables compared between both groups included Body Condition score, erythrocytes, hematocrit, platelets, hemoglobin, leukocytes, monocytes, lymphocytes, banded neutrophils, mature neutrophils, eosinophils, basophils, alanine aminotransferase, alkaline phosphatase, bilirubin, protein, albumin, urea, creatinine, glucose, chloride, and phosphorus.

A difference in the survival time between both groups was evaluated with a log-rank test. Differences between the 2 groups at day 0 (to rule out a bias between groups), day 7, and day 14 were investigated with a Student's t-test or a Mann-Whitney U-test. The t-test was used for normally distributed data; the Mann-Whitney U-test will be used for all other parameters. P values of # .05 was considered significant. A Bonferroni's correction was performed to rule out multiple test interference. A 5% significance level was assumed for all variables, and thus, .05 was divided through the number of tests performed. Therefore, a final P # .002 for each variable was considered significant.

Results: There was no statistically significant difference in any variable on day 0 between cats later treated with protocol (IP) and those that later received corticosteroids. There was a significant difference (P # .001) between the lymphocyte counts (reference interval 1-4 3 109/L) of IP treated versus cats receiving corticosteroids on day 7, 14, and two months later the start of the protocol. In the IP group, the mean lymphocyte count was 0.5 6 0.7 3 109/L; in the placebo group, the mean lymphocyte count was 1.7 6 2.1 3 109/L. There was statistically significant difference in macrophages activity scores of IP treated group and the corticosteroid group at every day evaluated (day 7, 14 and two months). Cats survived between 27 and 1095 days (median, 561 days). The median survival time of cats belonging to the IP group was 561 days. The median survival time of the corticosteroid group was 37.5 days. The difference in survival time between both groups was significant. Four of eight (50%) IP treated cats survived more than one year. Cats were classified in 4 groups according to their survival time. Four cats (three showed a dry form, and one with effusive form) of the 8 IP treated cats improved in their general condition during treatment (these animals are alive actually). Two cats (of six cats with dry form) and one cat with effusive form were classified as "long-term survivors" (surviving, respectively, 198, 157, and 136 days), and one cat with dry form was classified as a "medium-term survivor" developed a secondary infection (upper respiratory tract disease, panleukopenia, or bacterial cystitis) during treatment (after 58 days), after which they deteriorated rapidly and died or had to be euthanized after a mean period of 70 days. All the five cats belonging to corticosteroid treatment were classified as very short term survivor because their mean period of life, from the moment of the diagnosis, was 39.2 days (from 27 to 52 days).

### Example 4 - Feline Chronic lymphocytic plasmacytic gingivostomatitis

The Feline Chronic Gingivostomatitis (FCGS) is a complex disease, frustrating for the practitioner and despairing for the animal owner, due to its frequency, severity and chronicity of lesions, with frequent relapses (Wiggs, RB, Lobprise HB, 1997, Domestic Feline Oral and Dental Disease. In: Veterinary Dentistry: principles and practice, Editores: RB Wiggs and HB Lobprise. Lippincott-Raven, Filadelfia, Nova York, 482-517; Johnston N, 1998, Acquired feline oral cavity disease. In Practice, 20 (4), 171-179). It is difficult to control with the available therapeutic regimes. Different approaches, medical, surgical or a combination of both, are described in the literature (Chaudieu G, Blaizot A, 1999, Gingivites et stomatites felines. Pratique Médicale et Chirurgicale de I'Animal de Compagnie, 34, 135-144; Camy G, 2003b, Management of a cat with chronic gingivitis stomatitis, Le Point Veterinaire, Junho, 236 (Número especial; Clarke DE, 2001, Clinical and microbiological effects of oral zinc ascorbate gel in cats, Journal of Veterinary Dentistry, 18(4), 177-83).

Responses to treatment are variable and therapeutic success is often limited and of short-term value, being essential to establish the adequate approach strategy to each patient. The protocol as disclosed in the previous examples (IP) was applied to this pathology, in attempt to enhance firstly a general, and after a local cell-mediated immunoresponse that induced a mucosal restoring. The different treatment regimes were compared. A randomized, double-blind clinical trial in cats with FCGS treated with Immunomodulating Protocol or placebo has been performed. In this trial, cats didn't receive any drugs in addition to the IP or Conventional therapy. For this study, 25 cats with diagnosis of severe FCGS, in high percentage (75%) with experience of two or three relapses of the diseases in a period of two years, were enrolled for the study. Cats were randomized to receive IP therapy (11 cats) or conventional treatment (Corticosteroid + antibiotic, as reported by Harvey, CE, Thornsberry C, Miller BR, Shofer FS, 1995b, Antimicrobial susceptibility of subgingival bacterial flora in cats with gingivitis, Journal of Veterinary Dentistry, 12 (4), 157-160). (14 cats).

In Cats in which IP protocol was applied, the protocol was used according to the same schedule used also for treatment of Injection Fibrosarcoma and FIP (see Example 2 and 3). Cats randomly assigned to "conventional protocol" (CP), received an association of Prednisone (used at the dosage of 2-4 mg/kg B.I.D., and after two weeks, daily for two other weeks), and Clavulanic Acid associated with Doxiciclin (at the dosage recommended by the producer) daily for a minimum of three weeks. Of 14 cats in the CP group, 13 (93%) showed a severe recrudescence of the disease and, in 4 cases, we humanely perform euthanasia of very highly affected and/or unresponsive animals. The median time of relapse of FCGS was 9.8 months in this latter group of cats. Cats assigned to the IP group experienced a relapse of the pathology only in 8 (73%) cases; the median of the relapse time was 14.4 months, which was significantly longer than that of the CP group (P < 0.01).

Material and Methods and Results: 25 cats were randomized after a correct diagnostic algoritm of FCGS. Cats eligible for randomization ranged in age from 1 to 12 years, with a mean age of 7 years. There were 12 males and 13 females. Eleven cats (11) were randomized into the IP group and 14 were randomized into the CP group. Of the 14 cats in the CP group, 13 (93%) had early recurrence of FCGS and 1 died of an unrelated cause (neurological disease). The median pathology free interval for the cats in the CP group was 7.6 months. Four (29%) cats remained free from the pathology over 1 year. In the IP group, 8 (73%) of 11 cats showed a recurrence of the pathology. Two cats died of unrelated causes (both euthanized for chronic renal failure - CKD), and in three cases, any disease relapse was observed until now. In this IP treated group, the median FCGS-free interval was 14.4 months. Seven (64%) of the 8 relapsing cats, were healthy over 1 year. Those cats receiving IP had a significantly longer FCGS-free interval (P < 0.01) compared to cats given CP therapy.

### Example 5 - Psittacine PDD.

Proventricular Dilatation Disease (PDD) is a devastating disease for affected birds. It is also psychologically and financially devastating for their owner/caretaker. The diagnosis of PDD in an avicultural collection can have devastating financial effects on aviculturists. Counseling as well as establishing a long-term management plan are important aspects in veterinary care. As diagnostic screening becomes available and positive birds can be identified, management will be much easier. The social implications of a PDD diagnosis can also be devastating. Owners may be shunned from bird club functions or social interaction with other bird owners, or find it impossible to find a pet sitter. Counseling for owners needs to include the fact that PDD can be hard to diagnose, can take many forms and can have a very long incubation period. Visually healthy birds but with crop biopsy positive for PDD have been found and also cases with very subtle signs in which Avian Bornavirus was detected by PCR of blood and cloacal swabs. Waiting for the classic clinical signs such as wasting, vomiting or passing whole seeds reveal only the tip of the iceberg. Early diagnosis can enhance therapeutic outcome. Until causation is confirmed, histopathology of crop biopsy or tissues at necropsy is still the only definitive diagnosis of PDD. While crop biopsy has a high false negative rate, it is still a useful screening tool. Other helpful tests include radiographs, endoscopy and hematology and chemistry profiles. An exceptional test for live birds is fluoroscopy but this is rarely available to private practitioners (Gregory CR, Ritchie BR, Latimer KS, Progress in understanding proventricular dilatation disease, Proc Assoc Avian Vet, 200, pp. 269-276). As an infectious disease that causes inflammation of the nerves and affects the digestive system, preventing spread, reducing inflammation, aiding digestion and controlling secondary infections should be considered, for a long time, probably a year or more. Actually no specific therapeutic protocols are available to treat PDD, and only symptomatic therapies have been proposed.

The protocol IP as disclosed in the previous examples has been applied to a group of 18 severely PDD affected parrots, in which the diseases was diagnosed on the basis of clinical signs, histology on crop biopsy and Rx/Fluoroscopy tests. Birds were also screened for Avian Borna Virus (ABV) infection by mean of PCR test. Five PDD positive parrots were maintained as control group, and treated with a conventional palliative therapeutical protocol, based on the use of non steroidal anti inflammatory drugs (NSAIDs) administration, for long time as described by Dalhausen B, Aldred S, Colaizzi E, Resolution of clinical proventricular dilation disease by cyclooxygenase 2 inhibition, Proc Annu Conf. Assoc Avian Vet, 2002, 9-12.

Material and Methods and Results: Twenty-three parrots, belonging to different genera and species with severe form of clinically, histologically, and serologically confirmed PDD were selected. Parrots were subdivided into two groups:
Group A: 18 parrots treated with IP protocol with the same schedule of drugs administration reported in Example 1, 2, 3, and 4, but with the following doses: I.M. administration of an anti-COX2 drug (Robenacoxib, 2mg/kg weekly, for 5 weeks, and after monthly, for a minimum period of a year), I.D. administration (pathagial or inguinal area) of BCG; 0.1 ml weekly, for 5 weeks, and subsequently monthly for a minimum period of one year.
Group B: 5 parrots treated only with Celecoxib (anti-COX2 - NSAID - used at the dose of 20 mg/kg SID PO or 2x dose in favourite food).

Birds were weighed weekly and monthly; a radiologic control of G.I. apparatus was performed. Finally, a bioptic sample of gizzard and skin, at the site of intradermal administration, was also taken 3 months later the start of the protocol, to control the eventual gizzard-ganglia improvement. For protocol B (control NSAID Group) successful was adding Celecoxib to the bird's food. The dose in the food was double (40 mg/kg) since the half of it was expected to be lost. The medication was given on a small amount of food, preferably foods absorbing the drug so the chance of them eating it is improved. In most birds therapeutic response is gradual and many birds do not show much benefit for at least 2 weeks. Full recovery of clinical cases belonging to group A was gradual but complete, with evident differences between treated and untreated parrots after a period of 8-10 days. All the parrots of group A showed a total recovery in a period of two months. No deceased animals were observed during the period of six months of observation. Celecoxib only was also successfully used at the dose and with modalities reported in Dalhausen B, Aldred S, Colaizzi E, Resolution of clinical proventricular dilation disease by cyclooxygenase 2 inhibition, Proc Annu Conf. Assoc Avian Vet., 2002: 9-12. In these 5 control parrots treated only with anti-COX2, was observed a very slow and gradual improvement, with some important side effects (i.e., in two animal, a chronic bleeding was registered). An adult female of African Grey parrot (Psitthacus Erithacus), with crop biopsy positive for PDD, died within 7 days of initiation of Celecoxib therapy exhibiting an acute hemorrhagic event in the proventriculus on necropsy. All the birds of the study were routinely monitor for development of melena, which could indicate proventricular or intestinal bleeding. In such cases, Celecoxib is discontinued immediately and the bird is evaluated, including a gram stain to detect Clostridium in the feces. Celecoxib therapy was discontinuous also for inducing renal failure; NSAIDs are eliminated by renal clearance and should be used with caution in birds with renal disease. Nephrosis associated with NSAIDs used in birds is poorly documented.

Husbandry Considerations: if possible, the birds should be keep in roomy cages outside where sunlight and fresh air help to dilute/inactivate the virus, if present and related to the disease, also enhancing their general wellbeing. The stress should be maintained to a minimum, also by administration of a particular diet easily digested since proventricular and ventricular function is affected and GI motility is reduced or delayed. Liquid diets are available commercially for birds that are severely affected. Extruded diets (pellets) suitable for birds during the long recovery phase was used since they are easier to digest than seeds. An extruded diet also works well because Celecoxib diluted in water is absorbed into the food. Supplementing the diet with vegetables that are high in fibre might be beneficial to early cases by stimulating intestinal motility. Cruciferous vegetables are also beneficial sources of refined sugars (rich in oligofructosaccharides), which enhance viability of autochthonous flora (Lactobacillus and Bifidobacterium), thereby inhibiting gram-negative bacteria and Clostridium. In advanced cases however these foods may linger in the intestines and ferment. Periodic supplementation of probiotics is beneficial. 32% of birds involved in this study, that having PDD, often become anemic and hypo-proteinemic, The addition of powdered protein supplements such as egg or soy protein provides a readily utilizable source of protein. Also B complex vitamin supplementation was used to help correct anemia. Four of the 18 cases utilized to assess the IP protocol showed a central nervous system form of PDD (in which the prognosis is guarded); in these cases affected birds showed incoordination, tremors, and seizures.

Monitoring Progress of Therapy: therapy was monitored by weekly physical exam, monitoring body weight, repeated radiographs and evaluation to detect secondary infections. If monitoring by radiography the composition of the diet must be considered, especially if the bird was initially eating a primarily seed diet at the time of diagnosis and is later changed to an extruded diet. A substantial reduction of clinical symptoms was observed, almost in all treated parrots, beginning from the first week of treatment until to the total recovery. Treated birds, slowly regained their native bodily weight, in which the bodily weight trend is reported only for the six birds included in IP protocol. Only in a case we registered the death of a parrot, related to the development of a liver carcinoma. From the 5 parrots of the control group, 4 parrots died during four months after the end of the therapy, while 1 survived 1.5 year later. Histology performed at T1 (after six months of therapy) and at T2 (one year of therapy) demonstrated the progressive negativization of crop biopsies taken from all the parrots belonging to the Group A (94.44% of negativization), and only in a case of parrots belonging to the Group B (20% of negativization). The results demonstrated that the proposed treatment is safe, well tolerated by the animals and effective to reduce clinical signs and to improve body conditions in PDD affected parrots, the mechanism of protection based on BCG antigens administration is capable to redirect the autoimmune response away from central and peripheral nervous system. In particular the large percentage of ganglioside-specific CD4+ T cells accumulate in the antigen-induced granuloma sites, suggests a novel mechanism for infection-mediated modulation of autoimmunity.

### Example 6 - Canine IBD

Inflammatory bowel disease (IBD) is a gastrointestinal disorder where intestine remains irritated for a long period of time. IBD occurs when cells involved in inflammation and immune response are called into the lining of the GI tract. This causes the bowel lining to thicken and interferes with absorption and motility, or the ability of the bowel to contract and move food. IBD is usually seen in middle-aged to older dogs; however, some forms of IBD are seen in dogs less than 5 years old. Several breeds may be at increased risk for developing IBD, including the German Shepherd, Boxer, Shar-Pei, Soft Coated Wheaton Terrier and Rottweiler. The term IBD is often used interchangeably with other conditions including: Chronic Colitis/Colitis, Lymphocytic-plasmacytic Inflammatory Bowel Disease, Regional Enteritis, Granulomatous Enteritis, Spastic Bowel Syndrome. To further confuse matters, Irritable Bowel Syndrome (IBS) is often confused with IBD. Treatment for IBS is aimed at stress reduction; it is a completely different condition from IBD. The causes of inflammatory bowel disease are not well understood, the basic theory is that IBD is an immunologic reaction to some kind of stimulation, this could include an allergy, parasites, bacteria or an immune system problem. However, usually an extensive search for the cause is not made because of expense. A bacterium called Helicobacter is associated with inflammatory bowel disease. While it is not known which is the cause and which is the effect, Helicobacter infection can lead to ulceration and inflammation, compounding the problems of the IBD. Special treatment is necessary for this type of infection.

The most common symptoms of Inflammatory Bowel Disease include: persistent loose stools, frequent smaller stool, straining and diarrhea, weight loss, vomiting, low grade fevers, lethargy. Classical therapeutical approach is characterized by prednisone at a dose of 0.5-2.0 mg/kg divided twice daily for two to four weeks followed by a gradual decrease in 50 percent increments at two-week intervals. Alternate day or every third day treatment can often be reached by two to three months. Treatment may be discontinued altogether by three to six months in some cases. Moderate-to-severe cases and any cases in which the serum total protein levels are less than 5.5 g/dl should be treated more aggressively using an initial prednisone dose of 2.0 mg/kg per day for two to four weeks before an attempt is made to decrease the prednisone dosage. Dogs with this severity often require long-term therapy of months to years on an every-other-day or every third day basis to maintain remission of signs and maintain body weight. Use of combination drug therapy, such as prednisone and metronidazole, in moderate-to-severe IBD cases at the outset is recommended to improve chances of controlling clinical signs more quickly and to slow progression of the disease. Metronidazole has antibacterial and anti-inflammatory effects and is useful in treatment of IBD in dogs as well as in cats. The metronidazole is generally administered at 10-20 mg/kg in dogs and 5.0-7.5 mg/kg in cats two times daily. When prednisone and metronidazole are used in combination, the dosage level of each drug is generally gradually decreased as the animal's condition improves, serum total protein levels attain 6.0 g/dl or higher, and white blood cell counts return to normal. Steroids are then decreased gradually for several months before any reduction is made with the metronidazole dosage. If there has been an excellent response, it may be also possible for the metronidazole to be discontinued after several months. Alternatively, if chronic therapy is required, metronidazole can often be administered on a once daily and eventually on an every-other-day basis. If it is not possible to discontinue medications altogether because of recurrence of signs when no medications are given, satisfactory control may be maintained with prednisone and/or metronidazole given on an alternate day basis - giving prednisone on one day and metronidazole on the alternate day. Occasionally in dogs with moderate-to-severe IBD or in a case where both IBD and chronic bacterial overgrowth are present, it may be necessary to continue metronidazole on a long-term basis of months to years at 10-20 mg/kg twice daily.

Experimental Trial: Seventy-one (71) dogs were entered into this study. Seven dogs were not available for evaluation for the following reasons: three dogs died of unknown causes within 3 weeks of entering the study, one of which had a necropsy showing pancreatitis, one died of gastric torsion within 3 weeks of entering the study; two dogs were removed from the study within 2 weeks of being entered and one dog was removed from the study due to reclassification of its intestinal pathology to parassitosis. 64 dogs ranged in age from 1.5 to 14 years, with a mean age of 8 years. There were 25 males and 39 females. Twenty one (21) dogs were randomized to receive IP according to the treatment schedule mentioned in anti-leishmaniasis protocol as in see previous example 1, 21 received a classical therapeutical protocol CP (Metronidazole was administered at 10 mg/kg two times daily, and prednisone given at dosage of 2.0 mg/kg per day for four weeks before a decrease the dosage), and 22 received placebo once weekly. A total of 58 dogs completed all the doses (one year of protocol) of IP; 6 dogs did not receive all four doses because they developed a recrudescence of the syndrome before completion of the planned Immunomodulant. Median IBD symptoms free intervals for the three groups were 7.5, 6.3, and 2.2 months, respectively. In the IP group, 19 (90%) of 21 dogs showed a remission of the symptoms after the second administration; of this group, 12 dogs are actually free of disease; 7 (33%) dogs remained free of the symptoms for 1 year on longer. In the CP group of treated dogs, 18 (86%) of 21 dogs developed a relapse of the syndrome, with typical G.E. symptoms, within a period of few weeks. In the placebo group, 17 (77%) of 22 dogs maintained a severe symptomatology, while 5 dogs are alive and free of disease (after 1 year).

### Example 7 - Canine demodicosis

Twelve dogs diagnosed with generalized demodicosis were treated, 6 with IP and 6 with milbemycin oxime. Cases diagnosed before two years of age were classified as juvenile-onset (58.3%, 7 dogs) and those diagnosed after two years of age as adult-onset (41.6%, 5 dogs). Dogs were considered cleared of mites when none could be demonstrated in scrapings and cured if no relapse was seen for 12 months. As previous mentioned, in six dogs cured with milbemycin oxime, the drug was used for 1-6 months (mean 2.3 months) at a dosage of 0.5-1.6 mg kg-1 body weight (mean 0.75 mg kg-1). No significant difference in dosage or treatment time was seen between juvenile and adult cases. Six dogs cured with IP were treated at the same manner indicated as reported in Example 1, and 6). In group of IP treated dogs, not differences were observed between young and adult treated animals. On the contrary, for milbemycin oxime (M-O) protocol, chance of cure was significantly better in young animals (cured cases mean age 2.97 years) than in older animals (not cured cases mean age 8.02 years). For this second protocol, start of treatment early in the course of disease gave a significantly better chance of cure. Cases with severe pododemodicosis had less chance of a cure (2/3 not cured) by using M-O protocol, but not with IP protocol (2/2 cured).

Dogs: dogs of every age, breed or sex with generalized demodicosis were used in this study. All dogs. The diagnosis of generalized demodicosis was based on the presence of clinical lesions in conjunction with the demonstration of numerous mites, larvae or eggs in multiple skin scrapings (3-6) from different parts of the body or from one major region such as the head or all paws. Dogs developing the disease at less than two years of age were classified as juvenile-onset generalized demodicosis. Dogs that developed disease at two years of age or older were classified as adult onset generalized demodicosis.

Diagnostic tests: skin scrapings were performed as follows, a small area of =1 cm2 was clipped if necessary, one drop of mineral oil was applied on the skin surface and the skin was firmly squeezed between thumb and forefinger to express the mites out of the follicles into the mineral oil. The fold was then released and the extruded material transferred with a blunt scalpel from the surface of the skin to a glass slide, covered by a cover slip and examined microscopically. In the case of demodectic pododermatitis, hairs were also pulled from affected feet for microscopic examination. This latter method being helpful in demonstrating mites from areas of skin that are difficult to squeeze. A complete physical examination was performed on each animal. In animals with adult-onset generalized demodicosis, blood was drawn for a haemogram and a serum chemistry profile. The diagnosis of concurrent atopic dermatitis was based on history, clinical signs, ruling out of other causes of pruritic skin disease and supported by the performance of intradermal testing or skin biopsy.

Treatment: 6 dogs (3 young and 3 adults) were initially treated with milbemycin oxime orally at a minimum dosage of 0.5 mg kg body weight once daily. The remained 6 dogs (4 young and 2 adults) were treated by the IP protocol, use in the same manner as reported in Example 1, and 6.

Follow-up: follow-up visits were scheduled once a month for clinical evaluation and skin scrapings. The scraping sites were recorded and sampled at each subsequent examination. The dog was considered cleared of mites when no clinical lesions remained and negative scrapings on two subsequent visits one month apart were obtained. Treatment was then discontinued. If the mite count had not decreased from the previous examination, the dosage of milbemycin was doubled, but the IP protocol was unchanged. If on the higher dose of milbemycin no decrease in mite count was registered on two successive evaluations, the treatment was considered to have failed and no additional increase of dose was carried out. Dogs that were not cured obtained maintenance doses of milbemycin oxime once or twice a week to remain clinically asymptomatic. This dose was based on the dose for clinical cure. All dogs were followed for at least one year after their last treatment. During that time the owner was told to report any skin lesions on their dog and if so return to the clinic for skin scrapings. If mites were found, a relapse was declared. The dog was considered cured if there was no relapse during the 12-month follow-up period. For dogs that remained clinically normal, follow-up information was obtained by telephone.

Statistics: for statistical evaluation the Mantel-Haenszel correlation test, the Mann-Whitney U-test and the Fisher's exact test were used.

Results: a total of 12 cases entered the study. Of the dogs that completed the study, 41.6% were intact males (5/6), 16.6% were neutered males (2/6), and 41% (5/6) were females, two of which were spayed. Four breeds were represented, of which 83.3% (10/12) were pure breed, with a predilection for Staffordshire Bull Terrier (2), West Highland White Terrier (2), Dobermann (3), Boxer (1), PittBull (2). Fifty-eight per cent (7/12) of the patients had juvenile onset disease and 41.6% (5/12) had adult-onset disease. The dose rate of milbemycin oxime used was 0.5-1.6 mg kg-1 given once daily (mean 0.75 mg kg-1). Duration of treatment was 1-6 months (mean 2.3 months, the same for dogs with juvenile-onset disease as for dogs with adult-onset disease).

Statistical evaluation: There is a clear correlation between age at onset of disease and whether the disease could be cured. The older animals have less chance of cure (P< 0.0001 Fisher's exact test). In this study the median duration of disease prior to the initiation of treatment with Immunomodulatin protocol or milbemycin oxime was 2.3 months in cases cured and 6.7 months in cases not cured, indicating that the longer the disease has been going on before treatment the poorer the result (P< 0.0001 Mantel-Haenszel correlation test). No significant difference in time of treatment to cure was registered between juvenile- and adult-onset cases (P= 0.3048 NS, Mann-Whitney U -test). Two of a total of 5 dogs with severe pododemodicosis were not cured, but from the two cases belonging to IP treatment, we observed a total improvement. Chance of cure with M-O was significantly less in cases with severe pododermatitis (P< 0.0001 Fisher's exact test), but not in case o fuse of our IP protocol.

## Claims

1. Combination of Bacillus Calmette Guerin (BCG) with at least one COX-2 preferential FANS selected from the group consisting of meloxicam, nimesulide, tolfenamic, carprofen, etodolac, nabumetone.

2. Combination of claim 1 for use as a medicament.

3. Pharmaceutical composition comprising the combination of anyone of claim 1 and pharmaceutically acceptable diluents and/or adjuvants and/or excipients and/or vehicles.

4. Combination of claim 1 for use for the prevention or treatment of pathologies related to the loss of macrophages functions and deficiencies of cell-mediated immunity selected from the group consisting of Leishmaniasis, Vaccine Associated Feline Sarcoma, feline injection site sarcoma (FISS) Feline Infectious Peritonitis, dog idiopathic inflammatory bowel disease, human idiopathic inflammatory bowel disease, Feline Diarroic Syndromes, Malaria, Tuberculosis, Allergic Encephalitis, Atopic Dermatitis, HIV/AIDS, Crohn disease, Ulcerative colitis, Psoriasis, Feline Chronic lymphocytic plasmacytic gingivostomatitis, Feline odontoclastic resorptive lesions (FORLs), human Inflammatory Mammary Carcinoma, dog Inflammatory Mammary Carcinoma, Metastatic Melanoma, Avian Aspergillosis, Feline Squamous Carcinoma, Toxoplasmosis, Immune Complex Diseases, Avian Proventricular Dilatation Disease (PDD), Multiple Sclerosis, Canine demodicosis, Canine Systemic Lupus, Canine Leucocyte Adhesion Deficiency / Canine Granulocytopathy Syndrome, Combined Immunodeficiency, Complement (C3) Deficiency, Cyclic Haematopoiesis of Grey Collies, Canine Growth hormone deficiency, IgA Deficiency, Lethal Acrodermatitis in Bull Terriers, Pelger-Huet Anomaly, Feline Immunodeficiency Virus, Feline viral leucosis, Psittacine Beak and Feather Disease (PBFD), Polyomavirus infections, Secondary or acquired immunodeficiency syndromes

5. Combination for use of claim 4 **characterised in that** is administered once a week for 5 times (loading phase), followed by administration once a month for a year (maintenance phase).

6. Combination of Bacillus Calmette Guerin (BCG) with at least one anti-COX-2 selective molecule selected from the group consisting of celecoxib, deracoxib, etoricoxib, firocoxib, firecoxib, lumiracoxib, mavacoxib, parecoxib, robenacoxib, rofecoxib, valdecoxib and vaxamine for use for the treatment of pathology related to the loss of macrophages functions and deficiencies of cell-mediated immunity selected from the group consisting of: Leishmaniasis, Vaccine Associated Feline Sarcoma, feline injection site sarcoma (FISS) Feline Infectious Peritonitis, dog idiopathic inflammatory bowel diseaseMalaria,Feline Chronic lymphocytic plasmacytic gingivostomatitis Avian Proventricular Dilatation Disease (PDD) Canine demodicosis **characterised in that** is administered by systemic administration.

7. Combination for use according to claim 6 wherein the combination is administered once a week for 5 times (loading phase), followed by administration once a month for a year (maintenance phase).

## Patentansprüche

1. Kombination aus Bacillus Calmette Guerin (BCG) mit zumindest einem COX-2, bevorzugt FANS, ausgewählt aus der Gruppe, bestehend aus Meloxicam, Nimesulid, Tolfenaminsäure, Carprofen, Etodolac, Nabumeton.

2. Kombination nach Anspruch 1 für die Verwendung als Medikament.

3. Pharmazeutische Zusammensetzung umfassend jede Kombination nach Anspruch 1 und pharmazeutisch annehmbare Verdünnungsmittel und/oder Adjuvantien und/oder Hilfsstoffe und/oder Trägerstoffe.

4. Kombination nach Anspruch 1 für die Verwendung zur Prävention oder Behandlung von Pathologien, die mit dem Verlust von Makrophagen-Funktionen und Defiziten in der zellvermittelten Immunität zusammenhängen, ausgewählt aus der Gruppe, bestehend aus Leishmaniose, vakzinassoziiertes felines Sarkom, felines Injektionsstellen-Sarkom (FISS), feline infektiöse Peritonitis, idiopathische entzündliche Darmerkrankung bei Hunden, idiopathische entzündliche Darmerkrankung bei Menschen, feline Durchfallsyndrome, Malaria, Tuberkulose, allergische Enzephalitis, atopische Dermatitis, HIV/AIDS, Morbus Crohn, Colitis ulcerosa, Psoriasis, feline chronisch-lymphatisch-plasmatische Gingivostomatitis, feline odontoklastische resorptive Läsionen (FORLs), inflammatorisches Brustkarzinom bei Menschen, inflammatorisches Brustkarzinom bei Hunden, metastatisches Melanom, aviane Aspergillose, felines Plattenepithelkarzinom, Toxoplasmose, Immunkomplexkrankheiten, aviane Düsenmagendilatation (PDD), Multiple Sklerose, canine Demodikose, systemischer Lupus bei Hunden, canines leukozytisches Adhäsionsdefizit / canines Granulozytopathiesyndrom, kombinierter Immundefekt, komplement (C3) Defizit, zyklische Hämatopoese bei grauen Collies, canines Wachstumshormondefizit, IgA-Defizit, letale Akrodermatits bei Bullterriern, Pelger-Huet-Anomalie, Felines Immundefizienz-Virus, feline virale Leukose, Schnabel- und Federkrankheit bei Papageien (PBFD), Polyomavirusinfektionen, sekundäre oder erworbene Immundefektsyndrome.

5. Kombination zur Verwendung nach Anspruch 4, **dadurch gekennzeichnet, dass** sie fünfmal, einmal pro Woche (Initialphase) verabreicht wird, gefolgt von einer Verabreichung einmal im Monat für ein Jahr (Erhaltungsphase).

6. Kombination aus Bacillus Calmette Guerin (BCG) mit zumindest einem anti-Cox-2 selektiven Molekül, ausgewählt aus der Gruppe, bestehend aus Celecoxib, Deracoxib, Etoricoxib, Firocoxib, Firecoxib, Lumiracoxib, Mavacoxib, Parecoxib, Robenacoxib, Rofecoxib, Valdecoxib und Vaxamin zur Verwendung für die Behandlung von Pathologien, die mit dem Verlust von Makrophagen-Funktionen und Defiziten in der zellvermittelten Immunität zusammenhängen, ausgewählt aus der Gruppe, bestehend aus: Leishmaniose, vakzinassoziiertes felines-Sarkom, felines Injektionsstellen Sarkom (FISS), feline infektiöse Peritonitis, idiopathische entzündliche Darmerkrankung bei Hunden, Malaria, feline chronisch-lymphatischplasmatische Gingivostomatitis, aviane Düsenmagendilatation (PDD), canine Demodikose, **dadurch gekennzeichnet, dass** die Verabreichung mittels systemischer Verabreichung erfolgt.

7. Kombination für die Verwendung, gemäß Anspruch 6, wobei die Kombination fünfmal, einmal pro Woche (Initiierungsphase) verabreicht wird, gefolgt von einer Verabreichung einmal im Monat für ein Jahr (Erhaltungsphase).

## Revendications

1. Combinaison de Bacille de Calmette et Guérin (BCG) avec au moins un AINS préférentiel pour COX-2 choisi dans le groupe constitué des méloxicam, nimésulide, acide tolfénamique, carprofène, étodolac, nabumétone.

2. Combinaison de la revendication 1 pour utilisation en tant que médicament.

3. Composition pharmaceutique comprenant la combinaison de l'une quelconque de la revendication 1 et des diluants et/ou adjuvants et/ou excipients et/ou véhicules pharmaceutiquement acceptables.

4. Combinaison de la revendication 1 pour utilisation pour la prévention ou le traitement de pathologies liées à la perte de fonctions des macrophages et des déficiences de l'immunité à médiation cellulaire choisies dans le groupe constitué des leishmaniose, sarcome félin associé aux vaccins, sarcome félin au site d'injection (FISS), péritonite infectieuse féline, syndrome abdominal inflammatoire idiopathique canin, syndrome abdominal inflammatoire idiopathique humain, syndromes diarrhéiques félin, paludisme, tuberculose, encéphalite allergique, dermite atopique, VIH/SIDA, maladie de Crohn, rectocolite hémorragique, psoriasis, gingivostomatite plasmocytaire lymphocytaire chronique féline, lésions de résorption odontoclastique féline (FORL), carcinome mammaire inflammatoire humain, carcinome mammaire inflammatoire canin, mélanome métastasique, aspergillose aviaire, carcinome squameux félin, toxoplasmose, maladies du complexe immun, maladie de dilatation du proventricule aviaire (PDD), sclérose en plaques, démodécie canine, lupus disséminé canin, déficience canine d'adhésion des leucocytes / syndrome de granulocytopathie canine, immunodéficience combinée, déficience en complément (C3), hématopoïèse cyclique du Colley gris, déficience canine en hormone de croissance, déficience en IgA, acrodermatite létale du bull terrier, anomalie de Pelger-Huet, virus de l'immunodéficience féline, leucose virale féline, maladie du bec et des plumes des psittacidés (PBFD), infections à polyomavirus, syndromes d'immunodéficience secondaire ou acquise.

5. Combinaison pour utilisation de la revendication 4 **caractérisée en ce qu'**elle est administrée une fois par semaine 5 fois (phase de charge), suivie par l'administration une fois par mois pendant un an (phase de maintenance).

6. Combinaison de Bacille de Calmette et Guérin (BCG) avec au moins une molécule anti-COX-2 sélective choisie dans le groupe constitué des célécoxib, déracoxib, étoricoxib, firocoxib, firécoxib, lumiracoxib, mavacoxib, parécoxib, robénacoxib, rofécoxib, valdécoxib et vaxamine pour utilisation pour le traitement d'une pathologie liée à la perte de fonctions des macrophages et des déficiences de l'immunité à médiation cellulaire choisie dans le groupe constitué des : leishmaniose, sarcome félin associé aux vaccins, sarcome félin au site d'injection (FISS), péritonite infectieuse féline, syndrome abdominal inflammatoire idiopathique canin, paludisme, gingivostomatite plasmocytaire lymphocytaire chronique féline, maladie de dilatation du proventricule aviaire (PDD), démodécie canine, **caractérisée en ce qu'**elle est administrée par administration systémique.

7. Combinaison pour utilisation selon la revendication 6 dans laquelle la combinaison est administrée une fois par semaine 5 fois (phase de charge), suivie par l'administration une fois par mois pendant un an (phase de maintenance).
